# EUROPEAN PATENT SPECIFICATION

(11) **EP 0 294 035 B1**
(45) Date of publication and mention of the grant of the patent: **31.08.1994**
(21) Application number: 88304124.6
(22) Date of filing: 06.05.1988
(51) Int. Cl.: C07C 233/67, C07C 323/00, C07C 315/00, A61K 31/19, A61K 7/06

(54) **Benzoylaminophenoxybutanoic acid derivatives**
Benzoylaminophenoxybuttersäure-Derivate
Dérivés de l'acide benzoylaminophénoxybutyrique

(30) Priority: 04.06.1987 JP 138898/87; 27.08.1987 JP 211448/87
(43) Date of publication of application: 07.12.1988
(73) Proprietor: ONO PHARMACEUTICAL CO., LTD., Chuo-ku Osaka 541 (JP)
(72) Inventor: Nakai, Hisao, Takatsuki-city Osaka (JP); Terashima, Hiroshi, Takatsuki-city Osaka (JP); Arai, Yoshinobu, Shimamotocho Mishima-gun Osaka (JP)
(74) Representative: Bentham, Stephen

(56) References cited:
- EP-A- 0 173 516
- WO-A-86/05779
- GB-A- 1 088 295

## Description

This invention relates to novel benzoylaminophenoxybutanoic acid derivatives, to processes for their preparation, pharmaceutical compositions containing them and their use.

Many theories have been expounded concerning the origin of androgenic alopecia based on, for example, (1) an imbalance of hormones, (2) genetic factors, (3) circulatory failure or (4) nutrition. It has also been suggested that testosterone (androgenic hormone) played an important role in the generation of hair.

The theory of Adachi et al (Biochem. Biophys. Res. Commun., 41, 884 (1970)) in which the relation between testosterone and androgenic alopecia is proved by biochemical experiments, is as follows:
i) first, testosterone biosynthesized in the testis is converted into dihydrotestosterone by 5a-reductase present in, inter alia, hair follicles and sebaceous glands on the head;
ii) the dihydrotestosterone causes a substantial reduction in the activities of adenyl cyclase;
iii) the level of cyclic-AMP in cells decreases; and
iv) a lowering of energy generation of hairs and limbus and suppression of protein synthesis is induced.

According to this theory it is thought that, as a result of the series of phenomena just described, hairs in the growing phase shift to the resting phase, the terminal hairs change to soft hairs, and androgenic alopecia results.

A report by H.V. Schweikert (J. Clin. Endocr., 38, 811 (1974)) supports this theory in that large quantities of metabolites produced by 5a-reductase such as dihydrotestosterone are present in hair follicles of androgenic alopecia patients in quantities greater than those in female or healthy males.

It has also been reported that dihydrotestosterone produced from testosterone by 5a-reductase also plays an important physiological role in the generation of acnes (acne, pimples etc.) in addition to androgenic alopecia. J.B. Hay et al (Br. J. Dermatol., 91, 123 (1974)) has reported from a comparative study of the affected skin of acne-patients and healthy skin that the metabolism of testosterone by 5a-reductase was enhanced in the parts affected by aggravated acne.

G. Sansone et al (See J. Invest. Dermatol., 56, 366 (1971)) found that the ability to synthesise dihydrotestosterone from testosterone increased from two to twenty times in the affected part of acne-patients compared to that in healthy man, and suggested that dihydrotestosterone generated by 5a-reductase is strongly related to the generation or aggravation of acne.

Dihydrotestosterone is also related to hypertrophy of the prostate. Cowan et al (J. Steroid Biochemistry, 11, 609 (1979)) reported that much dihydrotestosterone existed in the prostate of prostatic hypertrophy patients. It has been reported (See J. Clinical Endocrinol. and Metabolism, 56, 139 (1983)) that the activity of 5a-reductase in the prostate of prostatic hypertrophy patients is abnormally increased.

It has become clear from these reports that dihydrotestosterone plays an important role in the generation and development of prostatic hypertrophy.

Research has been carried out to discover compounds active as 5a-reductase inhibitors: the compounds have been mainly steroids or derivatives thereof.

Widespread investigations have been carried out in order to discover compounds which have a non- steroidal structure and possess inhibitory activity on 5a-reductase. We have filed a number of applications directed to compounds wherein cinnamic acid or benzoic acids form amides with anilines, [See Japanese Patent Kokai Nos. 60-97946, 60-116657, 60-142936, 60-142941, 60-146855, 61-126061 (equivalent to EP 0173516), 62-198652 and 62-198653].

For example, EP 0173516 and Japanese Patent Kokai No. 61-126061 describe a very wide range of amide compounds which possess inhibitory activity on 5a-reductase. The compounds must closely related to those of the present invention, and which are described as possessing antagonistic activity on leukotrienes, inhibitory activity on 5a-reductase, on phospholipase and on aldose reductase are defined by the general formula: 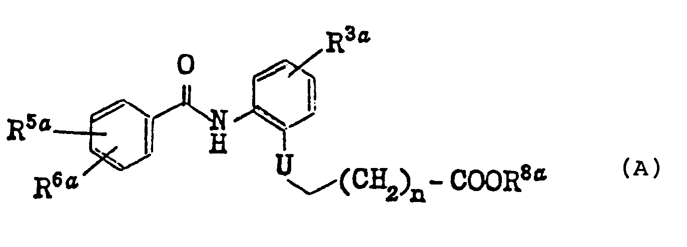 [wherein R^{3a} represents, inter alia, a hydrogen or halogen atom, R^{5a} and R^{6a} represent independently, a hydrogen or halogen atom, or a straight or branched chain alkyl, alkenyl or alkynyl group of from 1 to 20 carbon atom(s) in which from 1 to 5 carbon atoms may be replaced by an oxygen atom, sulfur atom, halogen atom, nitrogen atom, benzene ring, thiophene ring, naphthalene ring, carbocyclic ring of from 4 to 7 carbon atoms, carbonyl group, carbonyloxy group, hydroxy group, carboxy group, azido group, or nitro group, R8a represents a hydrogen atom or a straight or branched chain alkyl group of from 1 to 6 carbon atom(s), U represents an oxygen atom or sulfur atom, and n represents an integer of from 1 to 10].

Benzoylamino-phenoxy (and phenylthio) butanoic acids are also described in
British Patent No. 1077936,
British Patent No. 1088295,
United States Patent No. 3549689, and
PCT Patent Publication No. 8605779.

However, all of the compounds described in the British and US Patents are distinguished from those of the present invention both in chemical structure and in their biological activity. Whereas the compounds of the present invention possess inhibitory activity on 5a-reductase the known compounds are described as having anti-inflammatory activity (including antagonistic activity on SRS).

PCT Patent Publication No. 8605779 describes, inter alia, compounds embraced by the general formula: 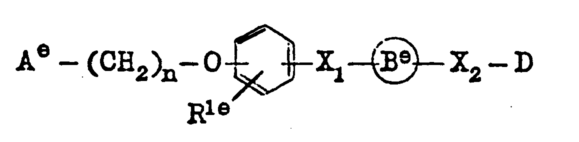
[wherein A^{e} represents hydrogen, phenyl or phenoxy,
n represents 3 - 10
R^{ie} represents hydrogen or lower alkoxy,
X¹ represents -CONH-, 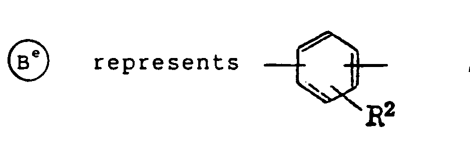
(wherein R² represents hydrogen, halogen or nitro)
X² represents -0-Y⁴⁻ (wherein Y⁴ represents alkylene of from 1 to 6 carbon atom(s)) and D represents carboxy or alkoxycarbonyl], which are described as possessing antagonistic activity on SRS.
The compounds of the present invention described hereinafter, fall within the general scope of the formula designated above as (A). However, none of the compounds has been previously prepared and all of them have been found to possess a surprisingly high level of inhibitory activity on 5a-reductase. There is no disclosure in EP 0173516 which might suggest that the compounds which have now been prepared might be of particular interest or that they might possess high activity in the inhibition of 5a-reductase.

The general formula disclosed in the PCT Patent Publication embraces certain of the compounds of the present invention. However, no compound of the present invention is disclosed. Furthermore, the inhibitory activity on 5a-reductase of a compound from the PCT Patent Publication having a chemical structure similar to those of the present invention has been determined and is very weak. The strong inhibitory activity on 5a-reductase of the compounds of the present invention is neither disclosed nor suggested by the PCT Publication.

The present invention accordingly provides a benzoylaminophenoxybutanoic acid derivative of the general formula: 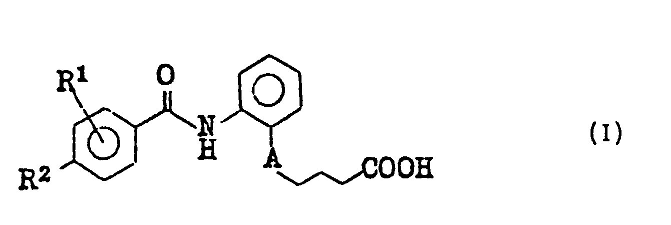
[wherein A represents an oxygen atom, a sulfur atom or a sulfinyl (SO) group,
R¹ represents a hydrogen atom or a methyl group,
R² represents a group of the general formula: 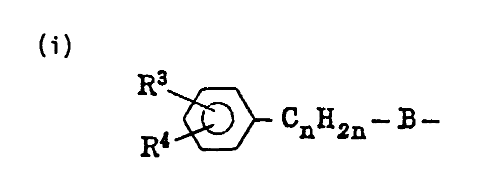 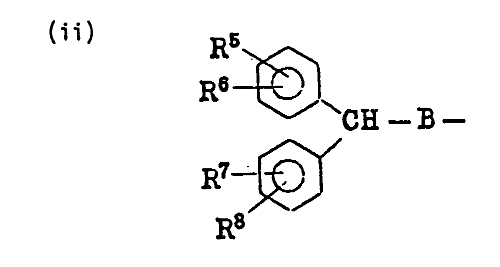 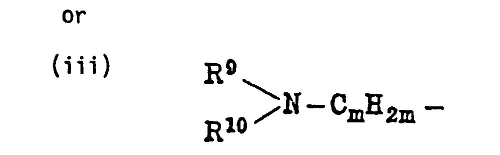 [wherein
B represents an oxygen atom, a sulfur atom or a group of the general formula:
NR¹¹ (wherein R¹¹ represents a hydrogen atom or an alkyl group of from 1 to 4 carbon atom(s)), R³, R⁴, R⁵,
R6, R⁷ and
R⁸ represent, independently, a hydrogen atom, an alkyl group of from 1 to 4 carbon atom(s), a halogen atom or a trifluoromethyl group,
m represents 0 or 1,
n represents an integer of from 1 to 4, and
R⁹ and R¹⁰, which may be the same or different, each represents a hydrogen atom, an alkyl group of from
1 to 5 carbon atom(s) or a group of the general formula: 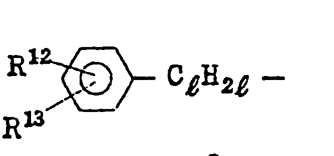 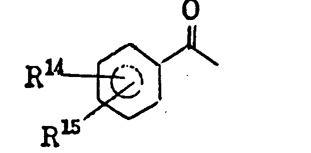 or 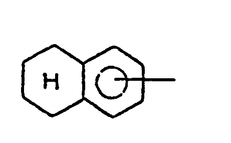
(wherein R¹², R¹³, R¹⁴ and R¹⁵ represent, independently, a hydrogen atom, an alkyl group of from 1 to 4 carbon atom(s), a halogen atom or a trifluoromethyl group, and 1 represents an integer of from 1 to 4), with the proviso that R⁹ and R¹⁰ do not simultaneously represent hydrogen atoms], or a non-toxic salt thereof.

In general formula (I), alkyl groups of from ¹ to ⁴ carbon atom(s) represented by ^{R3, R4, R5, R6,} R7, _{R8}, R¹¹, R¹², R¹³, R¹⁴ and R¹⁵ are methyl, ethyl, propyl and butyl groups and isomers thereof.

In general formula (I), alkyl groups of from 1 to 5 carbon atom(s) represented by R¹¹ are the groups described above and the pentyl group and isomers thereof.

In the general formula (I), halogen atoms represented by R³, R⁴, R⁵, R⁶, R⁷, R⁸, R¹², R¹3, R¹⁴ and R¹⁵ are fluorine, chlorine, bromine and iodine.

The present invention can be classified according to the values of R² into three groups of compounds represented by the general formulae: 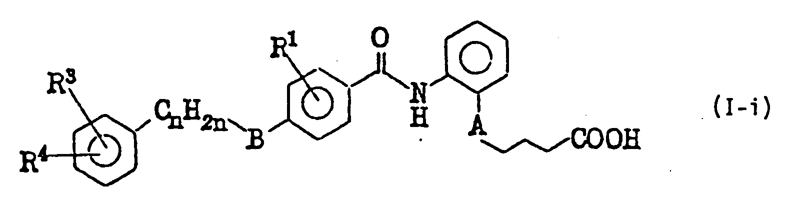 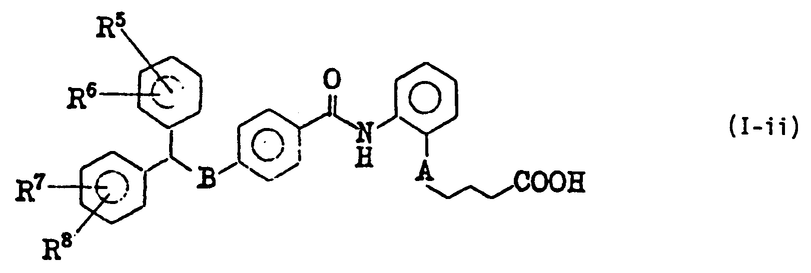 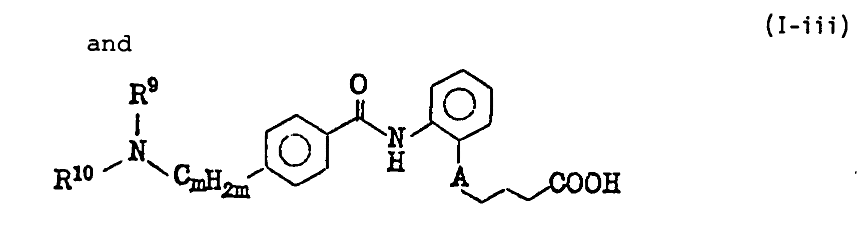 [wherein the various symbols are as hereinbefore defined].

The compounds of general formula (I) may be converted into the corresponding salts by known methods. Water soluble salts are preferable. Suitable salts include, for example,
. salts of alkali metals, e.g. sodium and potassium,
. salts of alkaline earth metals, e.g. calcium and magnesium,
. ammonium salts,
. salts of pharmaceutically acceptable amines, e.g. tetraalkylammonium salts such as tetramethylammonium, triethylamine, methylamine, dimethylamine, cyclopentylamine, benzylamine, phenethylamine, piperidine, monoethanolamine, diethanolamine, tris (hydroxymethyl)-aminomethane, lysine, arginine and N-methyl-D-glucamine salts.

According to a feature of the present invention compounds of the general formula (I), wherein A is an oxygen atom, a sulfur atom or a group of the general formula: NR¹¹ (wherein R11 is as hereinbefore defined), i.e. the compounds of the general formula: 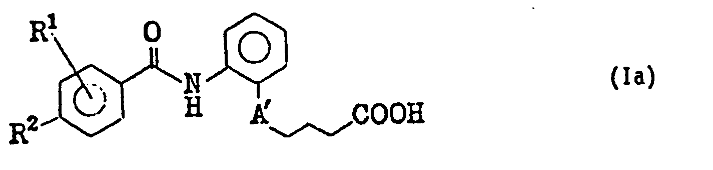 [wherein A' represents an oxygen atom, a sulfur atom or a group of general formula NR¹¹ (wherein R¹¹ is as hereinbefore defined), and the other symbols are as hereinbefore defined] are prepared by a process which comprises the saponification of a compound of the general formula: 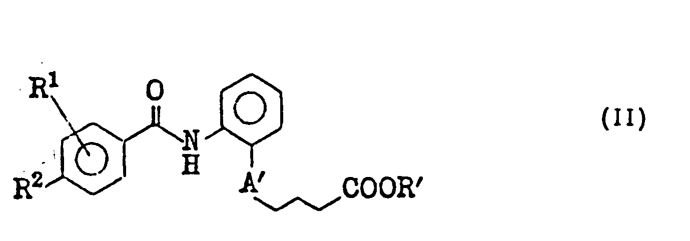
wherein R' represents an alkyl group of from 1 to 4 carbon atoms and the other symbols are as hereinbefore defined.

The saponification may be carried out by known methods, for example, using an aqueous solution of alkali (e.g. potassium hydroxide, sodium hydroxide, lithium hydroxide, potassium carbonate or sodium carbonate) in a water-miscible organic solvent (e.g. tetrahydrofuran (THF), dioxan, ethanol or methanol). The reaction is generally carried out at a temperature of from -10°C to 100°C.

According to a further feature of the invention compounds of general formula I wherein A represents a sulfinyl group can be prepared by oxidizing a compound of general formula (I) wherein A represents a sulfur atom, i.e. a compound of the general formula: 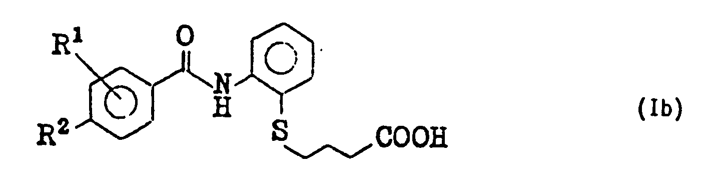 (wherein the various symbols are as hereinbefore defined) to convert the group -S- to a group -S(=0)-. The sulfinyl compound obtained is of the general formula: 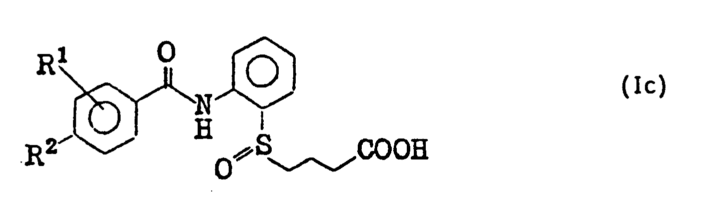 wherein the various symbols are as hereinbefore defined.

The oxidation may be carried out by known methods, for example, using an aqueous solution of perhalogenate (e.g. sodium periodate), in a water-miscible organic solvent (e.g. methanol, ethanol or dimethoxyethane).

The compounds of general formula (II) can be prepared by the reactions shown in the following scheme (A). The reactions in the scheme are known:
A' represents an oxygen atom or sulfur atom,
B' represents an oxygen atom or sulfur atom,
R" represents an alkyl group of from 1 to 4 carbon atom(s).
R represents a group of the general formula: 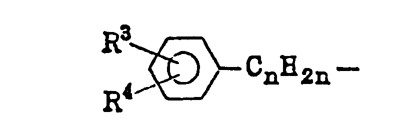 or 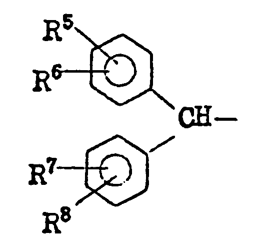
X¹, X², X³, X⁴, X⁵ represent a halogen atom, a tosyl group or a mesyl group.

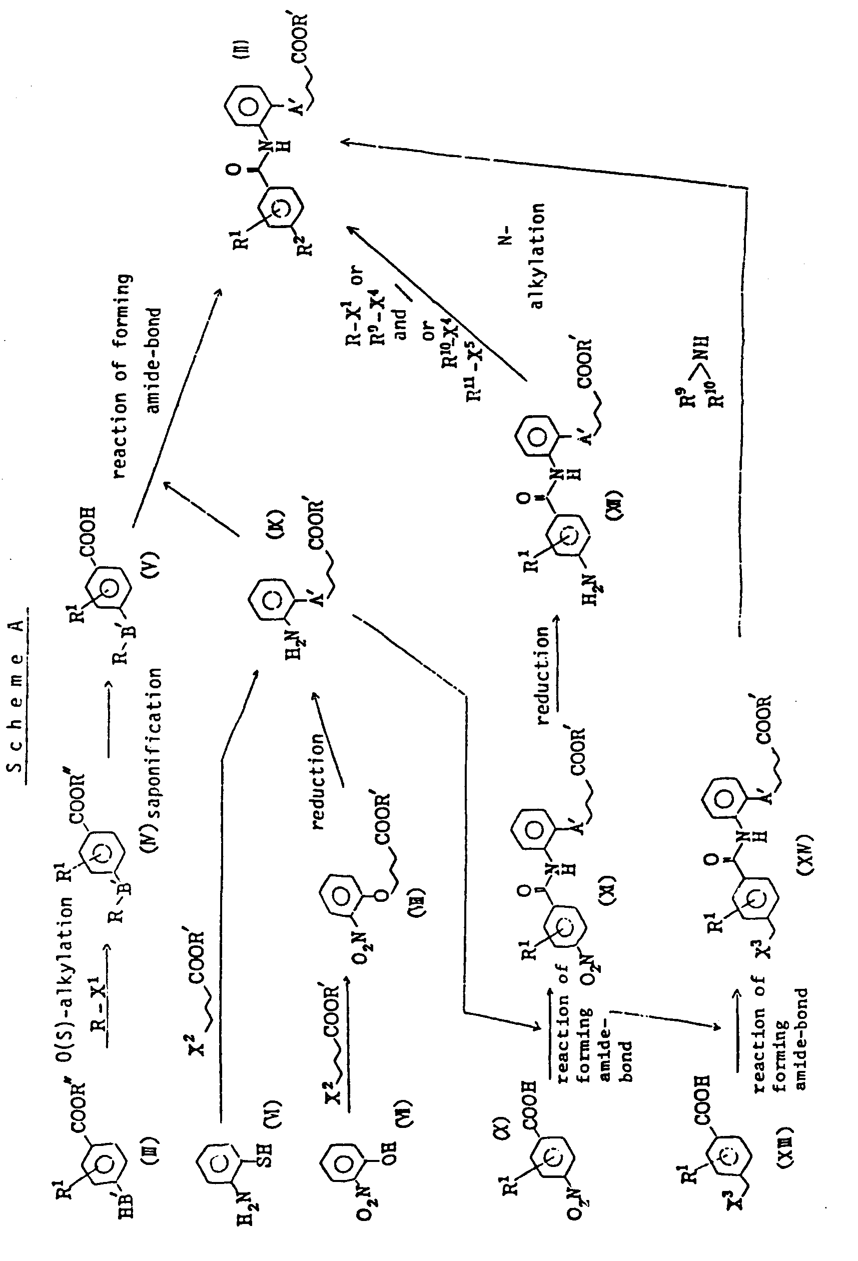

Methods for the preparation of an amide bond by reacting a carboxylic acid or an activated derivative thereof with an amine are known and include, for example,
(A) using a mixed acid anhydride as the activated derivative,
(B) using an acid halide as the activated derivative,
(C) using a condensing agent, e.g. dicyclohexylcarbodiimide (DCC).

Method (A) using a mixed acid anhydride may be carried out, for example, by reacting, a mixed acid anhydride obtained by acylating a carboxylic acid with an acyl halide (e.g. pivaloyl chloride, tosyl chloride or mesyl chloride) or an acid derivative (e.g. ethyl chloroformate or isobutyl chloroformate) in the presence of a tertiary amine (e.g. pyridine, triethylamine or picoline) in an inert organic solvent (e.g. chloroform, methylene chloride, diethyl ether or THF) or without solvent at from 0°C to 40 _{°} C, with an amine in an inert organic solvent (for example one of those described above) at from 0 _{°} C to 40 _{°} C.

Method (B) using acid halide may be carried out, for example, by reacting, an acid halide obtained by treating a carboxylic acid with an acid halide (e.g. thionyl chloride or oxalyl chloride) in an inert organic solvent (e.g. as described above) at from -20 °C to the reflux temperature of the solvent used, with an amine in an inert organic solvent (e.g. as described above) at from 0°C to 40°C.

Method (C) using a condensing agent such as DCC may be carried out, for example, by reacting a carboxylic acid with an amine, using a condensing agent such as DCC, in the presence or absence of tertiary amine (e.g as described above), in an inert organic solvent (e.g as described above) or without solvent, at from 0 _{°} C to 40 _{°} C.

The reactions of (A), (B) and (C) are preferably carried out in an inert gas atmosphere (using e.g. argon or nitrogen) and under anhydrous conditions.

In the processes described in this specification, in each reaction, products may be purified by conventional methods, for example, distillation at atmospheric or reduced pressure, high performance liquid chromatography, thin layer chromatography using silica gel or magnesium silicate or washing or recrystallization. Purification may be carried out after each reaction or after a series of reactions.

Starting materials and reagents for use in the processes of the present invention are known or may be prepared by known methods.

The compounds of general formula (I) possess an inhibitory activity on 5a-reductase and are therefore useful for the prevention and/or treatment of diseases resulting from the excess generation of dihydrotestosterone in mammals, especially humans. Such diseases include, for example, alopecia, e.g. androgenic alopecia, acnes and hypertrophy of the prostate.

The high inhibitory activity on 5a-reductase of the compounds of the present invention is demonstrated by the screening system described hereafter.

### Inhibitory activity on 5a-reductase in vitro

### (1) Method

The test was carried out with reference to the method of J. Shimazaki et al [See Endocrinol., Japan., 18, 179 (1971)].

Male rat's prostate (4g) was homogenized with three times its volume of 0.1 M HEPES buffer (pH 7.4) including 0.25M cane sugar and was then centrifuged at 3000 r.p.m. for 10 mins.

The precipitate was suspended in the buffer solution described above (10ml), and the suspension was centrifuged at 3000 r.p.m. for 5 mins. The resulting precipitate was suspended in the buffer solution (3ml) described above and was used as a source of enzyme.

A reaction mixture (total volume 0.1ml) of [4-C¹⁴]-testosterone (1.5n mol, 1.5 x 10⁵ cpm), NADPH (0.5 µmol), enzyme source (0.03ml) was prepared as described above and several concentrations of the compounds of the present invention was incubated for 60 mins at 37 °C. Enzyme reaction was quenched by addition of a mixture (0.4ml) of chloroform and methanol (1:2), and the mixture was centrifuged at 2000 r.p.m. for 3 mins. The supernatant (50 µl) was spotted on silica gel thin layer plate. The spot on the plate was developed with a mixture of chloroform, methanol and acetic acid (99.2 : 0.6 : 0.2). Radioactivity of the dihydrotestosterone on each plate was measured by radio-autography TLC scanning and an inhibitory ratio was calculated. The results are shown in the following Table 1.

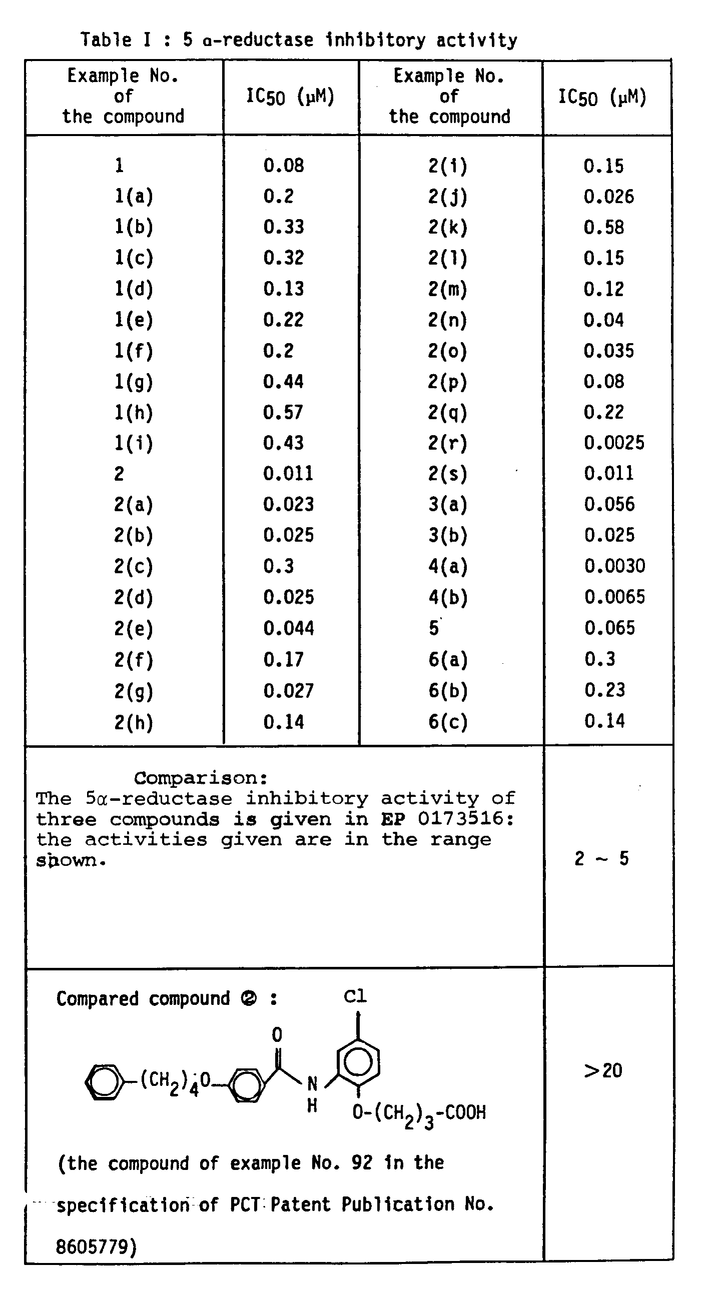

### (2) Results

The results of the test show that all of the compounds of the present invention possess a strong inhibitory activity on 5a-reductase. It will be seen that the inhibitory activities on 5a-reductase of the compounds of the present invention are from approximately 10 to 10³ times as strong as those of the comparison compounds from EP 0173516. The superiority of the compounds of the present invention is even more marked in comparison with the compound from PCT Patent Publication 8605779. There is no disclosure in EP 0173516 or in the PCT Publication to suggest that the compounds of the present invention would possess the high levels if 5a-reductase inhibitory activity shown by the results in Table I.

The results above were obtained in vitro. It is known that compounds wherein R¹ is a methyl group in the general formula (I) of the present invention are relatively stable to enzymes present in the liver and such compounds are therefore more likely to be useful in practice.

The high inhibitory activity on 5a-reductase of the compounds of the present invention render them useful for the prevention and/or treatment of disease resulting from excess generation of dihydrotestosterone in mammals, especially humans. Moreover, it was confirmed that the toxicity of the compounds of the present invention was very low so that they may be used as medicine with sufficient safety.

The compounds of the present invention will normally be administered systemically (mainly in the prevention and/or treatment of prostatic hypertrophy) or partially (mainly in the prevention and/or treatment of alopecia and acne), usually by oral or parenteral administration.

The doses to be administered are determined depending upon, for example, age, body weight, symptoms, the desired therapeutic effect, the route of administration, and the duration of the treatment. In the human adult, for the treatment and/or prevention of prostatic hypertrophy, the doses per person per dose are generally from 1 mg to 1 g, by oral administration, up to several times per day, and from 100 I1.g to 100 mg, by parenteral administration (preferably intravenous administration) up to several times per day.

In the human adult, for the treatment and/or prevention of alopecia and/or acne, the doses per person per dose are generally from 10 I1.g to 50 mg, by dermal administration up to several times per day.

As mentioned above, the doses to be used depend upon various factors. Therefore, there are cases in which doses lower than or greater than the ranges specified above may be used.

The present invention provides pharmaceutical compositions which comprise a compound of general formula I or a non-toxic salt thereof in association with a pharmaceutically acceptable carrier or coating.

The compounds of the present invention may be administered as solid compositions, liquid compositions or other compositions by oral administration and injections, external compositions and, e.g. suppositories, for parenteral administration.

Solid compositions for oral administration, include compressed tablets, pills, dispersible powders, capsules and granules. In such compositions, one or more of the active compound(s) is or are, admixed with at least one inert diluent (e.g. lactose, mannitol, glucose, hydroxypropylcellulose, microcrystalline cellulose, starch, polyvinylpyrrolidone or magnesium metasilicate aluminate). The compositions may also comprise, as is normal practice, additional substances other than inert diluents for example, lubricating agents (e.g. magnesium stearate), disintegrating agents (e.g. cellulose calcium gluconate), and agents to assist dissolution (e.g. glutamic acid or aspartic acid) and stabilizing agents (e.g. lactose).

The tablets or pills may, if desired, be coated with gastric or enteric material (e.g. sugar, gelatin, hydroxypropylcellulose or hydroxypropylmethylcellulose phthalate).

Capsules may be soft or hard.

Liquid compositions for oral administration include pharmaceutically-acceptable emulsions, solutions, suspensions, syrups and elixirs.

In such compositions, one or more of the active compound(s) is or are dissolved or dispersed in inert diluent(s) (e.g. purified water or ethanol).

Besides inert diluents, such compositions may also comprise adjuvants (e.g. wetting agents or suspending agents), sweetening agents, flavouring agents, perfuming agents and preserving agents.

Other compositions for oral administration include spray compositions which may be prepared by known methods and which comprise one or more of the active compound(s).

Spray compositions may comprise additional substances other than inert diluents, e.g. stabilizing agents (e.g. sodium sulfite), isotonic buffer (using, e.g. sodium chloride, sodium citrate, citric acid).

For the preparation of such spray compositions, for example, the method described in United States Patent No. 2868691 or 3095355 may be used.

Injectable compositions for parenteral administration include sterile aqueous or non-aqueous solutions, suspensions and emulsions. In such compositions, one or more of the active compound(s) is or are admixed with at least one inert aqueous diluent(s) (e.g. distilled water for injection or physiological salt solution) or inert non-aqueous diluent(s) [e.g. propylene glycol, polyethylene glycol, olive oil, ethanol, POLYSORBATE 80 (trade mark)]

Injectable compositions may comprise additional materials other than inert diluents e.g. preserving agents, wetting agents, emulsifying agents, dispersing agents, stabilizing agents (e.g. lactose), agents to assist dissolution (e.g. glutamic acid, aspartic acid).

They may be sterilized, for example, by filtration through a bacteria-retaining filter, by incorporation of sterilizing agents in the compositions or by irradiation. They may also be manufactured, for example, by freeze drying, in the form of sterile solid compositions which can be dissolved in sterile water or some other sterile diluent for injection immediately before use.

Other compositions for parenteral administration include liquids for external use, and endermic liniments (e.g. ointments), suppositories and pessaries which comprise one or more of the active compound(s) and may be prepared by known methods.

Compositions for dermal administration, especially for the treatment and prevent of alopecia and acne, include liquids for external use such as lotions, tonics, sprays, solutions, suspensions, emulsions and liniments such as ointments, gels and creams.

Such compositions may comprise one or more active ingredient(s) and at least one inert diluent(s), for example, distilled water, a lower alcohol such as ethanol, a higher alcohol such as cetanol, a poly alcohol such as polyethylene glycol, propylene glycol, a cellulose derivative such as hydroxypropyl cellulose, animal or plant fats, vaseline, wax, silicone, a vegetable oil such as olive oil, a surfactant, or zinc oxide.

Besides inert diluents, such compositions may also comprise adjuvants (e.g. wetting agents, suspending agents, perfuming agents and preserving agents).

### References Examples and Examples

The following Reference Examples and Examples illustrate the spresent invention.

Unless otherwise specified, "IR" were measured by the KBr tablet method.

### Reference Example 1

### Synthesis of 4-(2-nitrophenoxy)butanoic acid ethyl ester

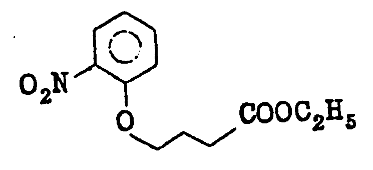

A DMF solution (100 ml) of o-nitrophenol (60 g) was added dropwise with stirring to a DMF suspension (500 ml) of sodium hydride (16.5 g : 62%) cooled with ice. The mixture was stirred for 1 hour at room temperature, and a DMF solution (200 ml) of 4-bromobutanoic acid ethyl ester 84.2 g) was added thereto. The reaction mixture was stirred for 15 hours at 70 °C, and then evaporated. Ethyl acetate (EtOAc) was added to the residue, and the mixture was washed successively with water and a saturated aqueous solution of sodium chloride, dried and evaporated.

The residue was purified by column chromatography on silica gel (EtOAc : hexane = 1 : 3) to give the title compound (77.3 g) having the following physical data:
TLC : Rf 0.35 (EtOAc : hexane = 1 : 2).

### Reference Example 2

### Synthesis of 4-(2-aminophenoxy) butanoic acid ethyl ester

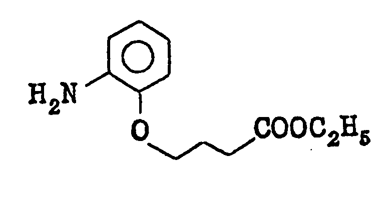

Under an atmosphere of hydrogen, an ethanolic solution (500 ml) of the compound (77g) prepared in Reference Example 1 was added to a suspension in chloroform (100 ml) and ethanol (500 ml) of palladium-carbon (13.1 g : 10%), and the mixture was stirred for 10 hours at room temperature. The reaction mixture was filtered and the filtrate was evaporated. A saturated aqueous solution of sodium bicarbonate (500 ml) was added to ethyl acetate (1000 ml) and the residue and the mixture was stirred at room temperature. The organic layer was washed with a saturated aqueous solution of sodium chloride, dried and evaporated. The residue was purified by column chromatography on silica gel (hexane : EtOAc : CH₂CI₂ = 70 : 15 : 15) to give the title compound (60 g) having the following physical data:
TLC : Rf 0.43
(hexane : EtOAc : CH₂ CI₂ = 2 : 1 : 1).

### Reference Example 3

### Synthesis of 4-isobutylbenzyl bromide

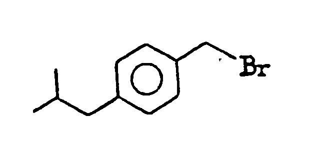

A carbon tetrachloride solution (15 ml) of 4-isobutylbenzyl alcohol (3.58 g) was added to a carbon tetrachloride solution (30 ml) of tribromophosphine (10.3 g) with stirring at room temperature. The reaction mixture was refluxed for two hours and cooled to room temperature. The reaction solution was poured into ice-cold water, and ethyl acetate was added to the solution obtained. The organic layer was separated and washed with a saturated aqueous solution of sodium chloride, dried and evaporated. The residue was purified by column chromatography on silica gel (EtOAc : hexane = 1 : 4) to give the title compound (4.47 g) having the following physical data:
TLC : Rf 0.89 (EtOAc : hexane = 1 : 4).

### Reference Example 4

### Synthesis of 4-(4-isobutylphenylmethoxy)benzoic acid

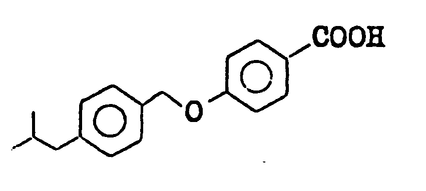

Under an atmosphere or argon, a DMF solution (5 ml) of 4-hydroxybenzoic acid methyl ester (1.67 g) was added dropwise to a DMF suspension (30 ml) of sodium hydride (423 mg : 62%) at room temperature, and the mixture stirred for 10 minutes. A DMF solution (5 ml) of the compound prepared in Reference Example 3 (2.26 g) was added to the reaction solution, and the mixture was stirred for 15 minutes at room temperature. The reaction mixture was diluted with ether, washed successively with water and a saturated aqueous solution of sodium chloride, dried and evaporated. The residue was washed with a small amount of hexane, and dissolved in a mixture of THF (6 ml) and methanol (18 ml). To the solution obtained was added a 2N aqueous solution of sodium hydroxide and the mixture was stirred for one and a half hours at room temperature.

The reaction solution was made acid with 1N aqueous HCI, and the solution obtained was extracted with ethyl acetate.

The organic layer was washed with a saturated aqueous solution of sodium chloride, dried and evaporated to give the title compound (2.15 g) having the following physical data:
TLC : Rf 0.28 (EtOAc : hexane = 1 : 1).

### Reference Example 5

### Synthesis of 4-[2-[4-(4-isobutylphenylmethoxy)benzoylamino]phenoxy butanoic acid ethyl ester

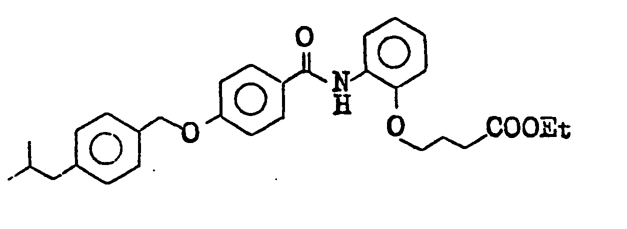

Thionyl chloride (4 ml) was added dropwise to a methylene chloride solution (2 ml) of 4-(4-isobutyl- phenylmethoxy)benzoic acid (227 mg), and the mixture was refluxed for 1 hour and then evaporated to yield an acid chloride.

Under an atmosphere of argon, a solution in a mixture of methylene chloride (5 ml) and pyridine (0.5 ml) of 4-(2-aminophenoxy)butanoic acid ethyl ester (179 mg) was cooled in an ice-bath, and the acid chloride was added dropwise to this cooled solution. After stirring for 10 minutes, the reaction solution was diluted with ethyl acetate. The diluted solution was washed successively with 1 N HCI and a saturated aqueous solution of sodium chloride, dried and evaporated.

The residue was purified by column chromatography on silica gel (EtOAc : hexane = 1 : 4) to give the title compound (374 mg) having the following physical data:
TLC : Rf 0.61 (EtOAc) : hexane = 1 : 1)

### Reference Example 6

### Synthesis of 4-[2-(2-methyl-4-nitrobenzoylamino)phenoxy]butanoic acid ethyl ester

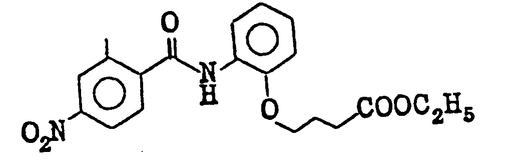

Thionyl chloride (35 ml) was added to a solution in methylene chloride (35 ml) of 2-methyl-4-nitrobenzoic acid (5.4 g), and the mixture was refluxed for one and a half hours. After completion of the reaction, the reaction solution was concentrated. The concentrate in methylene chloride (30 ml) was added dropwise to a mixture of amine (prepared in Reference Example 2 : 7.31 g), pyridine (10 ml) and methylene chloride (60 ml) cooled with ice.

The reaction solution was stirred for 12 hours at room temperature, and then evaporated.

Ethyl acetate was added to the residue, and the mixture was washed successively with water, dilute hydrochloric acid, water and a saturated aqueous solution of sodium chloride, dried and evaporated to give the title compound (11.0 g) having the following physical data:
TLC : Rf 0.83 (CHCIₐ : EtOAc = 10 : 1).

### Reference Example 7

### Synthesis of 4-[2-(4-amino-2-methylbenzoylamino)phenoxy]butanoic acid ethyl ester

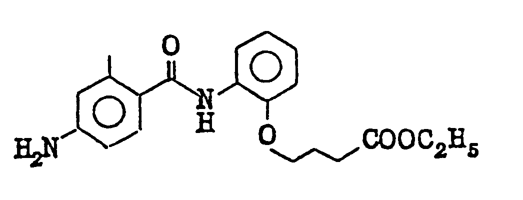

Ethanol (400 ml) and palladium-carbon (10% : 2 g) were added to the nitro compound (20.2 g) prepared in Reference Example 6. Under an atmosphere of hydrogen, the mixture was vigorously stirred for two and a half hours. The reaction solution was filtered and the filtrate was evaporated to give the title compound (18.37 g) having the following physical data:
TLC : Rf 0.28 (CHCl₃ : EtOAC = 10 : 1)

### Reference Example 8

### Synthesis of 4-[2-[2-methyl-4-[N,N-bis(4-propylbenzyl)amino]benzoylamino]phenoxy]butanoic acid ethyl ester

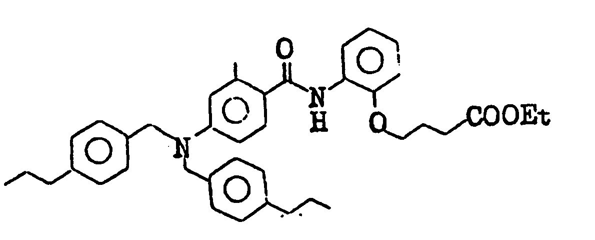

4-Propylbenzyl bromide (318 mg) and sodium bicarbonate (150 mg) were added to a solution of isopropyl alcohol (5 ml) and the amine (356 mg) prepared in Reference Example 7, and the mixture was stirred for one and a half hours at 70 °C. The reaction solution was evaporated, and the residue in ethyl acetate was washed successively with water and a saturated aqueous solution of sodium chloride, and then dried and evaporated.

The residue was purified by column chromatography on silica gel (hexane : EtOAc = 4 : 1) to give the title compound having the following physical data:
TLC : Rf 0.79 (CHCIₐ : EtOAc = 10 : 1).

### Reference Example 8(a)

### Synthesis of 4-[2-[2-methyl-4-[N-(4-propylbenzyl)amino]benzoylamino]phenoxy]butanoic acid ethyl ester

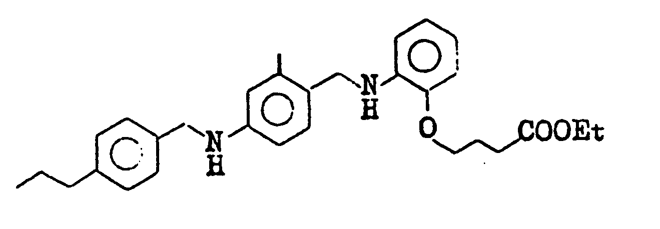

As a by-product in Reference Example 8, the title compound (137 mg) having the following physical data was obtained:
TLC : Rf 0.64 (CHCl₃ : EtOAc = 10 : 1).

### Reference Example 9

### Synthesis of 4-[2-(4-chloromethyl)benzoylamino)phenoxy]butanoic acid ethyl ester

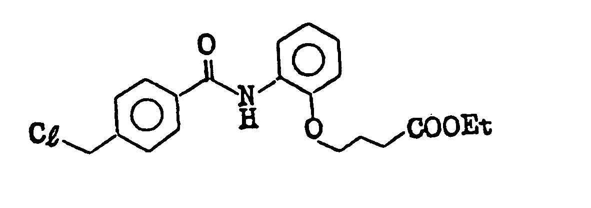

A thionyl chloride solution (6 ml) of p-chloromethylbenzoic acid (1.76 g) was refluxed for 1 hour, and the excess of thionyl chloride was then evaporated off. A methylene chloride solution (10 ml) of the acid chloride thus obtained was added dropwise to a solution in a mixture of pyridine (5 ml) and methylene chloride (20 ml) of 4-(2-aminophenoxy)butanoic acid ethyl ester cooled with ice, and the mixture was stirred for 2 hours at room temperature.

The reaction mixture was diluted with ethyl acetate, and the diluted solution was washed successively with 1N aqueous HCI and a saturated aqueous solution of sodium chloride, dried and evaporated.

The residue was purified by column chromatography on silica gel (hexane : EtOAc = 5 : 1) to give the title compound (3.38 g).

### Reference Example 10

### Synthesis of 4-[2-[4-(5,6,7,8-tetrahydronaphth-1-yl)aminomethylbenzoylamino]phenoxy]butanoic acid ethyl ester

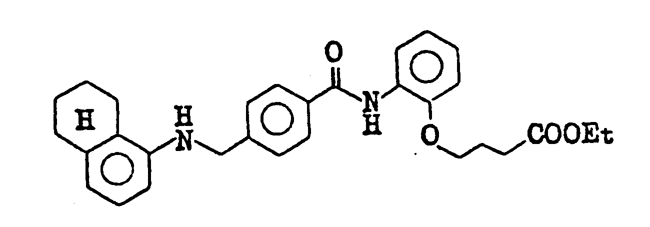

A mixture of the compound prepared in Reference Example 9 (770 mg), 1-amino-5,6,7,8-tetrahydronaphthalene (541 mg), potassium carbonate (276 mg) and DMF (6 ml) was stirred for 6 hours at 70_{°}C.

Under cooling with ice, the reaction mixture was neutralized with 1 N HCI (4 ml), and the neutral solution obtained was extracted with ethyl acetate. The organic layer was washed with water, dried and evaporated. The residue was purified by column chromatography on silica gel (hexane : EtOAc = 4 : 1) to give the title compound (650 mg).

### Reference Example 11

### Synthesis of 4-[2-[4-[N-methyl-N-(5,6,7,8-tetrahydronaphth-1-yl)aminomethylbenzoylamino]phenoxy]butanoic acid ethyl ester

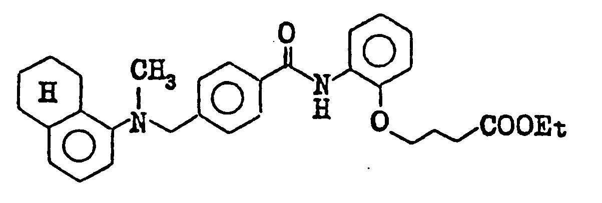

A mixture of the compound prepared in Reference Example 10 (393 mg), methyl iodide (574 mg) and DMF (4 ml) was stirred for 12 hours at room temperature. The reaction solution was evaporated, and ethyl acetate and sodium bicarbonate were added to the concentrate obtained, shaken and separated.

The organic layer was evaporated, and the residue was purified by column chromatography on silica gel (hexane : EtOAc = 4 : 1) to give the title compound (229 mg).

### Example 1

### Synthesis of 4-[2-[4-(4-isobutylphenylmethoxy)benzoylamino]phenoxy]butanoic acid

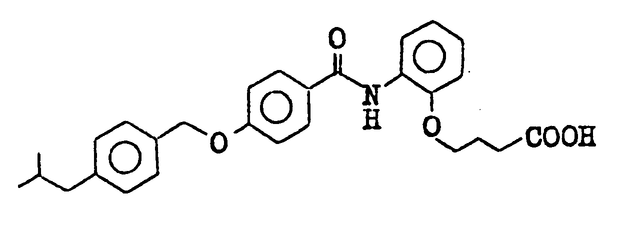

An aqueous solution of sodium hydroxide (2N : 4 ml) was added to a solution in a mixture of THF (4 ml) and methanol (8 ml) of the compound prepared in Reference Example 3 (373 mg), and the mixture was stirred for 1 hour at room temperature. The reaction solution was made acid with 1N HCI, and the solution obtained was extracted with ethyl acetate. The organic layer was washed with a saturated aqueous solution of sodium chloride, dried and evaporated.

The residue was washed with hexane to give the title compound (255 mg) having the following physical data:
TLC : Rf 0.38 (EtOAc : hexane = 2 : 1
mp : 131 - 134°C.

### Example 1 (a) - 1 (i)

By a procedure similar to that described in Reference Examples 1 to 3 and Example 1, using the appropriate alcohol, compounds having the following physical data shown in Tables II, III and IV were obtained.

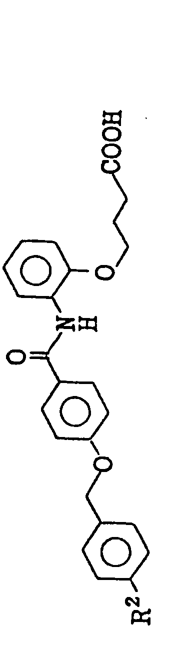

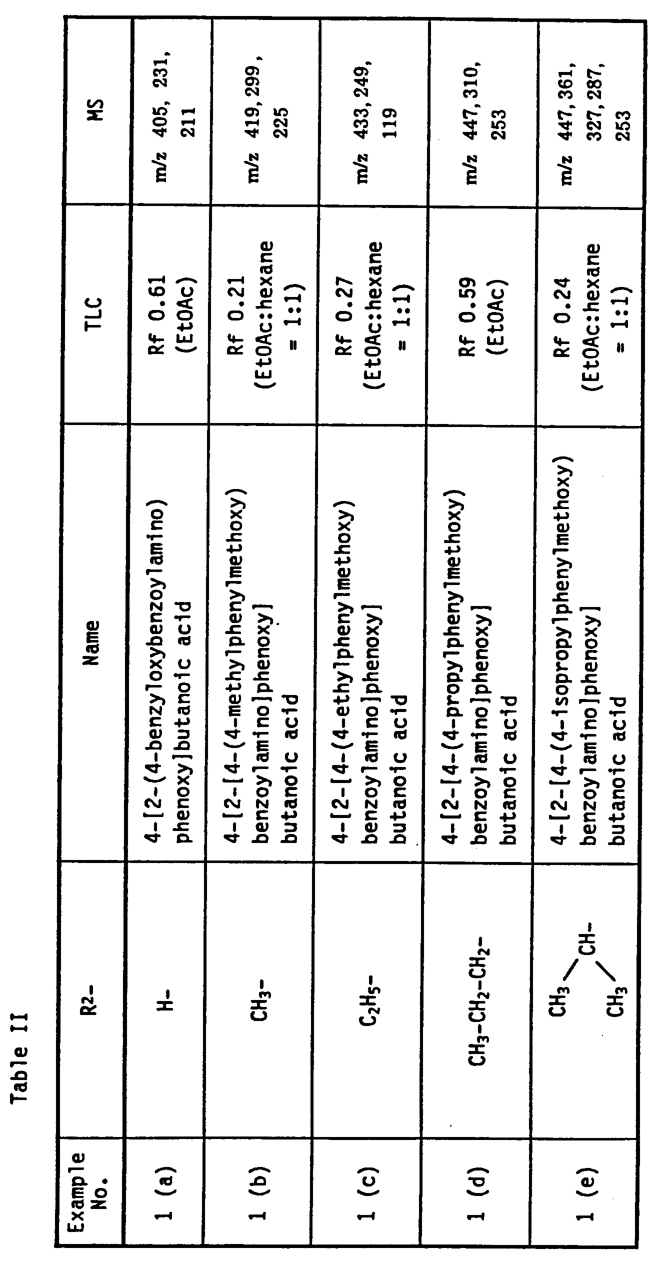

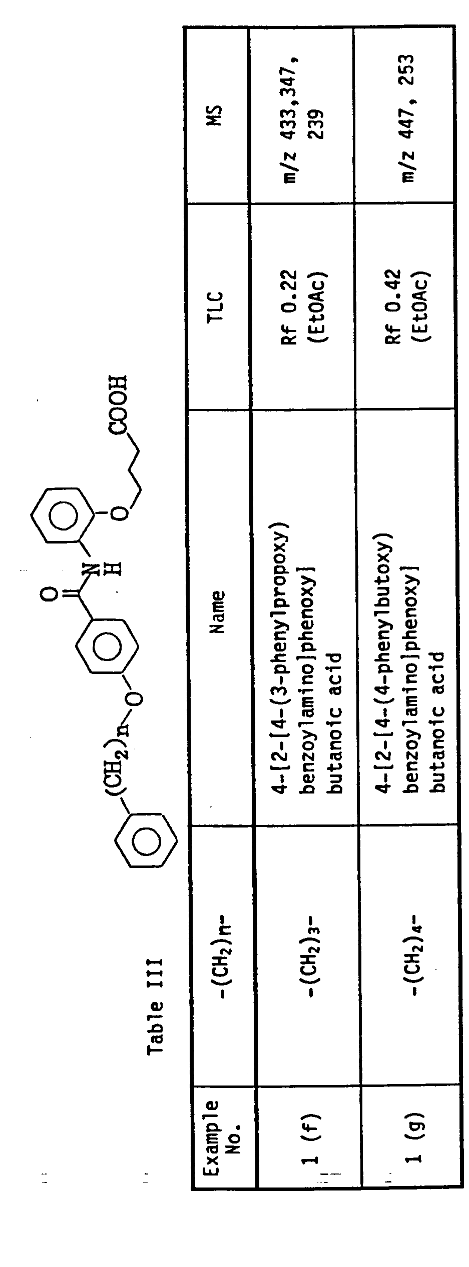

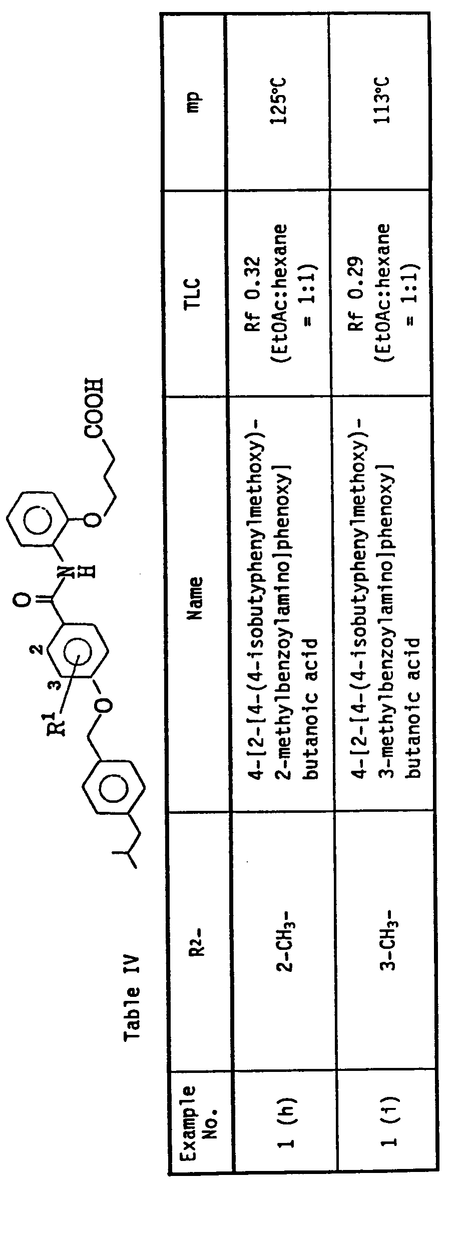

### Example 2

### Synthesis of 4-[2-[2-methyl-4-[N,N-bis(4-propylbenzyl)amino]benzoylamino]phenoxy]butanoic acid

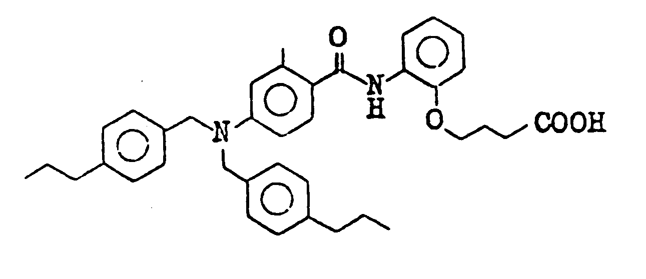

An aqueous solution of lithiumm hydroxide (1 N : 37 ml) was added to a solution in dimethoxyethane (70 ml) of the ester prepared in Reference Example 8 (5.41 g) and the mixture was stirred for one and a half hours at 50 °C, and evaporated. Water was added to the residue, the mixture was made acid with HCI to pH7, and the solution obtained was extracted with ethyl acetate. The organic extract was washed successively with water and a saturated aqueous solution of sodium chloride, dried and evaporated. The residue was purified by recrystallization (hexane-EtOAc) to give the title compound (4.76 g) having the following physical data:
TLC : Rf 0.58 (EtOAc : hexane = 2 : 1
IR : v 3320, 3200-2000, 1710, 1650, 1605, 1515, 1450 cm-¹.

### Example 2(a) - 2(t)

By a procedure similar to that described in Reference Examples 6, 7 and 8 (or 8(a)) and Example 2, compounds having the physical data shown in Tables V and VI were obtained.

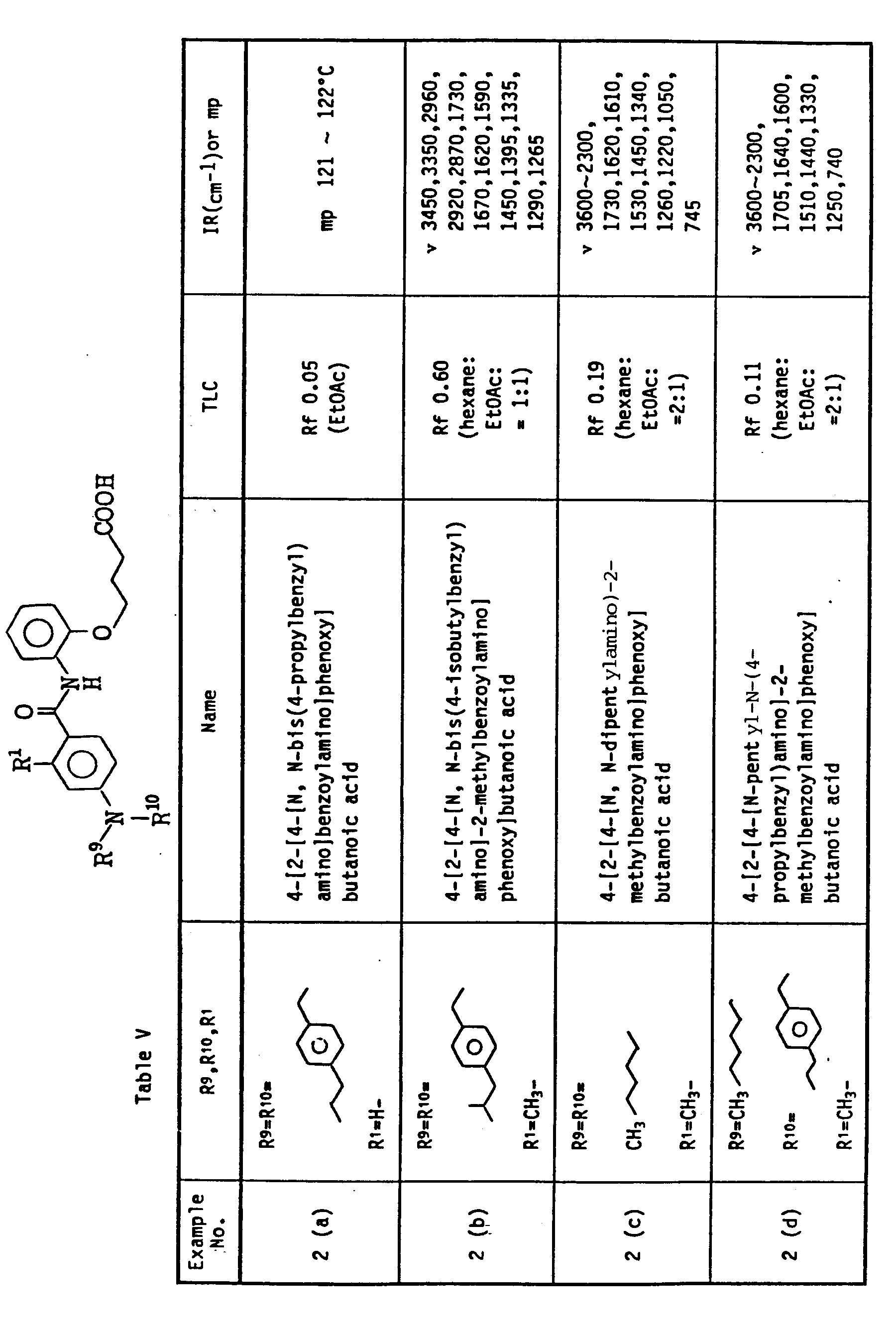

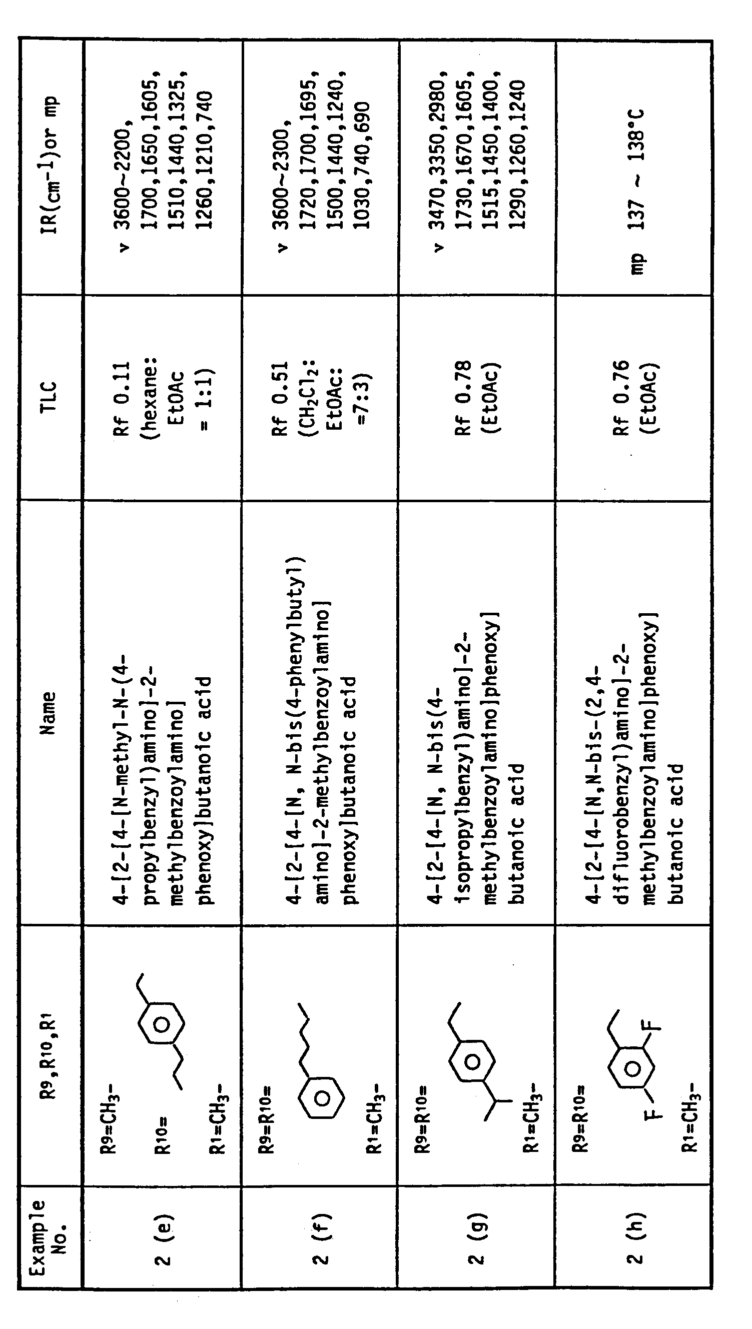

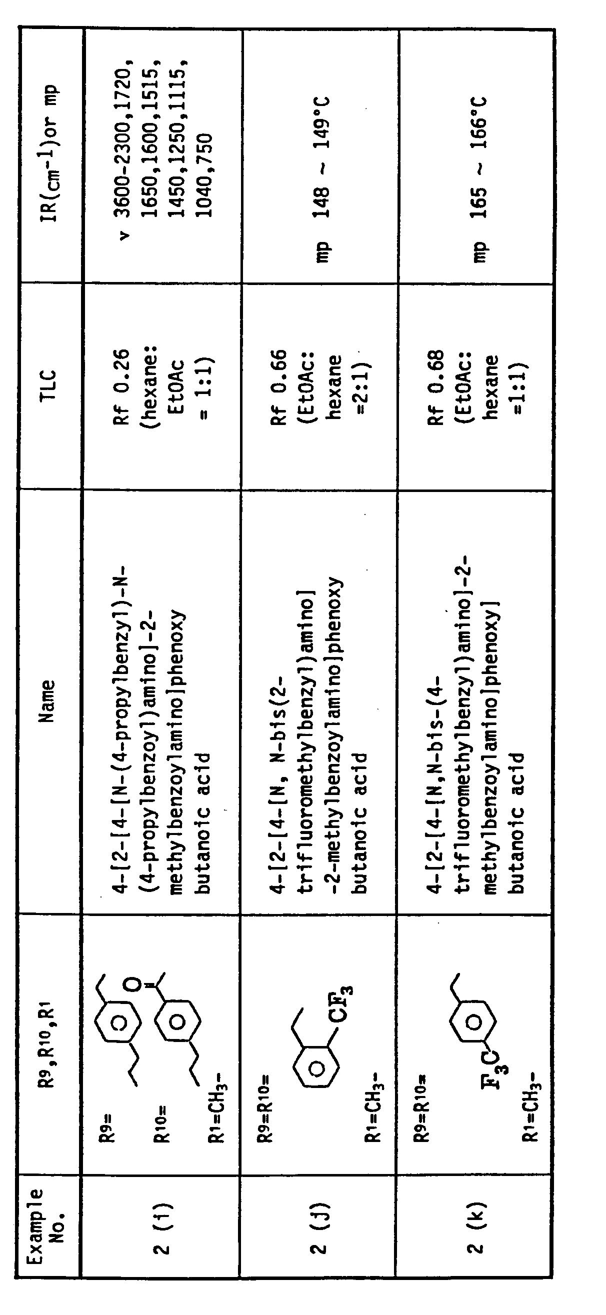

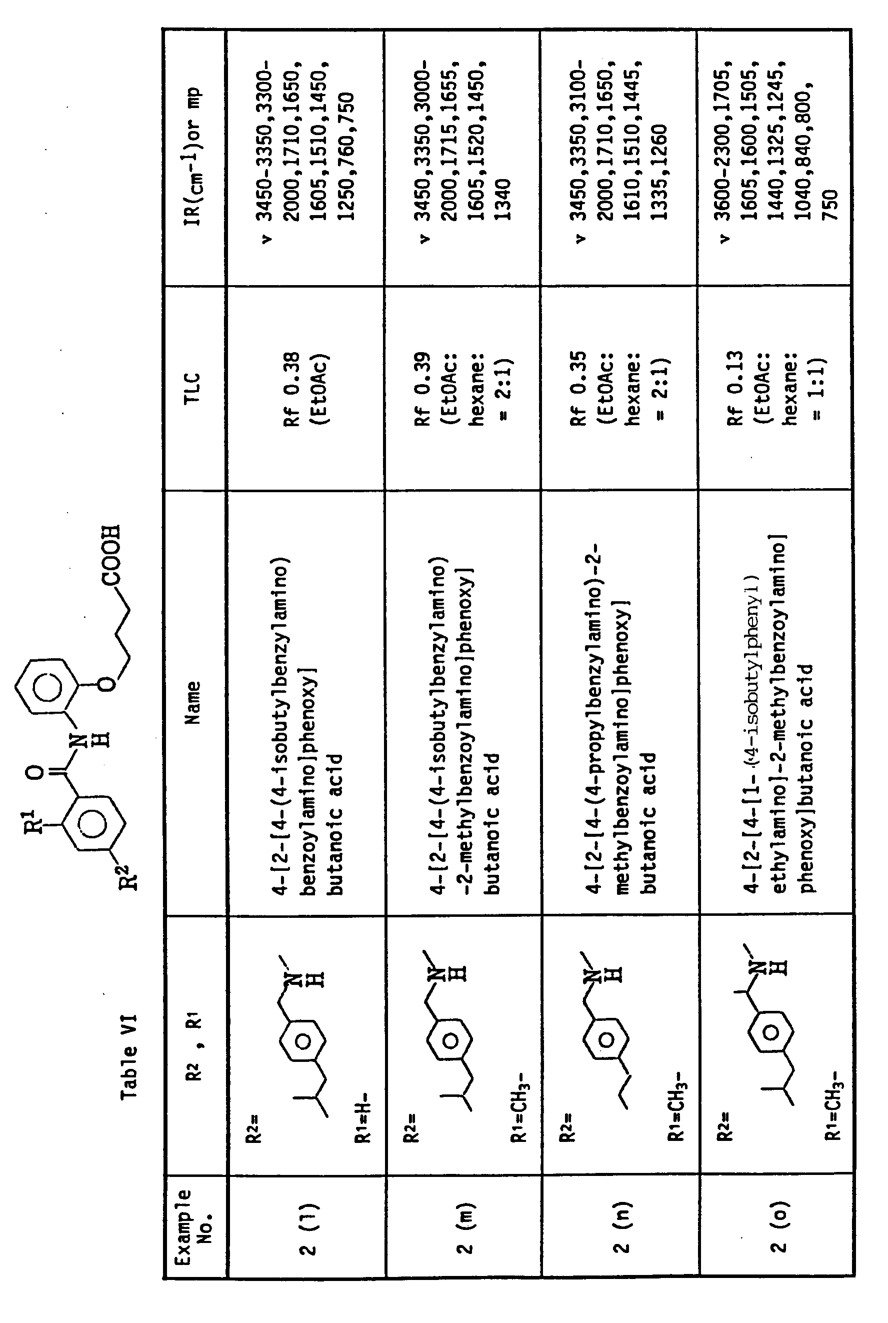

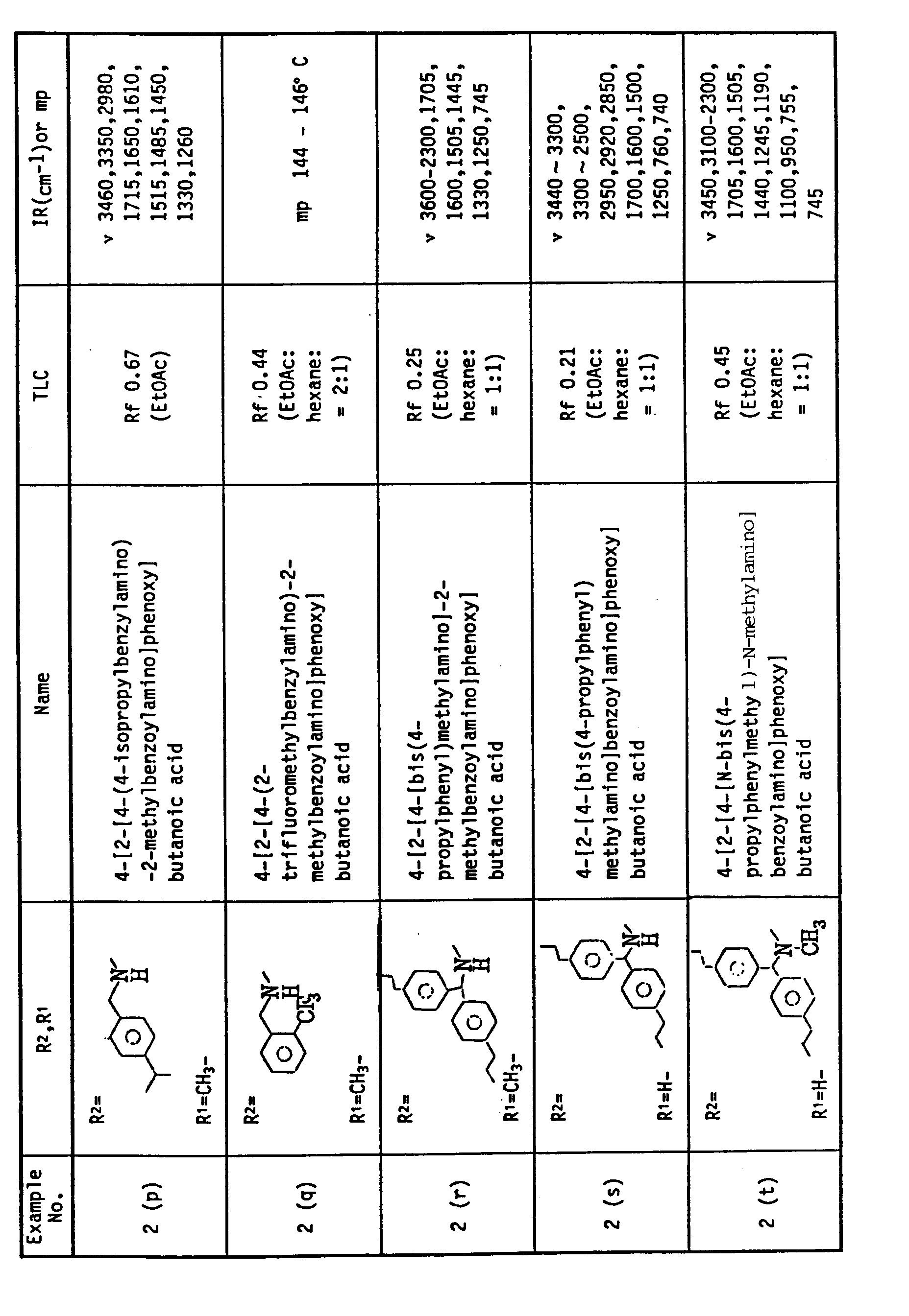

### Example 3(a) - 3(b)

By a procedure similar to that described in Reference Examples 1, 6, 7, 8 and Example 2, compounds having the following physical data shown in Table VII were obtained.

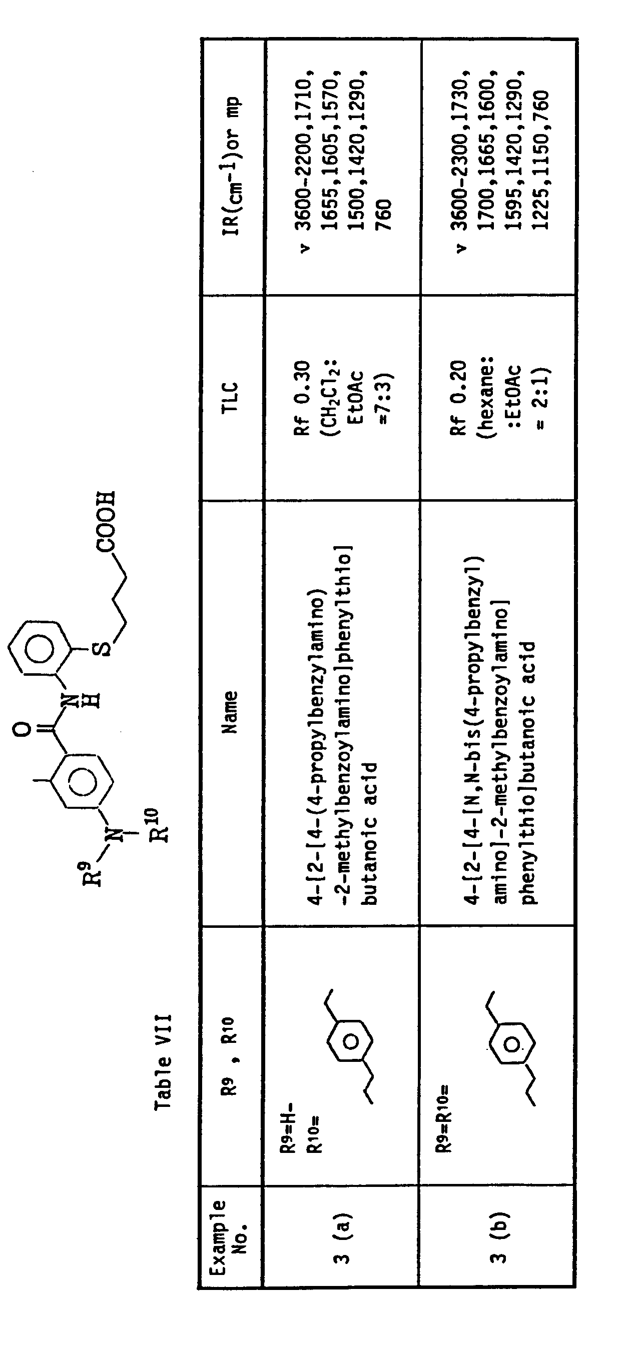

### Example 4(a) - 4(f)

By a procedure similar to that described in Reference Examples 1 to 5 and Example 1, compounds having the following physical data shown in Table VIII were obtained.

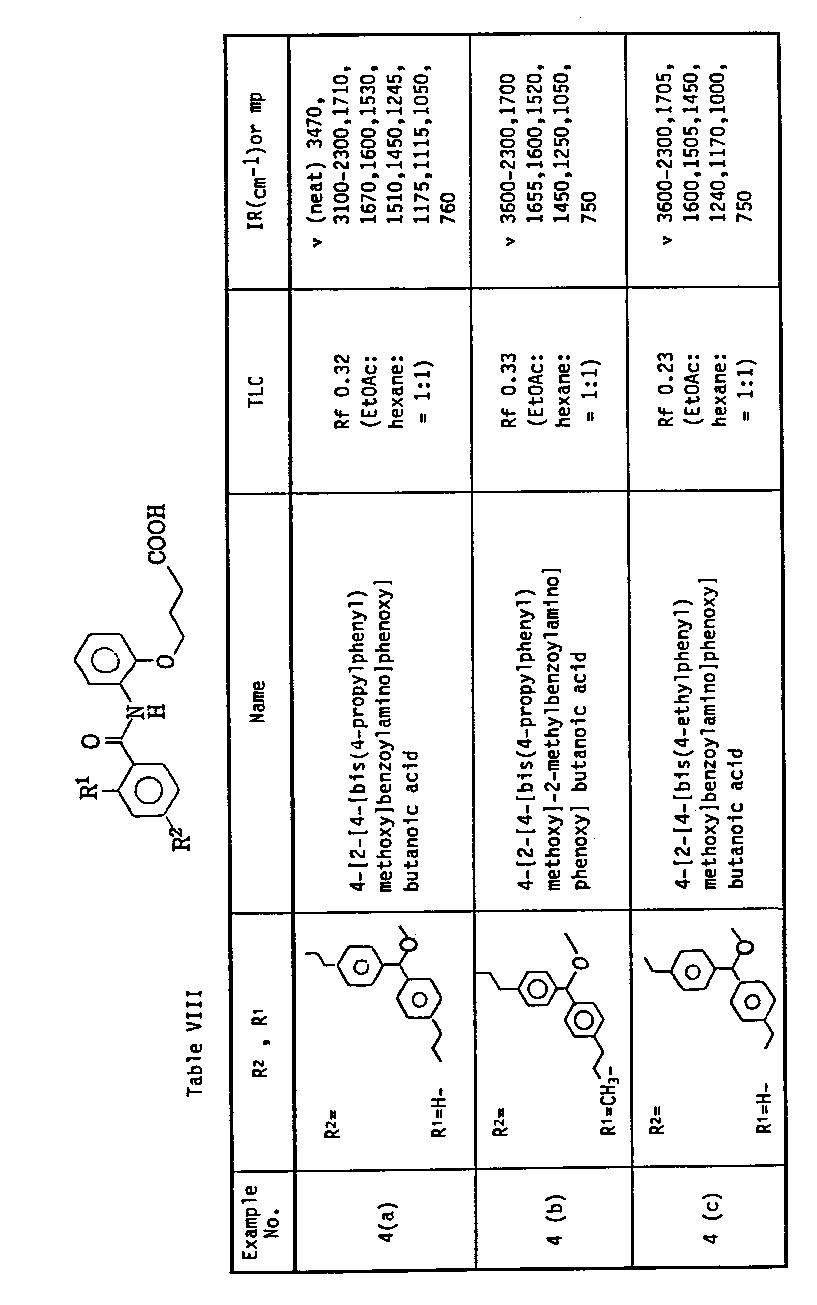

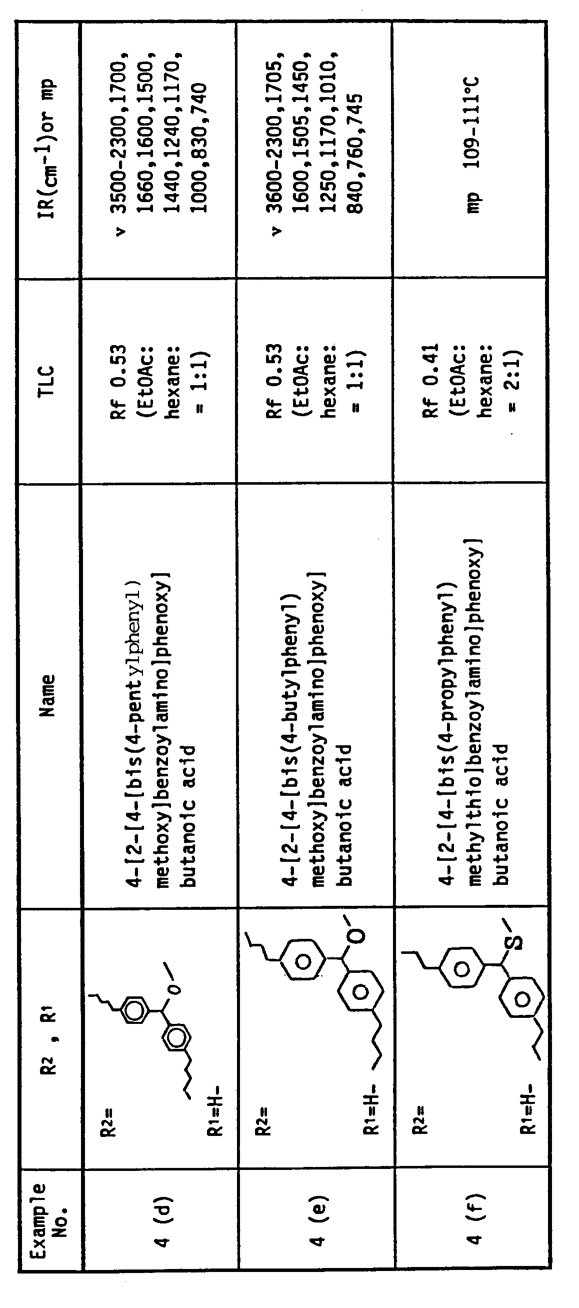

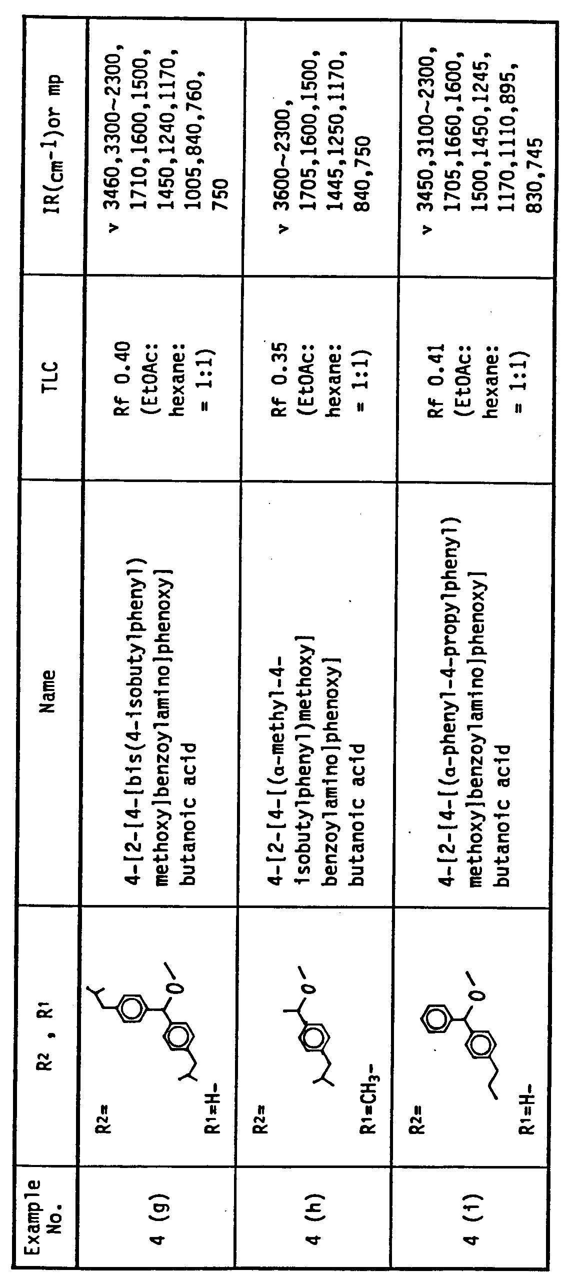

### Example 5

### Synthesis of 4-[2-[4-[N,N-bis(4-propylbenzyl)amino]-2-methylbenzoylamino]phenylsulfinyl]butanoic acid

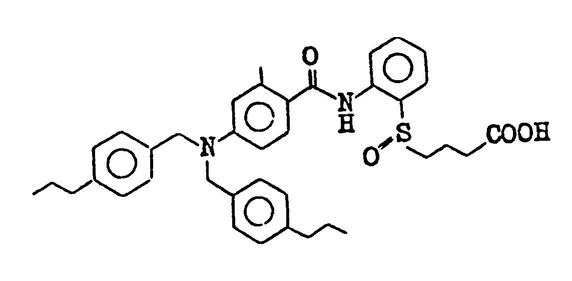

An aqueous solution (3.5 ml) of sodium periodate (137 mg) was added to a solution in methanol (13.5 ml) of the compound prepared in Example 3(b) (260 mg), and the mixture was stirred for 2 hours. Additional aqueous solution (1 ml) of sodium periodate (45 mg) was then added to the reaction mixture, and stirred for 12 hours. The reaction solution was filtered, dried and azeotroped with toluene. The residue was purified by column chromatography (CHCl₃-MeOH) to give the title compound (198 mg) having the following physical data.
TLC : Rf 0.15 (CH₂CI₂ : EtOAc = 7: 3) ;
IR : v 3600-2200, 1720, 1660, 1600, 1500, 1290, 1230, 1150, 760 cm-¹.

### Example 6(a) - 6(c)

By a procedure similar to that described in Reference Example 2, using the compounds prepared in Reference Examples 10 and 11, and by a procedure similar to that described in Reference Examples 9, 10 and Example 1, the title compounds having the following physical data shown in Table IX were obtained.

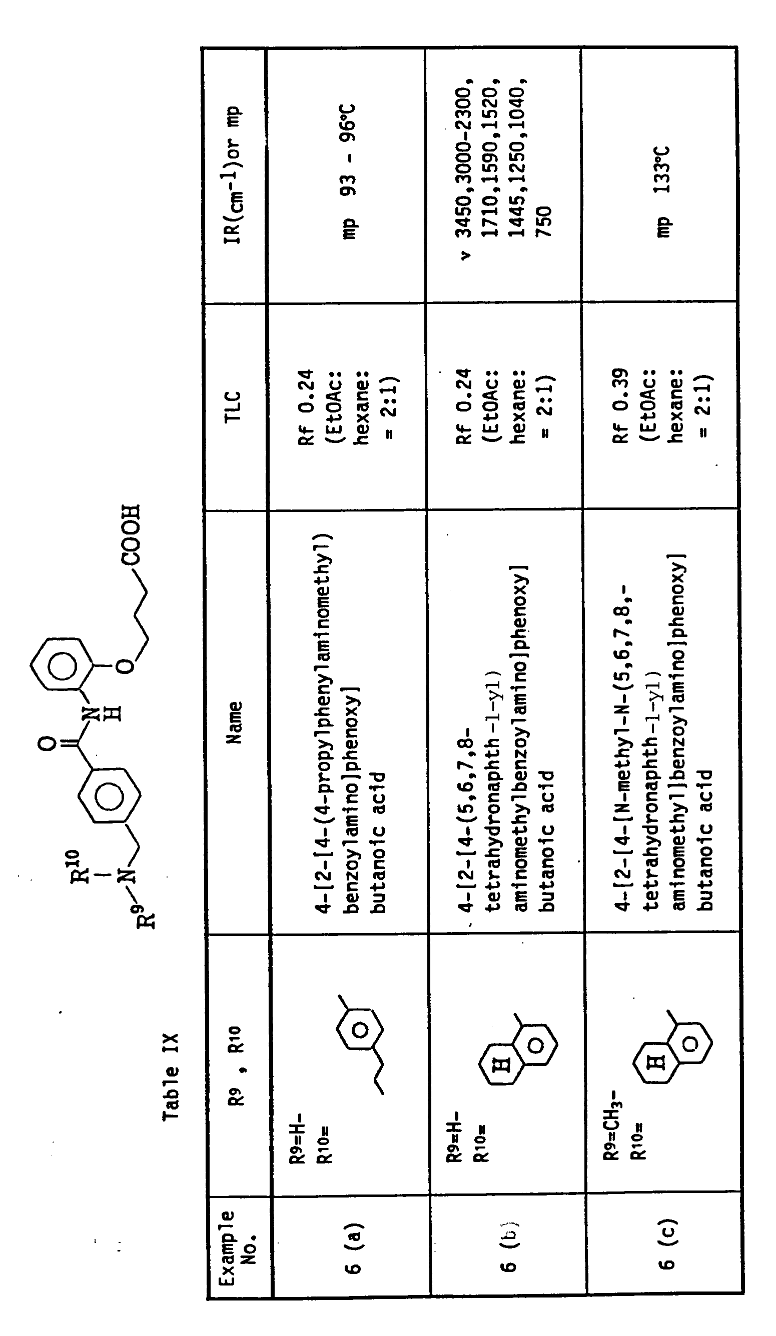

### Formulation Example

The following components were admixed in conventional manner and the mixture punched out to obtain 100 tablets each containing 50 mg of active ingredient.

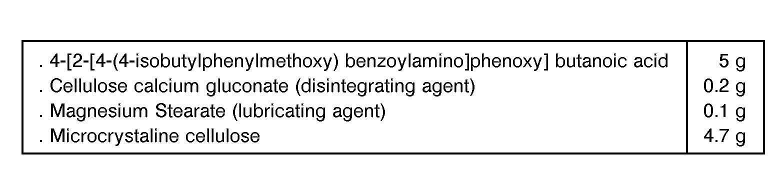

## Claims (Claims for the following Contracting State(s) : AT, BE, CH, DE, FR, GB, IT, LI, LU, NL, SE)

1. A benzoylaminophenoxybutanoic acid derivative of the general formula: 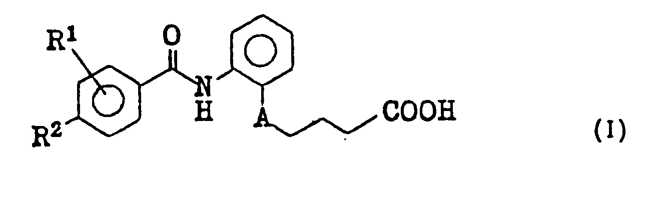 [wherein A represents an oxygen atom, a sulfur atom or a sulfinyl (SO) group,
R¹ represents a hydrogen atom or a methyl group,
R² represents a group of the general formula: 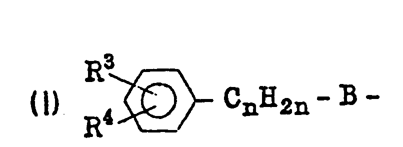 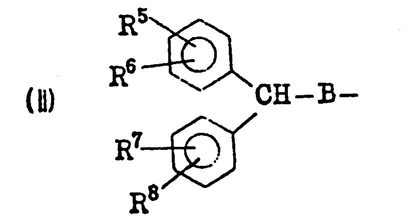 or 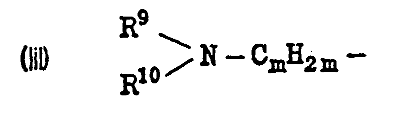 [wherein
B represents an oxygen atom, a sulfur atom or a group of the general formula:
NR" (wherein R" represents a hydrogen atom or an alkyl group of from 1 to 4 carbon atom(s)), R³, R⁴,
R⁵, R⁶, R⁷ and R⁸ represent, independently, a hydrogen atom, an alkyl group of from 1 to 4 carbon atom(s), a halogen atom or a trifluoromethyl group,
m represents 0 or 1,
n represents an integer of from 1 to 4, and
R⁹ and R¹⁰, which may be the same or different, each represents a hydrogen atom, an alkyl group of from 1 to 5 carbon atom(s) or a group of the general formula: 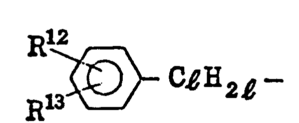 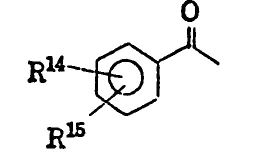 or 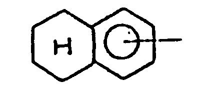
(wherein R¹², R¹³, R14 and R¹⁵ represent, independently, a hydrogen atom, an alkyl group of from 1 to 4 carbon atom(s), a halogen atom or a trifluoromethyl group, and I represents an integer of from 1 to 4), with the proviso that R⁹ and R¹⁰ do not simultaneously represent hydrogen atoms] or a non-toxic salt thereof.

2. A compound according to claim 1, which conforms to the general formula: 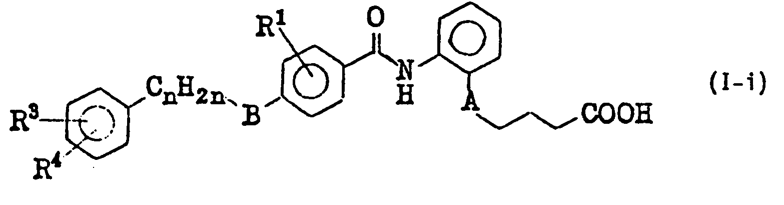 (wherein the symbols are as defined in claim 1).

3. A compound according to claim 1 or 2, wherein B is an oxygen atom.

4. A compound according to claim 1, 2 or 3 which is:
4-[2-[4-(4-isobutylphenylmethoxy)benzoylamino]phenoxy]butanoic acid,
4-[2-(4-benzyloxybenzoylamino)phenoxy]butanoic acid,
4-[2-[4-(4-methylphenylmethoxy)benzoylamino]phenoxy]butanoic acid,
4-[2-[4-(4-ethylphenylmethoxy)benzoylamino]phenoxy]-butanoic acid,
4-[2-[4-(4-propylphenylmethoxy)benzoylamino]phenoxy]butanoic acid,
4-[2-[4-(4-isopropylphenylmethoxy)benzoylamino]phenoxy]butanoic acid,
4-[2-[4-(3-phenylpropoxy)benzoylamino]phenoxy]butanoic acid,
4-[2-[4-(4-phenylbutoxy)benzoylamino]phenoxy]butanoic acid,
4-[2-[4-(4-isobutylphenylmethoxy)-2-methylbenzoylamino]phenoxy]butanoic acid or
4-[2-[4-(4-isobutylphenylmethoxy)-3-methylbenzoylamino]phenoxy]butanoic acid, or a non-toxic salt thereof.

5. A compound according to claim 1 or 2, wherein B is a group of the general formula NR¹¹ (wherein R11 is as defined in claim 1).

6. A compound according to claim 1, 2 or 5, which is:
4-[2-[4-(4-isobutylbenzylamino)benzoylamino]phenoxy]butanoic acid,
4-[2-[4-(4-isobutylbenzylamino)-2-methylbenzoylamino]phenoxy]butanoicacid,
4-[2-[4-(4-propylbenzylamino)-2-methylbenzoylamino]phenoxy]butanoic acid,
4-[2-[4-[l-(4-isobutylphenyl)ethylamino]-2-methylbenzoylamino]phenoxy]butanoic acid,
4-[2-[4-(4-isopropylbenzylamino)-2-methylbenzoylamino]phenoxy]butanoic acid,
4-[2-[4-(4-propylbenzylamino)-2-methylbenzoylamino]phenylthio]butanoic acid or
4-[2-[4-(2-trifluoromethylbenzylamino)-2-methylbenzoylamino]phenoxy]butanoic acid, or a non-toxic salt thereof.

7. A compound according to claim 1, which conforms to the general formula: 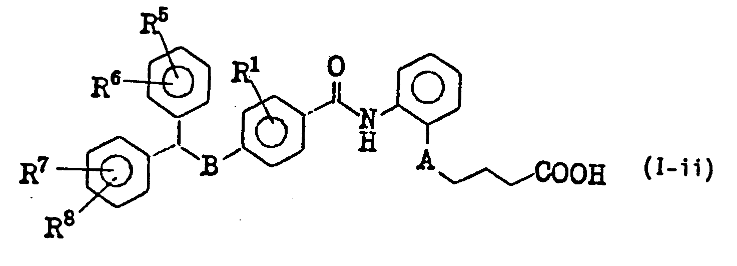 (wherein the symbols are as defined in claim 1).

8. A compound according to claim 1 or 7, wherein B is an oxygen atom.

9. A compound according to claim 1, 7 or 8, which is:
4-[2-[4-[bis(4-propylphenyl)methoxy]benzoylamino]phenoxy]butanoic acid,
4-[2-[4-[bis(4-propylphenyl)methoxy]-2-methylbenzoylamino]phenoxy]butanoic acid,
4-[2-[4-[bis(4-ethylphenyl)methoxy]benzoylamino]phenoxy]butanoic acid,
4-[2-[4-[bis(4-pentylphenyl)methoxy]benzoylamino]phenoxy]butanoic acid, or
4-[2-[4-[bis(4-butylphenyl)methoxy]benzoylamino]phenoxy]butanoic acid.

10. A compound according to claim 1 or 7, wherein B is a group of the general formula NR¹¹ (wherein R¹¹ is as defined in claim 1).

11. A compound according to claim 1, 7 or 10, which is:
4-[2-[4-[bis(4-propylphenyl)methylamino]-2-methylbenzoylamino]phenoxy]butanoic acid,
4-[2-[4-[bis(4-propylphenyl)methylamino]benzoylamino]phenoxy]butanoic acid or
4-[2-[4-[N-bis(4-propylphenylmethyl)-N-methylamino]benzoylamino]phenoxy]butanoic acid or a non-toxic salt thereof.

12. A compound according to claim 1 or 7, wherein B is a sulfur atom.

13. A compound according to claim 1, 7 or 12, which is:
4-[2-[4-[bis(4-propylphenyl)methylthio]benzoylamino]phenoxy]butanoic acid, or a non-toxic salt thereof.

14. A compound according to claim 1, which conforms to the general formula: 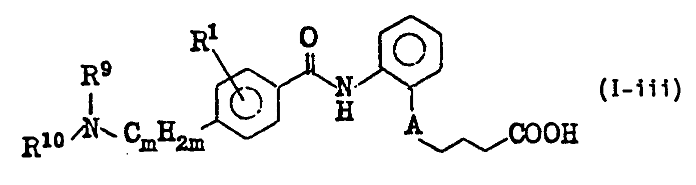 (wherein the symbols are as defined in claim 1).

15. A compound according to claim 1 or 14, wherein R¹ is a hydrogen atom.

16. A compound according to claim 1, 14 or 15, which is:
4-[2-[4-[N,N-bis(4-propylbenzyl)amino]benzoylamino]phenoxy]butanoic acid,
4-[2-[4-(4-propylphenylaminomethyl)benzoylamino]phenoxy]butanoic acid,
4-[2-[4-(5,6,7,8-tetrahydronaphth-1-yl)aminomethylbenzoylamino]phenoxy]butanoic acid or
4-[2-[4-[N-methyl-N-(5,6,7,8-tetrahydronaphth-1-yl)aminomethyl]benzoylamino]phenoxy]butanoic acid, or a non-toxic salt thereof.

17. A compound according to claim 1 or 14 wherein R¹ is a methyl group.

18. A compound according to claim 1, 14 or 17, which is:
4-[2-[2-methyl-4-[N,N-bis(4-propylbenzyl)amino]benzoylamino]phenoxy]butanoic acid,
4-[2-[4-[N,N-bis(4-isobutylbenzyl)amino]-2-methylbenzoylamino]phenoxy]butanoic acid,
4-[2-[4-(N,N-dipentylamino)-2-methylbenzoylamino]phenoxy]butanoic acid,
4-[2-[4-[N-pentyl-N-(4-propylbenzyl)amino]-2-methylbenzoylamino]phenoxy]butanoic acid,
4-[2-[4-[N-methyl-N-(4-propylbenzyl)amino]-2-methylbenzoylamino]phenoxy]butanoic acid,
4-[2-[4-[N,N-bis(4-phenylbutyl)amino]-2-methylbenzoylamino]phenoxy]butanoic acid,
4-[2-[4-[N,N-bis(4-isopropylbenzyl)amino]-2-methylbenzoylamino]phenoxy]butanoic acid,
4-[2-[4-[N,N-bis(4-propylbenzyl)amino]-2-methylbenzoylamino]phenylthio]butanoic acid,
4-[2-[4-[N,N-bis(4-propylbenzyl)amino]-2-methylbenzoylamino]phenylsulfinyl]butanoic acid,
4-[2-[4-[N,N-bis(2,4-difluorobenzyl)amino]-2-methylbenzoylamino]phenoxy]butanoic acid,
4-[2-[4-[N-(4-propylbenzyl)-N-(4-propylbenzoyl)amino]-2-methylbenzoylamino]phenoxy]butanoic acid,
4-[2-[4-[N,N-bis(2-trifluoromethylbenzyl)amino]-2-methylbenzoylamino]phenoxy]butanoic acid or
4-[2-[4-[N,N-bis(4-trifluoromethylbenzyl)amino]-2-methylbenzoylamino]phenoxy]butanoic acid, or a non-toxic salt thereof.

19. A process for the preparation of a benzoylaminophenoxybutanoic acid derivative of the general formula: 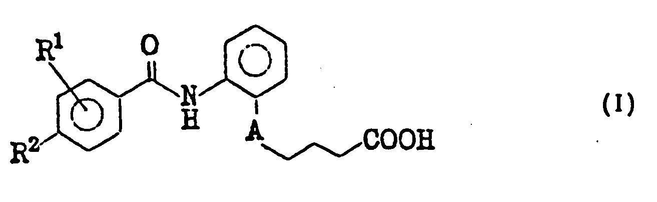 [wherein the various symbols are as defined in claim 1 ] which comprises:
(A) when A represents a sulfur atom or an oxygen atom the saponification of a compound of the general formula: 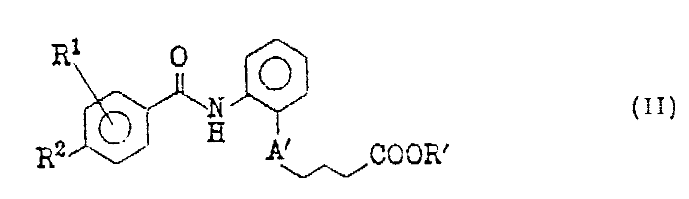 (wherein R' represents an alkyl group of from 1 to 4 carbon atoms, A' represents an oxygen atom or sulfur atom and the other symbols are as defined in claim 1), or
(B) when A in general formula (I) represents a sulfinyl group and the other symbols are as defined in claim 1, oxidizing a compound of the general formula: 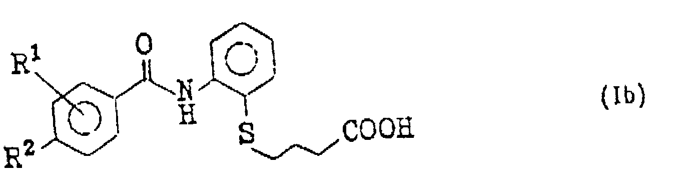 (wherein R¹ and R² are as defined in claim 1) to convert the group -S- to a group -S(=0)-, and then if desired, converting a compound of general formula (I) into a non-toxic salt thereof.

20. A pharmaceutical composition which comprises a benzoylaminophenoxybutanoic acid derivative according to claim 1 or a non-toxic salt thereof in association with a pharmaceutically acceptable carrier or coating.

21. A benzoylaminophenoxybutanoic acid derivative according to claim 1, or a non-toxic salt thereof, for use in the manufacture of a medicament.

22. Use of a benzoylaminophenoxybutanoic acid derivative according to claim 1, or a non-toxic salt thereof, in the manufacture of a medicament for the treatment of alopecia, acne or prostatic hypertrophy.

23. A cosmetic composition which comprises a benzoylaminophenoxybutanoic acid derivative according to claim 1 or a non-toxic salt thereof.

24. Use as a cosmetic product of a benzoylaminophenoxybutanoic acid derivative according to claim 1 or a non-toxic salt thereof.

25. A compound of general formula (II) depicted in claim 19 wherein R' and A' are as defined in claim 19 and the other symbols are as defined in claim 1.

26. A process for the preparation of a compound of general formula (II) as defined in claim 19 which comprises:
(A) the reaction of the carboxy group (COOH) of a compound of the general formula: 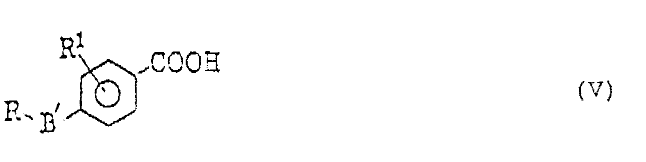 wherein B' represents an oxygen atom or a sulfur atom; R¹ is as defined in claim 1, and R represents a group of the general formula: 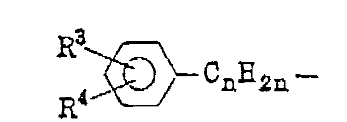 or 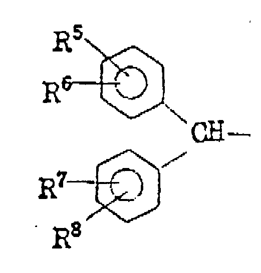 wherein R³, R⁴, R⁵, R⁶, R⁷, and R⁸ are as defined in claim 1, with the amino group (NH₂) of a compound of the general formula: 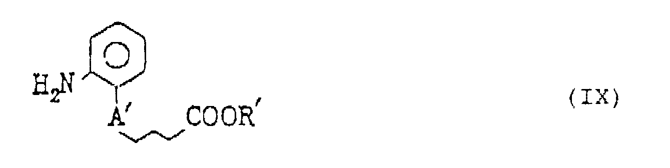 wherein A' represents an oxygen atom or sulfur atom and R' represents an alkyl group of from 1 to 4 carbon atoms, to form an amide (-CONH-) bond;
(B) the reaction of a compound of the general formula 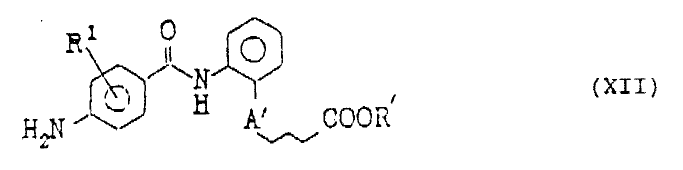 wherein R¹ is as defined in claim 1 and A' and R' are as hereinbefore defined with a compound of the general formula R-X¹, R⁹⁻X⁴ and/or R¹⁰-X⁴, or R¹¹-X⁵ wherein R is as hereinbefore defined, R⁹, R¹⁰ and R¹¹ are as defined in claim 1 and X¹, X⁴ and X⁵ each represents a halogen atom, a tosyl group or a mesyl group; or
(C) the reaction of a compound of the general formula 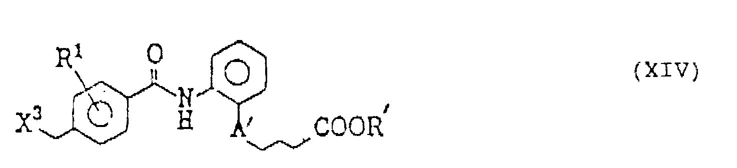 wherein R¹ is as defined in claim 1, A' and R' are as hereinbefore defined and X³ represents a halogen atom, a tosyl group and or a mesyl group, with a compound of the general formula: 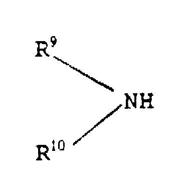 wherein R⁹ and R¹⁰ are as defined in claim 1.

## Claims (Claims for the following Contracting State(s) : ES)

1. A process for the preparation of a benzoylaminophenoxybutanoic acid derivative of the general formula: 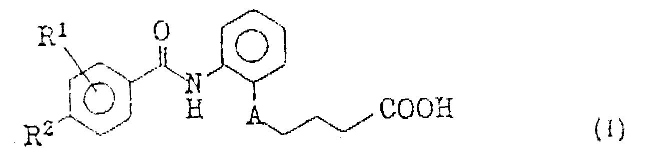 [wherein A represents an oxygen atom, a sulfur atom or a sulfinyl (SO) group,
R¹ represents a hydrogen atom or a methyl group,
R² represents a group of the general formula: 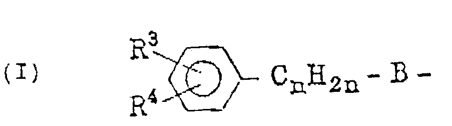 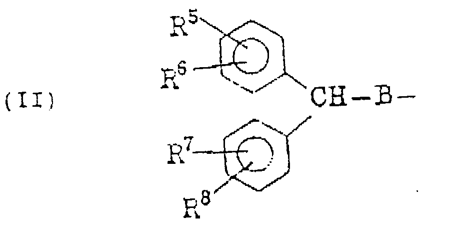 or 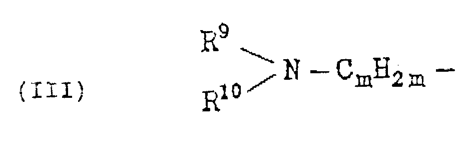 [wherein
B represents an oxygen atom, a sulfur atom or a group of the general formula:
NR¹¹ (wherein R¹¹ represents a hydrogen atom or an alkyl group of from 1 to 4 carbon atom(s)), R³, R⁴, R⁵, R⁶, R⁷ and R⁸ represent, independently, a hydrogen atom, an alkyl group of from 1 to 4 carbon atom(s) , a halogen atom or a trifluoromethyl group,
m represents 0 or 1,
n represents an integer of from 1 to 4, and
R⁹ and R¹⁰, which may be the same or different, each represents a hydrogen atom, an alkyl group of from 1 to 5 carbon atom(s) or a group of the general formula: 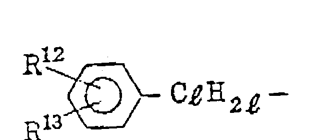 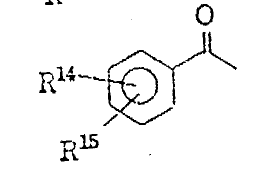 or 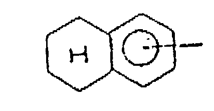 (wherein R¹², R¹³, R¹⁴, R¹⁵ represent, independently, a hydrogen atom, an alkyl group of from 1 to 4 carbon atoms(s), a halogen atom or a trifluoromethyl group, and I represents an integer of from 1 to 4), with the proviso that R⁹ and R¹⁰ do not simultaneously represent hydrogen atoms] or a non-toxic salt thereof, which process comprises:
(A) when A represents a sulfur atom or an oxygen atom, the saponification of a compound of the general formula: 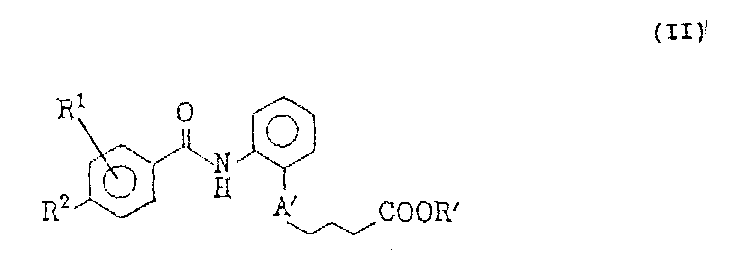 (wherein R' represents an alkyl group of from 1 to 4 carbon atoms, A' represents an oxygen atom or a sulfur atom and the other symbols are as hereinbefore defined), or
(B) when A in general formula (I) represents a sulfinyl group and the other symbols are as hereinbefore defined, oxidizing a compound of the general formula: 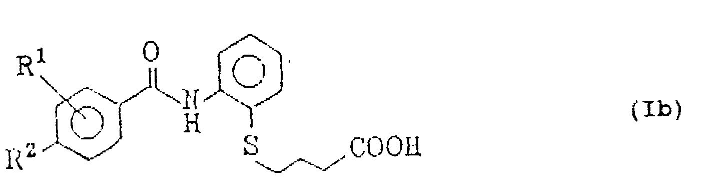 (wherein R¹ and R² are as hereinbefore defined) to convert the group -S- to a group -S(=0)-, and then if desired, converting a compound of general formula (I) into a non-toxic salt thereof.

2. A process according to claim 1, for the preparation of a compound of the general formula: 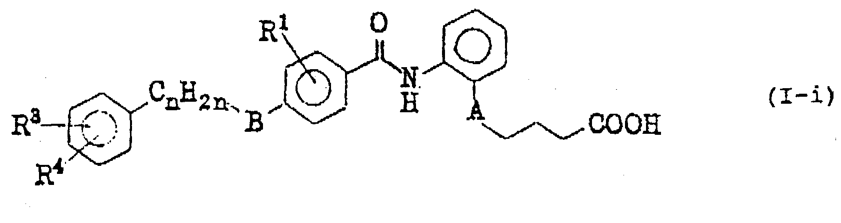 (wherein the symbols are as defined in claim 1).

3. A process according to claim 1 or 2 for the preparation of a compound of the general formula (I), wherein B is an oxygen atom.

4. A process according to claim 1, 2 or 3 for the preparation of a compound of the general formula (I) which is:
4-[2-[4-(4-isobutylphenylmethoxy)benzoylamino]phenoxy]butanoic acid,
4-[2-(4-benzyloxybenzoylamino)phenoxy]butanoic acid,
4-[2-[4-(4-methylphenylmethoxy) benzoylamino]phenoxy]butanoic acid,
4-[2-[4-(4-ethylphenylmethoxy)benzoylamino]phenoxy]-butanoic acid,
4-[2-[4-(4-propylphenylmethoxy)benzoylamino]phenoxy]butanoic acid,
4-[2-[4-(4-isopropylphenylmethoxy)benzoylamino]phenoxy]butanoic acid,
4-[2-[4-(3-phenylpropoxy)benzoylamino]phenoxy]butanoic acid,
4-[2-[4-(4-phenylbutoxy)benzoylamino]phenoxy]butanoic acid,
4-[2-[4-(4-isobutylphenylmethoxy)-2-methylbenzoylamino]phenoxy]butanoic acid or
4-[2-[4-(4-isobutylphenylmethoxy)-3-methylbenzoylamino]phenoxy]butanoic acid, or a non-toxic salt thereof

5. A process according to claim 1 or 2, for the preparation of a compound of the general formula (I) wherein B is a group of the general formula NR¹¹ (wherein R11 is as defined in claim 1).

6. A process according to claim 1, 2 or 5, for the preparation of a compound of the general formula (I) which is:
4-[2-[4-(4-isobutylbenzylamino)benzoylamino]phenoxy]butanoic acid,
4-[2-[4-(4-isobutylbenzylamino)-2-methylbenzoylamino]phenoxy]butanoicacid,
4-[2-[4-(4-propylbenzylamino-2-methylbenzoylamino]phenoxy]butanoic acid,
4-[2-[4-[l-(4-isobutylphenyl)ethylamino]-2-methylbenzoylamino]-phenoxy]butanoic acid,
4-[2-[4-(4-isopropylbenzylamino)-2-methylbenzoylamino]phenoxy]butanoic acid
4-[2-[4-(4-propylbenzylamino)-2-methylbenzoylaminophenylthio]butanoic acid or
4-[2-[4-(2-trifluoromethylbenzylamino)-2-methylbenzoylamino]phenoxy]butanoic acid, or a non-toxic salt thereof

7. A process according to claim 1, for the preparation of a compound of the general formula (I) which conforms to the general formula: 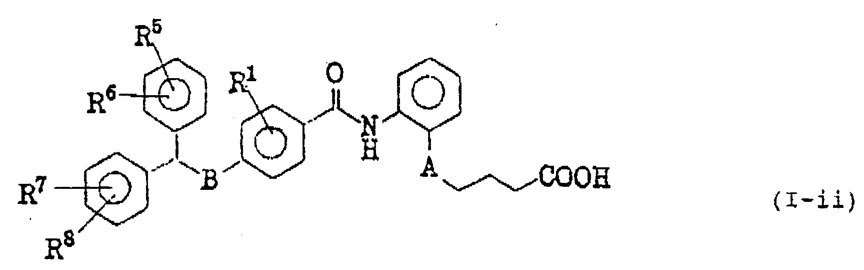 (wherein the symbols are as defined in claim 1).

8. A process according to claim 1 or 7, for the preparation of a compound of the general formula (I) wherein B is an oxygen atom.

9. A process according to claim 1, 7 or 8, for the preparation of a compound of the general formula (I) which is:
4-[2-[4-[bis(4-propylphenyl)methoxy]benzoylamino]phenoxy]butanoic acid,
4-[2-[4-[bis(4-propylphenyl)methoxy]-2-methylbenzoylamino]phenoxy]butanoic acid,
4-[2-[4-[bis(4-ethylphenyl)methoxy]benzoylamino]phenoxy]butanoic acid,
4-[2-[4-[bis(4-pentylphenyl)methoxy]benzoylamino]phenoxy]butanoic acid, or
4-[2-[4-[bis(4-butylphenyl)methoxy] benzoylamino] phenoxy]butanoic acid, or a non-toxic salt thereof.

10. A process according to claim 1 or 7, for the preparation of a compound of the general formula (I) wherein B is a group of the general formula NR¹¹ (wherein R11 is as defined in claim 1).

11. A process according to claim 1, 7 or 10, for the preparation of a compound of the general formula (I) which is:
4-[2-[4-[bis(4-propylphenyl)methylamino]-2-methylbenzoylamino]phenoxy]butanoic acid,
4-[2-[4-bis (4-propylphenyl)methylamino]benzoylamino]phenoxy]butanoic acid or
4-[2-[4-[N-bis(4-propylphenylmethyl)-N-methylamino]benzoylamino]phenoxy]butanoic acid or a non-toxic salt thereof.

12. A process according to claim 1 or 7, for the preparation of a compound of the general formula (I) wherein B is a sulfur atom.

13. A process according to claim 1, 7 or 12, for the preparation of a compound of the general formula (I) which is:
4-[2-[4-[bis(4-propylphenyl)methylthio]benzoylamino]phenoxy]butanoic acid, or a non-toxic salt thereof.

14. A process according to claim 1, for the preparation of a compound of the general formula (I) which conforms to the general formula: 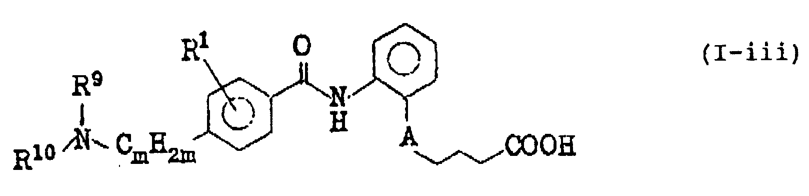 (wherein the symbols are as defined in claim 1).

15. A process according to claim 1 or 14, for the preparation of a compound of the general formula I wherein R¹ is a hydrogen atom.

16. A process according to claim 1, 14, or 15, for the preparation of a compound of the general formula I which is:
4-[2-[4-[N,N-bis(4-propylbenzyl)amino]benzoylamino]phenoxy]butanoic acid,
4-[2-[4-(4-propylphenylaminomethyl)benzoylamino] phenoxy]butanoic acid,
4-[2-[4-(5,6,7,8-tetrahydronaphth-1-yl)aminomethylbenzoylamino]phenoxy]butanoic acid or
4-[2-[4-[N-methyl-N-(5,6,7,8-tetrahydronaphth-1-yl) aminomethyl]benzoylamino]phenoxy]butanoic acid, or a non-toxic salt thereof.

17. A process according to claim 1 or 14 for the preparation of a compound of the general formula I wherein R¹ is a methyl group.

18. A process according to claim 1, 14 or 17, for the preparation of a compound of the general formula I which is:
4-[2-[2-methyl-4-[N,N-bis(4-propylbenzyl)amino]benzoylamino]phenoxy]butanoic acid,
4-[2-[4-[N,N-bis(4-isobutylbenzyl)amino]-2-methylbenzoylamino]phenoxy]butanoic acid,
4-[2-[4-[N,N-dipentylamino)-2-methylbenzoylamino]phenoxy]butanoic acid,
4-[2-[4-[N-pentyl-N-(4-propylbenzyl)amino]-2-methylbenzoylamino]phenoxy]butanoic acid,
4-[2-[4-[N-methyl-N-(4-propylbenzyl)amino]-2-methylbenzoylamino]phenoxy]butanoic acid,
4-[2-[4-N,N-bis(4-phenylbutyl)amino]-2-methylbenzoylamino]phenoxy]butanoic acid,
4-[2-[4-[N,N-bis(4-isopropylbenzyl)amino]-2-methylbenzoylamino]phenoxy]butanoic acid,
4-[2-[4-[N,N-bis(4-propylbenzyl)amino]-2-methylbenzoylamino]phenylthio]butanoic acid,
4-[2-[4-[N,N-bis(4-propylbenzyl)amino]-2-methylbenzoylamino]phenylsulfinyl]butanoic acid
4-[2-[4-[N,N-bis(2,4-difluorobenzyl)amino]-2-methtylbenzoylamino]phenoxy]butanoic acid,
4-[2-[4-[N-(4-propylbenzyl)-N-(4-propylbenzoyl)amino]-2-methylbenzoylamino]phenoxy]butanoic acid,
4-[2-[4-[N,N-bis(2-trifluoromethylbenzyl)amino]-2-methylbenzoylamino]phenoxy]butanoic acid or
4-[2-[4-[N,N-bis(4-trifluoromethylbenzyl)amino]-2-methylbenzoylamino]phenoxy]butanoic acid, or a non-toxic salt thereof.

19. A process according to any one of the preceding claims followed by the step of formulating a benzoylaminophenoxybutanoic acid derivative according to claim 1 or a non-toxic salt thereof with a pharmaceutically acceptable carrier or coating.

20. Use of a benzoylaminophenoxybutanoic acid derivative according to claim 1, or a non-toxic salt thereof, in the manufacture of a medicament for the treatment of alopecia, acne or prostatic hypertrophy.

21. Use as a cosmetic product of a benzoylaminophenoxybutanoic acid derivative according to claim 1 or a non-toxic salt thereof.

22. A process for the preparation of a compound of general formula (II) as defined in claim 1 which comprises:
(A) the reaction of the carboxy group (COOH) of a compound of the general formula: 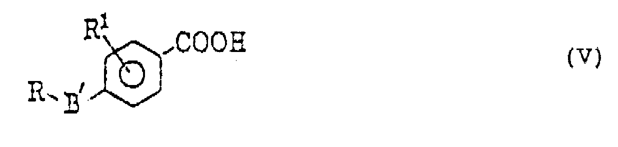 wherein B' represents an oxygen atom or a sulfur atom, R¹ is as defined in claim 1, and R represents a group of the general formula: 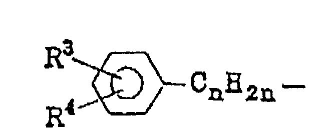 or 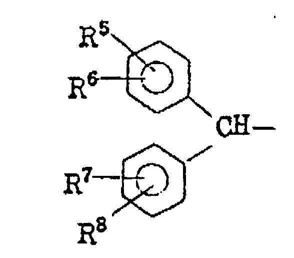 wherein R³' R⁴, R⁵, R⁶, R⁷, and R⁸ are as defined in claim 1, with the amino group (NH₂) of a compound of the general formula: 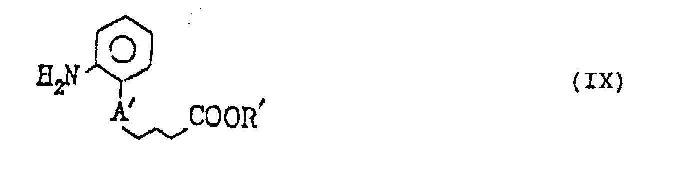 wherein A' represents an oxygen atom or sulfur atom and R' represents an alkyl group of from 1 to 4 carbon atoms, to form an amide (-CONH-) bond;
(B) the reaction of a compound of the general formula 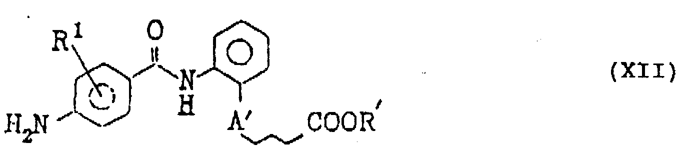 wherein R¹ is as defined in claim 1 and A' and R' are as hereinbefore defined with a compound of the general formula R-X¹, R⁹⁻X⁴ and/or R¹⁰-X⁴, or R¹¹-X⁵ wherein R is as hereinbefore defined, R⁹, R¹⁰ and R¹¹ are as defined in claim 1 and X¹, X⁴ and X⁵ each represents a halogen atom, a tosyl group or a mesyl group; or
(C) the reaction of a compound of the general formula 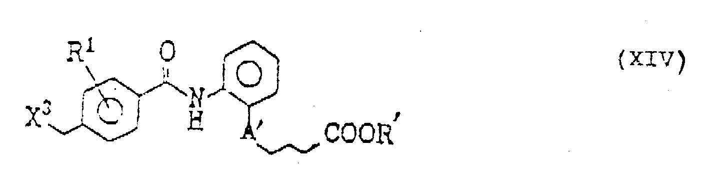 wherein R¹ is as defined in claim 1, A' and R' are as hereinbefore defined and X³ represents a halogen atom, a tosyl group and or a mesyl group, with a compound of the general formula: 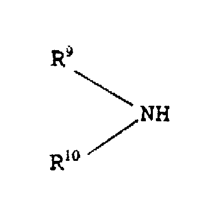 wherein R⁹ and R¹⁰ are as defined in claim 1.

## Claims (Claims for the following Contracting State(s) : GR)

1. A process for the preparation of a benzoylaminophenoxybutanoic acid derivative of the general formula: 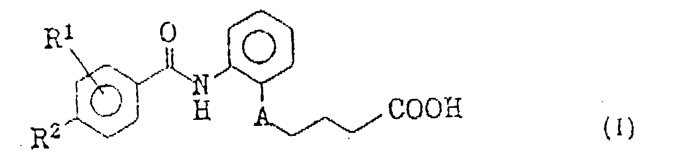 [wherein A represents an oxygen atom, a sulfur atom or a sulfinyl (SO) group,
R¹ represents a hydrogen atom or a methyl group,
R² represents a group of the general formula: 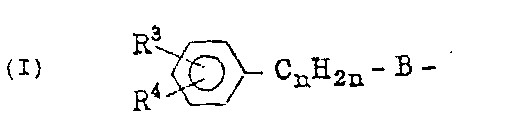 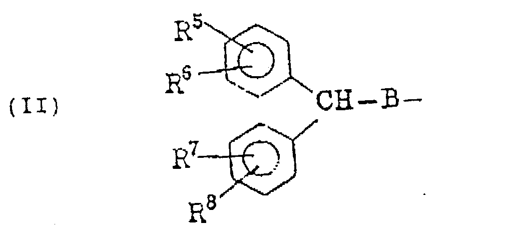 or 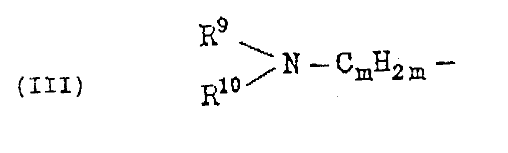 [wherein
B represents an oxygen atom, a sulfur atom or a group of the general formula:
NR¹¹ (wherein R¹¹ represents a hydrogen atom or an alkyl group of from 1 to 4 carbon atom(s)) , R³, R⁴, R⁵, R⁶, R⁷ and R⁸ represent, independently, a hydrogen atom, an alkyl group of from 1 to 4 carbon atom(s) , a halogen atom or a trifluoromethyl group,
m represents 0 or 1,
n represents an integer of from 1 to 4, and
R⁹ and R¹⁰, which may be the same or different , each represents a hydrogen atom, an alkyl group of from 1 to 5 carbon atom ( s) or a group of the general formula: 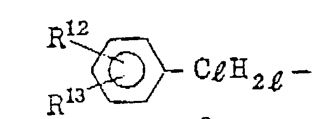 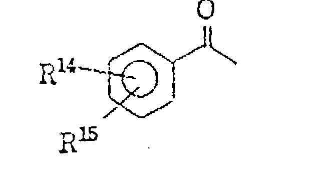 or 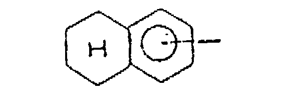 (wherein R¹², R¹³, R¹⁴ and R¹⁵ represent, independently, a hydrogen atom, an alkyl group of from 1 to 4 carbon atom(s), a halogen atom or a trifluoromethyl group, and I represents an integer of from 1 to 4), with the proviso that R⁹ and R¹⁰ do not simultaneously represent hydrogen atoms] or a non-toxic salt thereof, which process comprises:
(A) when A represents a sulfur atom or an oxygen atom, the saponification of a compound of the general formula: 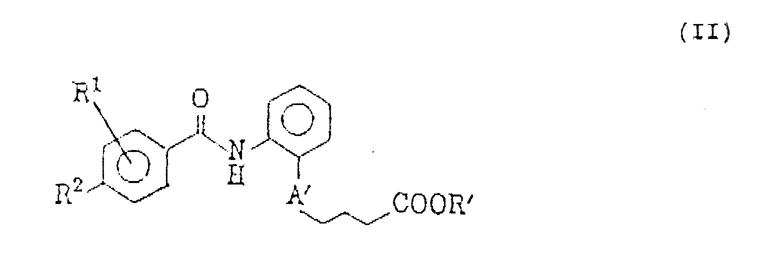 (wherein R' represents an alkyl group of from 1 to 4 carbon atoms, A' represents an oxygen atom or a sulfur atom and the other symbols are as hereinbefore defined), or
(B) when A in general formula (I) represents a sulfinyl group and the other symbols are as hereinbefore defined, oxidizing a compound of the general formula: 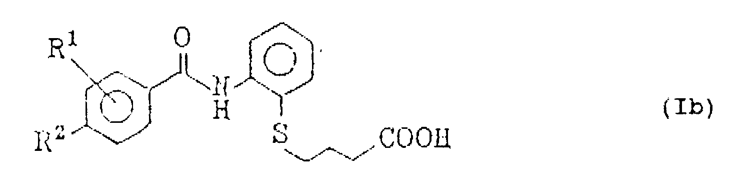 (wherein R¹ and R² are as hereinbefore defined) to convert the group -S- to a group -S(=0)-, and then if desired, converting a compound of general formula (I) into a non-toxic salt thereof.

2. A process according to claim 1, for the preparation of a compound of the general formula: 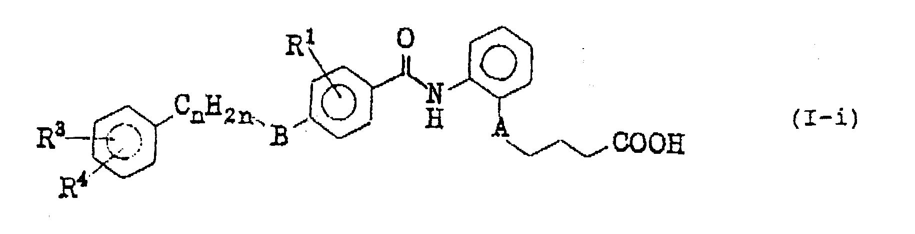 (wherein the symbols are as defined in claim 1).

3. A process according to claim 1 or 2 for the preparation of a compound of the general formula (I), wherein B is an oxygen atom.

4. A process according to claim 1, 2 or 3 for the preparation of a compound of the general formula (I) which is:
4-[2-[4-(4-isobutylphenylmethoxy)benzoylamino]phenoxy]butanoic acid,
4-[2-(4-benzyloxybenzoylamino)phenoxy]butanoic acid,
4-[2-[4-(4-methylphenylmethoxy) benzoylamino]phenoxy]butanoic acid,
4-[2-[4-(4-ethylphenylmethoxy)benzoylamino]phenoxy]-butanoic acid,
4-[2-[4-(4-propylphenylmethoxy)benzoylamino]phenoxy]butanoic acid,
4-[2-[4-(4-isopropylphenylmethoxy)benzoylamino]phenoxy]butanoic acid,
4-[2-[4-(3-phenylpropoxy)benzoylamino]phenoxy]butanoic acid,
4-[2-[4-(4-phenylbutoxy)benzoylamino]phenoxy]butanoic acid,
4-[2-[4-(4-isobutylphenylmethoxy)-2-methylbenzoylamino]phenoxy]butanoic acid or
4-[2-[4-(4-isobutylphenylmethoxy)-3-methylbenzoylamino]phenoxy]butanoic acid, or a non-toxic salt thereof

5. A process according to claim 1 or 2, for the preparation of a compound of the general formula (I) wherein B is a group of the general formula NR¹¹ (wherein R11 is as defined in claim 1).

6. A process according to claim 1, 2 or 5, for the preparation of a compound of the general formula (I) which is:
4-[2-[4-(4-isobutylbenzylamino)benzoylamino]phenoxy]butanoic acid,
4-[2-[4-(4-isobutylbenzylamino)-2-methylbenzoylamino]phenoxy]butanoic acid,
4-[2-[4-(4-propylbenzylamino-2-methylbenzoylamino]phenoxy]butanoic acid,
4-[2-[4-[l-(4-isobutylphenyl)ethylamino]-2-methylbenzoylamino]-phenoxy]butanoic acid,
4-[2-[4-(4-isopropylbenzylamino)-2-methylbenzoylamino]phenoxy]butanoic acid
4-[2-[4-(4-propylbenzylamino)-2-methylbenzoylaminophenylthio]butanoic acid or
4-[2-[4-(2-trifluoromethylbenzylamino)-2-methylbenzoylamino]phenoxy]butanoic acid, or a non-toxic salt thereof

7. A process according to claim 1, for the preparation of a compound of the general formula (I) which conforms to the general formula: 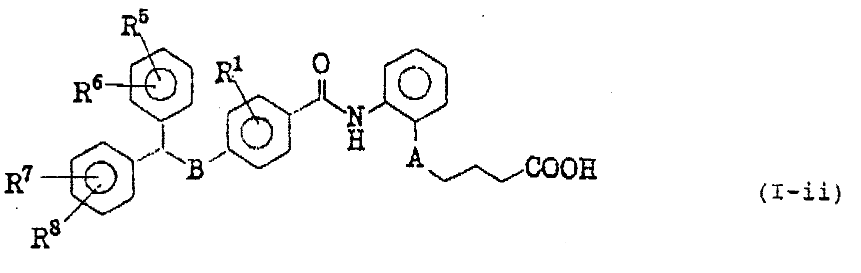 (wherein the symbols are as defined in claim 1).

8. A process according to claim 1 or 7, for the preparation of a compound of the general formula (I) wherein B is an oxygen atom.

9. A process according to claim 1, 7 or 8, for the preparation of a compound of the general formula (I) which is:
4-[2-[4-[bis(4-propylphenyl)methoxy]benzoylamino]phenoxy]butanoic acid,
4-[2-[4-[bis(4-propylphenyl)methoxy]-2-methylbenzoylamino]phenoxy]butanoic acid,
4-[2-[4-[bis(4-ethylphenyl)methoxy]benzoylamino]phenoxy]butanoic acid,
4-[2-[4-[bis(4-pentylphenyl)methoxy]benzoylamino]phenoxy]butanoic acid, or
4-[2-[4-[bis(4-butylphenyl)methoxy] benzoylamino] phenoxy]butanoic acid, or a non-toxic salt thereof.

10. A process according to claim 1 or 7, for the preparation of a compound of the general formula (I) wherein B is a group of the general formula NR¹¹ (wherein R11 is as defined in claim 1).

11. A process according to claim 1, 7 or 10, for the preparation of a compound of the general formula (I) which is:
4-[2-[4-[bis(4-propylphenyl)methylamino]-2-methylbenzoylamino]phenoxy]butanoic acid,
4-[2-[4-bis (4-propylphenyl)methylamino]benzoylamino]phenoxy]butanoic acid or
4-[2-[4-[N-bis(4-propylphenylmethyl)-N-methylamino]benzoylamino]phenoxy]butanoic acid or a non-toxic salt thereof.

12. A process according to claim 1 or 7, for the preparation of a compound of the general formula (I) wherein B is a sulfur atom.

13. A process according to claim 1, 7 or 12, for the preparation of a compound of the general formula (I) which is:
4-[2-[4-[bis(4-propylphenyl)methylthio]benzoylamino]phenoxy]butanoic acid, or a non-toxic salt thereof.

14. A process according to claim 1, for the preparation of a compound of the general formula (I) which conforms to the general formula: 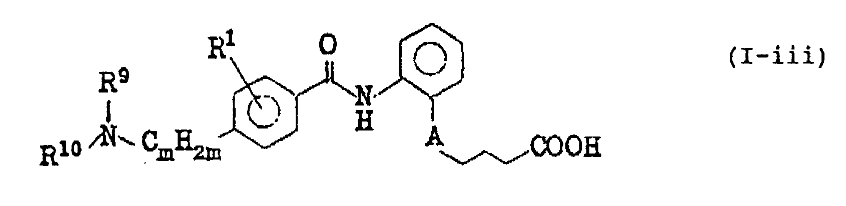 (wherein the symbols are as defined in claim 1).

15. A process according to claim 1 or 14, for the preparation of a compound of the general formula I wherein R¹ is a hydrogen atom.

16. A process according to claim 1, 14, or 15, for the preparation of a compound of the general formula I which is:
4-[2-[4-[N,N-bis(4-propylbenzyl)amino]benzoylamino]phenoxy]butanoic acid,
4-[2-[4-(4-propylphenylaminomethyl)benzoylamino] phenoxy]butanoic acid,
4-[2-[4-(5,6,7,8-tetrahydronaphth-1-yl)aminomethylbenzoylamino]phenoxy]butanoic acid or
4-[2-[4-[N-methyl-N-(5,6,7,8-tetrahydronaphth-1-yl) aminomethyl]benzoylamino]phenoxy]butanoic acid, or a non-toxic salt thereof.

17. A process according to claim 1 or 14 for the preparation of a compound of the general formula I wherein R¹ is a methyl group.

18. A process according to claim 1, 14 or 17, for the preparation of a compound of the general formula I which is:
4-[2-[2-methyl-4-[N,N-bis(4-propylbenzyl)amino]benzoylamino]phenoxy]butanoic acid,
4-[2-[4-[N,N-bis(4-isobutylbenzyl)amino]-2-methylbenzoylamino]phenoxy]butanoic acid,
4-[2-[4-[N,N-dipentylamino)-2-methylbenzoylamino]phenoxy]butanoic acid,
4-[2-[4-[N-pentyl-N-(4-propylbenzyl)amino]-2-methylbenzoylamino]phenoxy]butanoic acid,
4-[2-[4-[N-methyl-N-(4-propylbenzyl)amino]-2-methylbenzoylamino]phenoxy]butanoic acid,
4-[2-[4-N,N-bis(4-phenylbutyl)amino]-2-methylbenzoylamino]phenoxy]butanoic acid,
4-[2-[4-[N,N-bis(4-isopropylbenzyl)amino]-2-methylbenzoylamino]phenoxy]butanoic acid,
4-[2-[4-[N,N-bis(4-propylbenzyl)amino]-2-methylbenzoylamino]phenylthio]butanoic acid,
4-[2-[4-[N,N-bis(4-propylbenzyl)amino]-2-methylbenzylamino]phenylsulfinyl]butanoic acid
4-[2-[4-[N,N-bis(2,4-difluorobenzyl)amino]-2-methylbenzoylamino]phenoxy]butanoic acid,
4-[2-[4-[N-(4-propylbenzyl)-N-(4-propylbenzoyl)amino]-2-methylbenzoylamino]phenoxy]butanoic acid,
4-[2-[4-[N,N-bis(2-trifluoromethylbenzyl)amino]-2-methylbenzoylamino]phenoxy]butanoic acid or
4-[2-[4-[N,N-bis(4-trifluoromethylbenzyl)amino]-2-methylbenzoylamino]phenoxy]butanoic acid, or a non-toxic salt thereof.

19. A process according to any one of the preceding claims followed by the step of formulating a benzoylaminophenoxybutanoic acid derivative according to claim 1 or a non-toxic salt thereof with a pharmaceutically acceptable carrier or coating.

20. Use of a benzoylaminophenoxybutanoic acid derivative according to claim 1, or a non-toxic salt thereof, in the manufacture of a medicament for the treatment of alopecia, acne or prostatic hypertrophy.

21. Use as a cosmetic product of a benzoylaminophenoxybutanoic acid derivative according to claim 1 or a non-toxic salt thereof.

22. A compound of general formula (II) depicted in claim 1 wherein the various symbols are as defined in claim 1.

23. A process for the preparation of a compound of general formula (II) as defined in claim 1 which comprises:
(A) the reaction of the carboxy group (COOH) of a compound of the general formula: 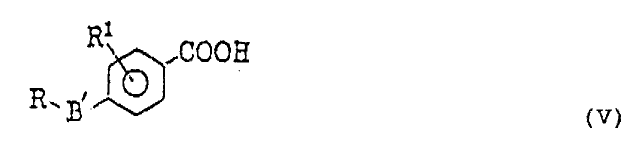 wherein B' represents an oxygen atom or a sulfur atom, R¹ is as defined in claim 1, and R Cl1 represents a group of the general formula: 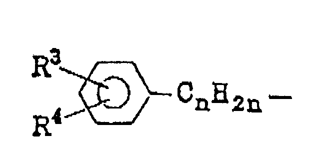 or 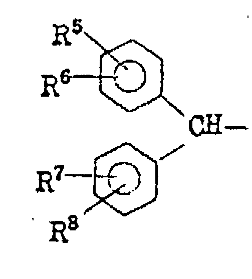 wherein R³, R⁴, R⁵, R⁶, R⁷, and R⁸ are as defined in claim 1, with the amino group (NH₂) of a compound of the general formula: 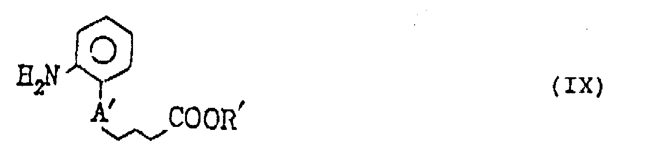 wherein A' represents an oxygen atom or sulfur atom and R' represents an alkyl group of from 1 to 4 carbon atoms, to form an amide (-CONH-) bond;
(B) the reaction of a compound of the general formula 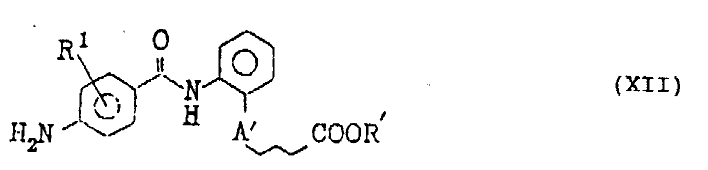 wherein R¹ is as defined in claim 1 and A' and R' are as hereinbefore defined with a compound of the general formula R-X¹, R⁹⁻X⁴ and/or R¹⁰-X⁴, or R¹¹-X⁵ wherein R is as hereinbefore defined, R⁹, R¹⁰ and R¹¹ are as defined in claim 1 and X¹, X⁴ and X⁵ each represents a halogen atom, a tosyl group or a mesyl group; or
(C) the reaction of a compound of the general formula 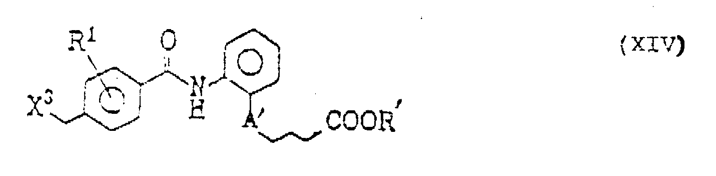 wherein R¹ is as defined in claim 1, A' and R' are as hereinbefore defined and X³ represents a halogen atom, a tosyl group and or a mesyl group, with a compound of the general formula; 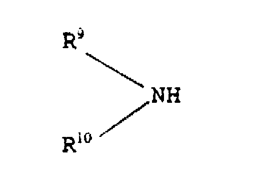
wherein R⁹ and R¹⁰ are as defined in claim 1.

## Patentansprüche (Patentansprüche für folgende(n) Vertragsstaat(en) : AT, BE, CH, DE, FR, GB, IT, LI, LU, NL, SE)

1. Benzoylaminophenoxybutansäurederivat der allgemeinen Formel: 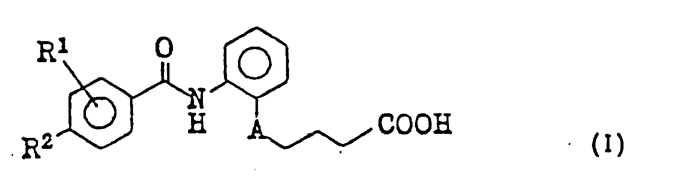 worin bedeuten:
A ein Sauerstoffatom, ein Schwefelatom oder eine Sulfinyl (SO)-Gruppe,
R¹ ein Wasserstoffatom oder eine Methylgruppe;
R² eine Gruppe der allgemeinen Formeln: 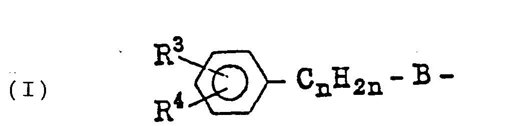 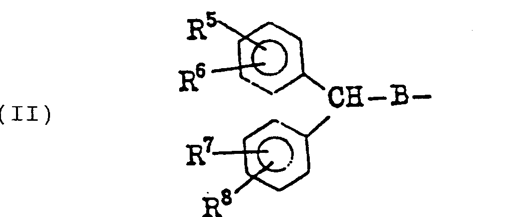 oder 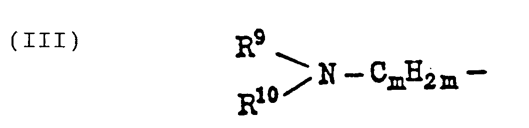 in welchen bedeuten:
B ein Sauerstoffatom, ein Schwefelatom oder eine Gruppe der allgemeinen Formel
NR¹¹
mit R¹¹ gleich einem Wasserstoffatom oder einer Alkylgruppe mit 1 bis 4 Kohlenstoffatom(en);
R³, R⁴, R⁵, R⁶, R⁷ und R⁸ unabhängig voneinander jeweils ein Wasserstoffatom, eine Alkylgruppe mit 1 bis 4 Kohlenstoffatom(en), ein Halogenatom oder eine Trifluormethylgruppe;
m 0 oder 1;
n eine ganze Zahl von 1 bis 4 und
R⁹ und R¹⁰, die gleich oder verschieden sein können, jeweils ein Wasserstoffatom, eine Alkylgruppe mit 1 bis 5 Kohlenstoffatom(en) oder eine Gruppe der allgemeinen Formel: 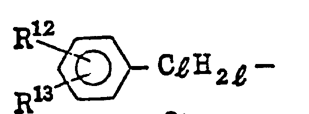 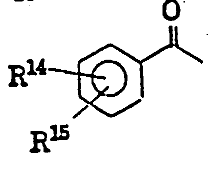 oder 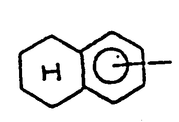 mit R¹², R¹³, R¹⁴ und R¹⁵ unabhängig voneinander jeweils gleich einem Wasserstoffatom, einer Alkylgruppe mit 1 bis 4 Kohlenstoffatom(en), einem Halogenatom oder einer Trifluormethylgruppe und I gleich einer ganzen Zahl von 1 bis 4,
wobei gilt, daß R⁹ und R¹⁰ nicht gleichzeitig für Wasserstoffatome stehen dürfen,
oder ein nicht-toxisches Salz desselben.

2. Verbindung nach Anspruch 1 entsprechend der allgemeinen Formel: 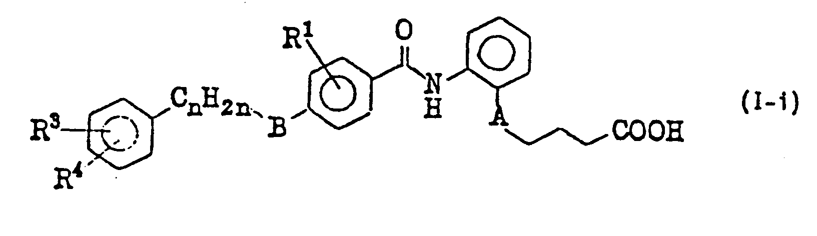 worin sämtliche Symbole die in Anspruch 1 angegebene Bedeutung besitzen.

3. Verbindung nach Anspruch 1 oder 2, worin B für ein Sauerstoffatom steht.

4. Verbindung nach Anspruch 1, 2 oder 3, nämlich
4-[2-[4-(4-Isobutylphenylmethoxy)-benzoylamino]-phenoxy]-butansäure,
4-[2-(4-Benzyloxybenzoylamino)-phenoxy]-butansäure,
4-[2-[4-(4-Methylphenylmethoxy)-benzoylamino]-phenoxy]-butansäure,
4-[2-[4-(4-Ethylphenylmethoxy)-benzoylamino]-phenoxy]-butansäure,
4-[2-[4-(4-Propylphenylmethoxy)-benzoylamino]-phenoxy]-butansäure,
4-[2-[4-(4-Isopropylphenylmethoxy)-benzoylamino]-phenoxy]-butansäure,
4-[2-[4-(3-Phenylpropoxy)-benzoylamino]-phenoxy]-butansäure,
4-[2-[4-(4-Phenylbutoxy)-benzoylamino]-phenoxy]-butansäure,
4-[2-[4-(4-Isobutylphenylmethoxy)-2-methylbenzoylamino]-phenoxy]-butansäure oder
4-[2-[4-(4-Isobutylphenylmethoxy)-3-methylbenzoylamino]-phenoxy]-butansäure,
oder ein nicht-toxisches Salz derselben.

5. Verbindung nach Anspruch 1 oder 2, worin B für eine Gruppe der allgemeinen Formel NR¹¹ mit R11 in der in Anspruch 1 angegebenen Bedeutung steht.

6. Verbindung nach Anspruch 1, 2 oder 5, nämlich
4-[2-[4-(4-Isobutylbenzylamino)-benzoylamino]-phenoxy]-butansäure,
4-[2-[4-(4-lsobutylbenzylamino)-2-methylbenzoylamino]-phenoxy]-butansäure,
4-[2-[4-(4-Propylbenzylamino)-2-methylbenzoylamino]-phenoxy]-butansäure,
4-[2-[4-[1-(4-Isobutylphenyl)-ethylamino]-2-methylbenzoylamino]-phenoxy]-butansäure,
4-[2-[4-(4-Isopropylbenzylamino)-2-methylbenzoylamino]-phenoxy]-butansäure,
4-[2-[4-(4-Propylbenzylamino)-2-methylbenzoylamino]-phenylthio]-butansäure oder
4-[2-[4-(2-Trifluormethylbenzylamino)-2-methylbenzoylamino]-phenoxy]-butansäure oder ein nicht-toxisches Salz derselben.

7. Verbindung nach Anspruch 1, entsprechend der allgemeinen Formel: 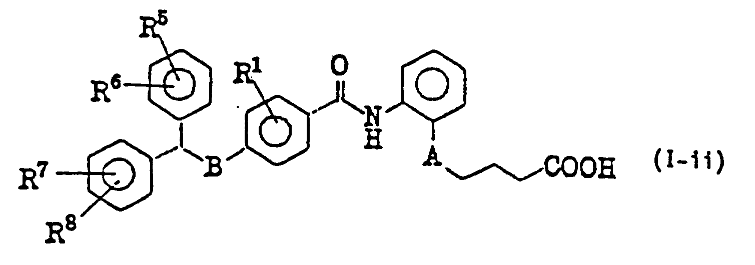 worin sämtliche Symbole die in Anspruch 1 angegebene Bedeutung besitzen.

8. Verbindung nach Anspruch 1 oder 7, worin B für ein Sauerstoffatom steht.

9. Verbindung nach Anspruch 1, 7 oder 8, nämlich
4-[2-[4-[Bis-(4-propylphenyl)-methoxy]-benzoylamino]-phenoxy]-butansäure,
4-[2-[4-[Bis-(4-propylphenyl)-methoxy]-2-methylbenzoylamino]-phenoxy]-butansäure,
4-[2-[4-[Bis-(4-ethylphenyl)-methoxy]-benzoylamino]-phenoxy]-butansäure,
4-[2-[4-[Bis-(4-pentylphenyl)-methoxy]-benzoylamino]-phenoxy]-butansäure oder
4-[2-[4-[Bis-(4-butylphenyl)-methoxy]-benzoylamino]-phenoxy]-butansäure.

10. Verbindung nach Anspruch 1 oder 7, worin B für eine Gruppe der allgemeinen Formel NR¹¹ mit R¹¹ in der in Anspruch 1 angegebenen Bedeutung steht.

11. Verbindung nach Anspruch 1, 7 oder 10, nämlich:
4-[2-[4-[Bis-(4-propylphenyl)-methylamino]-2-methylbenzoylamino]-phenoxy]-butansäure,
4-[2-[4-[Bis-(4-propylphenyl)-methylamino]-benzoylamino]-phenoxy]-butansäure oder
4-[2-[4-[N-Bis-(4-propylphenylmethyl)-N-methylamino]-benzoylamino]-phenoxy]-butansäure oder ein nicht-toxisches Salz derselben.

12. Verbindung nach Anspruch 1 oder 7, worin B für ein Schwefelatom steht.

13. Verbindung nach Anspruch 1, 7 oder 12, nämlich
4-[2-[4-[Bis-(4-propylphenyl)-methylthio]-benzoylamino]-phenoxy]-butansäure oder ein nicht-toxisches Salz derselben.

14. Verbindung nach Anspruch 1 entsprechend der allgemeinen Formel: 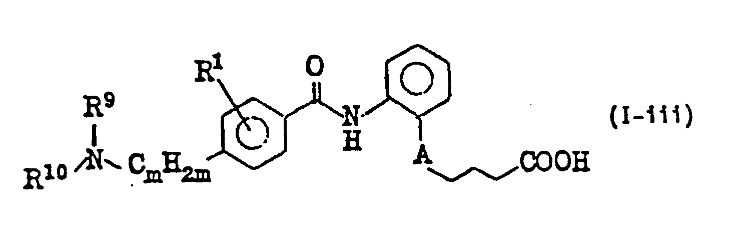 worin sämtliche Symbole die in Anspruch 1 angegebene Bedeutung besitzen.

15. Verbindung nach Anspruch 1 oder 14, worin R¹ für ein Wasserstoffatom steht.

16. Verbindung nach Anspruch 1, 14 oder 15, nämlich:
4-[2-[4-[N,N-Bis-(4-propylbenzyl)-amino]-benzoylamino]-phenoxy]-butansäure,
4-[2-[4-(4-Propylphenylaminomethyl)-benzoylamino]-phenoxy]-butansäure,
4-[2-[4-(5,6,7,8-Tetrahydronaphth-1 -yl)-aminomethylbenzoylamino]-phenoxy]-butansäure,
4-[2-[4-[N-Methyl-N-(5,6,7,8-tetrahydronaphth-1 -yl)-aminomethyl]-benzoylamino]-phenoxy]-butansäure oder ein nicht-toxisches Salz derselben.

17. Verbindung nach Anspruch 1 oder 14, worin R¹ für eine Methylgruppe steht.

18. Verbindung nach Anspruch 1, 14 oder 17, nämlich:
4-[2-[2-Methyl-4-[N,N-bis-(4-propylbenzyl)-amino]-benzoylamino]-phenoxy]-butansäure,
4-[2-[4-[N,N-Bis-(4-isobutylbenzyl)-amino]-2-methylbenzoylamino]-phenoxy]-butansäure,
4-[2-[4-(N,N-Dipentylamino)-2-methylbenzoylamino]-phenoxy]-butansäure,
4-[2-[4-[N-Pentyl-N-(4-propylbenzyl)-amino]-2-methylbenzoylamino]-phenoxy]-butansäure,
4-[2-[4-[N-Methyl-N-(4-propylbenzyl)-amino]-2-methylbenzoylamino]-phenoxy]-butansäure,
4-[2-[4-[N,N-Bis-(4-phenylbutyl)-amino]-2-methylbenzoylamino]-phenoxy]-butansäure,
4-[2-[4-[N,N-Bis-(4-isopropylbenzyl)-amino]-2-methylbenzoylamino]-phenoxy]-butansäure,
4-[2-[4-[N,N-Bis-(4-propylbenzyl)-amino]-2-methylbenzoylamino]-phenylthio]-butansäure,
4-[2-[4-[N,N-Bis-(4-propylbenzyl)-amino]-2-methylbenzoylamino]-phenylsulfinyl]-butansäure,
4-[2-[4-[N,N-Bis-(2,4-difluorbenzyl)-amino]-2-methylbenzoylamino]-phenoxy]-butansäure,
4-[2-[4-[N-Propylbenzyl)-N-(4-propylbenzoyl)-amino]-2-methylbenzoylamino]-phenoxy]-butansäure,
4-[2-[4-[N,N-Bis-(2-trifluormethylbenzyl)-amino]-2-methylbenzoylamino]-phenoxy]-butansäure oder
4-[2-[4-[N,N-Bis-(4-trifluormethylbenzyl)-amino]-2-methylbenzoylamino]-phenoxy]-butansäure oder ein nicht-toxisches Salz derselben.

19. Verfahren zur Herstellung eines Benzoylaminophenoxybutansäurederivats der allgemeinen Formel: 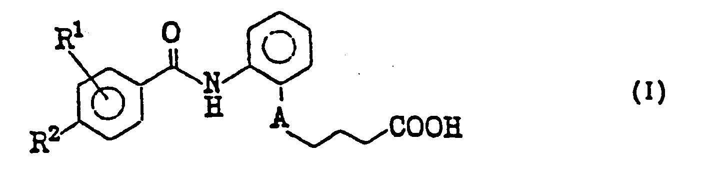 worin die verschiedenen Symbole die in Anspruch 1 angegebene Bedeutung besitzen, durch
(A) - wenn A für ein Schwefelatom oder ein Sauerstoffatom steht - die Verseifung einer Verbindung der allgemeinen Formel: 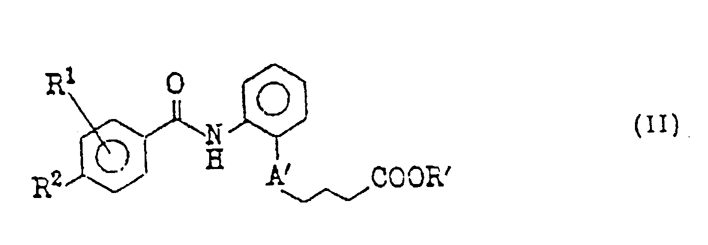 (worin R' für eine Alkylgruppe mit 1 bis 4 Kohlenstoffatom(en) steht, A' ein Sauerstoffatom oder Schwefelatom darstellt und die sonstigen Symbole die in Anspruch 1 angegebene Bedeutung besitzen), oder
(B) - wenn A in der allgemeinen Formel (I) für eine Sulfinylgruppe steht und die sonstigen Symbole die in Anspruch 1 angegebene Bedeutung besitzen - Oxidation einer Verbindung der allgemeinen Formel: 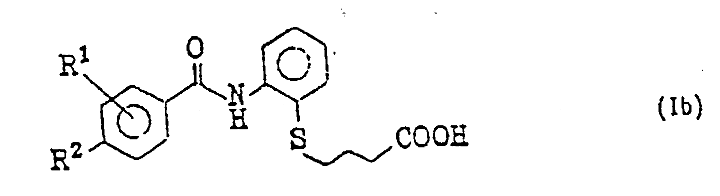 (worin R¹ und R² die in Anspruch 1 angegebene Bedeutung besitzen) zur Umwandlung der Gruppe -S- in eine Gruppe -S(=0)-, und anschließend gewünschtenfalls Umwandlung einer Verbindung der allgemeinen Formel (I) in ein nicht-toxisches Salz derselben.

20. Arzneimittelzubereitung, umfassend ein Benzoylaminophenoxybutansäurederivat nach Anspruch 1 oder ein nicht-toxisches Salz derselben in Verbindung mit einem pharmazeutisch akzeptablen Träger oder Überzug.

21. Benzoylaminophenoxybutansäurederivat nach Anspruch 1 oder ein nicht-toxisches Salz derselben zur Verwendung bei der Herstellung eines Medikaments.

22. Verwendung eines Benzoylaminophenoxybutansäurederivats nach Anspruch 1 oder eines nicht-toxischen Salzes desselben bei der Herstellung eines Medikaments zur Behandlung von Alopezie, Akne oder Prostatahypertropie.

23. Kosmetische Zubereitung, enthaltend ein Benzoylaminophenoxybutansäurederivat nach Anspruch 1 oder ein nicht-toxisches Salz desselben.

24. Verwendung eines Benzoylaminophenoxybutansäurederivats nach Anspruch 1 oder eines nicht-toxischen Salzes desselben als kosmetisches Produkt.

25. Verbindung der in Anspruch 19 dargestellten allgemeiner Formel (11), worin R' und A' die in Anspruch 19 und die sonstigen Symbole die in Anspruch 1 angegebenen Bedeutungen besitzen.

26. Verfahren zur Herstellung einer Verbindung der in Anspruch 19 definierten allgemeinen Formel (11) durch
(A) Umsetzung der Carboxygruppe (COOH) einer Verbindung der allgemeinen Formel: 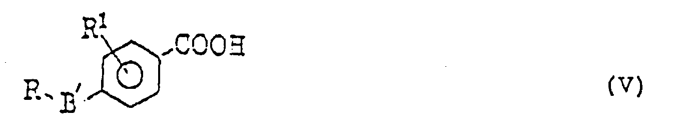 worin B' für ein Sauerstoffatom oder ein Schwefelatom steht, R¹ die in Anspruch 1 angegebene Bedeutung besitzt und R eine Gruppe der allgemeinen Formeln: 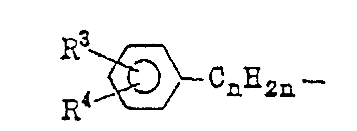 oder 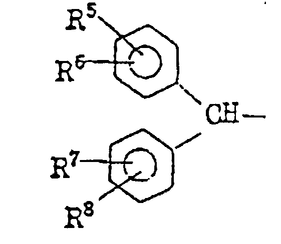 worin R³, R⁴, R⁵, R⁶, R⁷ und R⁸ die in Anspruch 1 angegebene Bedeutung besitzen, darstellt, mit der Aminogruppe (NH₂) einer Verbindung der allgemeinen Formel: 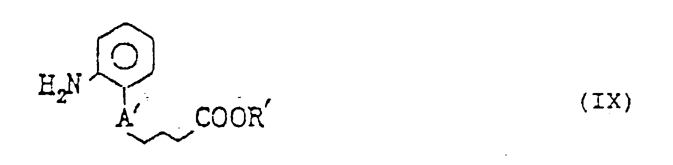 worin A' für ein Sauerstoffatom oder Schwefelatom steht und R' eine Alkylgruppe mit 1 bis 4 Kohlenstoffatom(en) darstellt, zur Bildung einer Amid (-CONH-)-Bindung;
(B) Umsetzung einer Verbindung der allgemeinen Formel 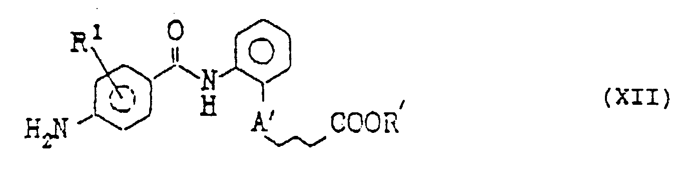 worin R¹ die in Anspruch 1 angegebene Bedeutung besitzt und A' und R' der zuvor angegebenen Definition genügen, mit einer Verbindung der allgemeinen Formel R-X¹, R⁹⁻X⁴ und/oder R¹⁰-X⁴ oder R¹¹-X⁵ mit R in der zuvor angegebenen Bedeutung, R⁹, R¹⁰ und R¹¹ in der in Anspruch 1 angegebenen Bedeutung und X¹, X⁴ und X⁵ jeweils gleich einem Halogenatom, einer Tosylgruppe oder einer Mesylgruppe oder
(C) Umsetzung einer Verbindung der allgemeinen Formel 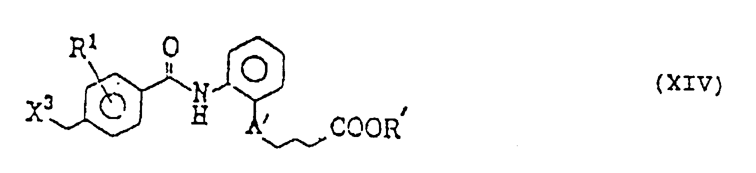 worin R¹ die in Anspruch 1 angegebene Bedeutung besitzt, A' und R' der zuvor angegebenen Definition genügen und X³ für ein Halogenatom, eine Tosylgruppe oder eine Mesylgruppe steht, mit einer Verbindung der allgemeinen Formel 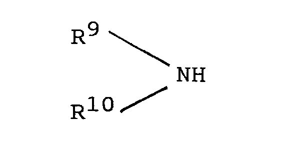 worin R⁹ und R¹⁰ die in Anspruch 1 angegebene Bedeutung besitzen.

## Patentansprüche (Patentansprüche für folgende(n) Vertragsstaat(en) : ES)

1. Verfahren zur Herstellung eines Benzoylaminophenoxybutansäurederivats der allgemeinen Formel: 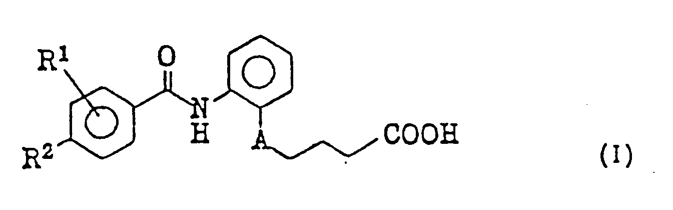 worin bedeuten:
A ein Sauerstoffatom, ein Schwefelatom oder eine Sulfinyl (SO)-Gruppe,
R¹ ein Wasserstoffatom oder eine Methylgruppe;
R² eine Gruppe der allgemeinen Formeln: 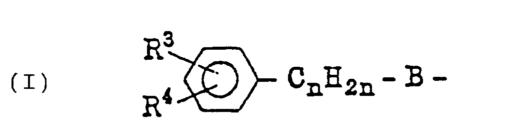 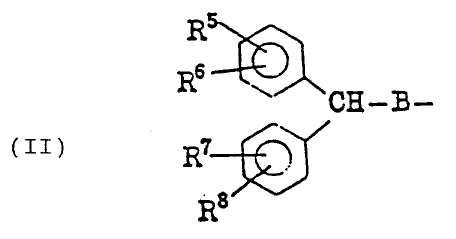 oder 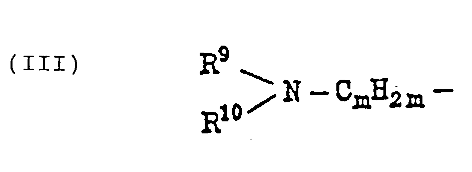 in welchen bedeuten:
B ein Sauerstoffatom, ein Schwefelatom oder eine Gruppe der allgemeinen Formel
NR¹¹
mit R¹¹ gleich einem Wasserstoffatom oder einer Alkylgruppe mit 1 bis 4 Kohlenstoffatom(en);
R³, R⁴, R⁵, R⁶, R⁷ und R⁸ unabhängig voneinander jeweils ein Wasserstoffatom, eine Alkylgruppe mit 1 bis 4 Kohlenstoffatom(en), ein Halogenatom oder eine Trifluormethylgruppe;
m 0 oder 1;
n eine ganze Zahl von 1 bis 4 und
R⁹ und R¹⁰, die gleich oder verschieden sein können, jeweils ein Wasserstoffatom, eine Alkylgruppe mit 1 bis 5 Kohlenstoffatom(en) oder eine Gruppe der allgemeinen Formel: 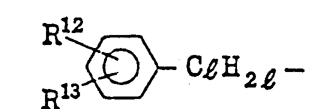 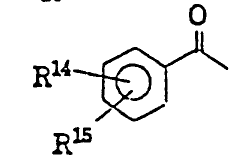 oder 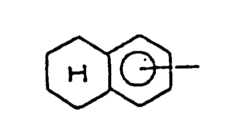 mit R¹², R¹³, R¹⁴ und R¹⁵ unabhängig voneinander jeweils gleich einem Wasserstoffatom, einer Alkylgruppe mit 1 bis 4 Kohlenstoffatom(en), einem Halogenatom oder einer Trifluormethylgruppe und I gleich einer ganzen Zahl von 1 bis 4, wobei gilt, daß R⁹ und R¹⁰ nicht gleichzeitig für Wasserstoffatome stehen dürfen, oder eines nicht-toxischen Salzes desselben, durch
(A) - wenn A für ein Schwefelatom oder ein Sauerstoffatom steht - Verseifung einer Verbindung der allgemeinen Formel: 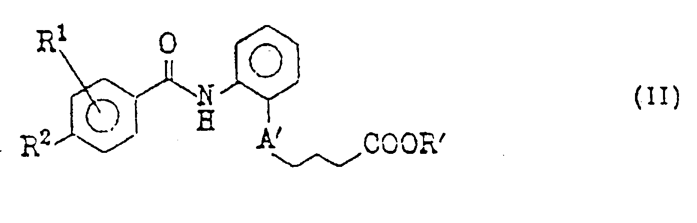 (worin R' für eine Alkylgruppe mit 1 bis 4 Kohlenstoffatom(en) steht, A' ein Sauerstoff- oder Schwefelatom darstellt und die sonstigen Symbole die zuvor angegebene Bedeutung besitzen), oder
(B) - wenn A in der allgemeinen Formel (I) für eine Sulfinylgruppe steht und die sonstigen Symbole die zuvor angegebene Bedeutung besitzen - Oxidation einer Verbindung der allgemeinen Formel: 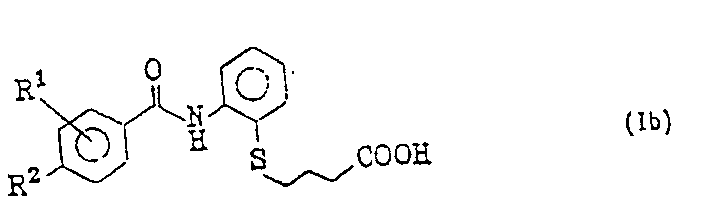 (worin R¹ und R² die zuvor angegebene Bedeutung besitzen) zur Umwandlung der Gruppe -S- in eine Gruppe -S(=O)-, und anschließend gewünschtenfalls Umwandlung einer Verbindung der allgemeinen Formel (I) in ein nicht-toxisches Salz derselben.

2. Verfahren nach Anspruch 1 zur Herstellung einer Verbindung der allgemeinen Formel: 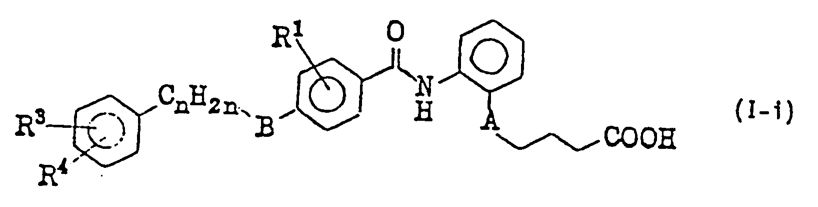 (worin die einzelnen Symbole die in Anspruch 1 angegebene Bedeutung besitzen).

3. Verfahren nach Anspruch 1 oder 2 zur Herstellung einer Verbindung der allgemeinen Formel (I), worin B für ein Sauerstoffatom steht.

4. Verfahren nach Anspruch 1, 2 oder 3 zur Herstellung einer Verbindung der allgemeinen Formel (I), nämlich:
4-[2-[4-(4-Isobutylphenylmethoxy)-benzoylamino]-phenoxy]-butansäure,
4-[2-(4-Benzyloxybenzoylamino)-phenoxy]-butansäure,
4-[2-[4-(4-Methylphenylmethoxy)-benzoylamino]-phenoxy]-butansäure,
4-[2-[4-(4-Ethylphenylmethoxy)-benzoylamino]-phenoxy]-butansäure,
4-[2-[4-(4-Propylphenylmethoxy)-benzoylamino]-phenoxy]-butansäure,
4-[2-[4-(4-Isopropylphenylmethoxy)-benzoylamino]-phenoxy]-butansäure,
4-[2-[4-(3-Phenylpropoxy)-benzoylamino]-phenoxy]-butansäure,
4-[2-[4-(4-Phenylbutoxy)-benzoylamino]-phenoxy]-butansäure,
4-[2-[4-(4-Isobutylphenylmethoxy)-2-methylbenzoylamino]-phenoxy]-butansäure oder
4-[2-[4-(4-Isobutylphenylmethoxy)-3-methylbenzoylamino]-phenoxy]-butansäure, oder eines nicht-toxischen Salzes derselben.

5. Verfahren nach Anspruch 1 oder 2 zur Herstellung einer Verbindung der allgemeinen formel (I), worin B für eine Gruppe der allgemeinen Formel NR¹¹ mit R¹¹ in der in Anspruch 1 angegebenen Bedeutung steht.

6. Verfahren nach Anspruch 1, 2 oder 5 zur Herstellung einer Verbindung der allgemeinen Formel (I), nämlich:
4-[2-[4-(4-Isobutylbenzylamino)-benzoylamino]-phenoxy]-butansäure,
4-[2-[4-(4-Isobutylbenzylamino)-2-methylbenzoylamino]-phenoxy]-butansäure,
4-[2-[4-(4-Propylbenzylamino)-2-methylbenzoylamino]-phenoxy]-butansäure,
4-[2-[4-[1-(4-Isobutylphenyl)-ethylamino]-2-methylbenzoylamino]-phenoxy]-butansäure,
4-[2-[4-(4-Isopropylbenzylamino)-2-methylbenzoylamino]-phenoxy]-butansäure,
4-[2-[4-(4-Propylbenzylamino)-2-methylbenzoylamino]-phenylthio]-butansäure oder
4-[2-[4-(2-Trifluormethylbenzylamino)-2-methylbenzoylamino]-phenoxy]-butansäure oder eines nicht-toxischen Salzes derselben.

7. Verfahren nach Anspruch 1 zur Herstellung einer Verbindung der allgemeinen Formel (I) entsprechend der allgemeinen Formel: 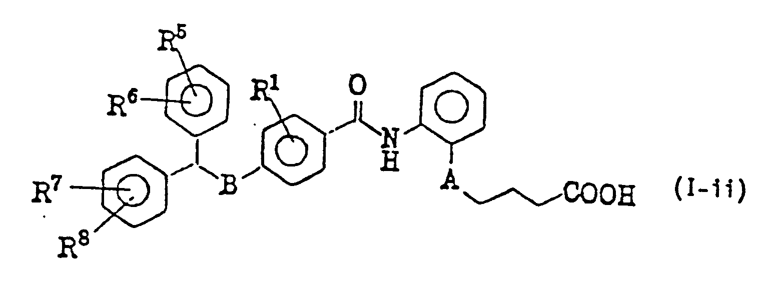 (worin sämtliche Symbole die in Anspruch 1 angegebene Bedeutung besitzen).

8. Verfahren nach Anspruch 1 oder 7 zur Herstellung einer Verbindung der allgemeinen Formel (I) mit B gleich einem Sauerstoffatom.

9. Verfahren nach Anspruch 1, 7 oder 8 zur Herstellung einer Verbindung der allgemeinen Formel (I), nämlich:
4-[2-[4-[Bis-(4-propylphenyl)-methoxy]-benzoylamino]-phenoxy]-butansäure,
4-[2-[4-[Bis-(4-propylphenyl)-methoxy]-2-methyl-benzoylamino]-phenoxy]-butansäure,
4-[2-[4-[Bis-(4-ethylphenyl)-methoxy]-benzoylamino]-phenoxy]-butansäure,
4-[2-[4-[Bis-(4-pentylphenyl)-methoxy]-benzoylamino]-phenoxy]-butansäure oder
4-[2-[4-[Bis-(4-butylphenyl)-methoxy]-benzoylamino]-phenoxy]-butansäure oder eines nicht-toxischen Salzes derselben.

10. Verfahren nach Anspruch 1 oder 7 zur Herstellung einer Verbindung der allgemeinen Formel (I), worin B für eine Gruppe der allgemeinen Formel NR¹¹ mit R11 in der in Anspruch 1 angegebenen Bedeutung steht.

11. Verfahren nach Anspruch 1, 7 oder 10 zur Herstellung einer Verbindung der allgemeinen Formel (I), nämlich:
4-[2-[4-[Bis-(4-propylphenyl)-methylamino]-2-methylbenzoylamino]-phenoxy]-butansäure,
4-[2-[4-[Bis-(4-propylphenyl)-methylamino]-benzoylamino]-phenoxy]-butansäure oder
4-[2-[4-[N-Bis-(4-propylphenylmethyl)-N-methylamino]-benzoylamino]-phenoxy]-butansäure oder eines nicht-toxischen Salzes derselben.

12. Verfahren nach Anspruch 1 oder 7 zur Herstellung einer Verbindung der allgemeinen Formel (I), worin B für ein Schwefelatom steht.

13. Verfahren nach Anspruch 1, 7 oder 12 zur Herstellung einer Verbindung der allgemeinen Formel (I), nämlich:
4-[2-[4-[Bis-(4-propylphenyl)-methylthio]-benzoylamino]-phenoxy]-butansäure oder eines nicht-toxischen Salzes derselben.

14. Verfahren nach Anspruch 1 zur Herstellung einer Verbindung der allgemeinen Formel (I) entsprechend der allgemeinen Formel: 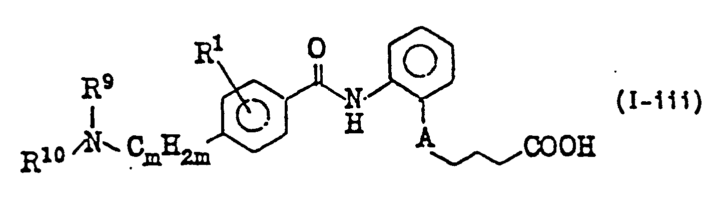 (worin die einzelnen Symbole die in Anspruch 1 angegebene Bedeutung besitzen).

15. Verfahren nach Anspruch 1 oder 14 zur Herstellung einer Verbindung der allgemeinen Formel (I), worin R¹ für ein Wasserstoffatom steht.

16. Verfahren nach Anspruch 1, 14 oder 15 zur Herstellung einer Verbindung der allgemeinen Formel (I), nämlich:
4-[2-[4-[N,N-Bis-(4-propylbenzyl)-amino]-benzoylamino]-phenoxy]-butansäure,
4-[2-[4-(4-Propylphenylaminomethyl)-benzoylamino]-phenoxy]-butansäure,
4-[2-[4-(5,6,7,8-Tetrahydronaphth-1 -yl)-aminomethylbenzoylamino]-phenoxy]-butansäure,
4-[2-[4-[N-Methyl-N-(5,6,7,8-tetrahydronaphth-1 -yl)-aminomethyl]-benzoylamino]-phenoxy]-butansäure oder eines nicht-toxischen Salzes derselben.

17. Verfahren nach Anspruch 1 oder 14 zur Herstellung einer Verbindung der allgemeinen Formel (I), worin R¹ für eine Methylgruppe steht.

18. Verfahren nach Anspruch 1, 14 oder 17 zur Herstellung einer Verbindung der allgemeinen Formel (I), nämlich:
4-[2-[2-Methyl-4-[N,N-bis-(4-propylbenzyl)-amino]-benzoylamino]-phenoxy]-butansäure,
4-[2-[4-[N,N-Bis-(4-isobutylbenzyl)-amino]-2-methylbenzoylamino]-phenoxy]-butansäure,
4-[2-[4-(N,N-Dipentylamino)-2-methylbenzoylamino]-phenoxy]-butansäure,
4-[2-[4-[N-Pentyl-N-(4-propylbenzyl)-amino]-2-methylbenzoylamino]-phenoxy]-butansäure,
4-[2-[4-[N-Methyl-N-(4-propylbenzyl)-amino]-2-methylbenzoylamino]-phenoxy]-butansäure,
4-[2-[4-[N,N-Bis-(4-phenylbutyl)-amino]-2-methylbenzoylamino]-phenoxy]-butansäure,
4-[2-[4-[N,N-Bis-(4-isopropylbenzyl)-amino]-2-methylbenzoylamino]-phenoxy]-butansäure,
4-[2-[4-[N,N-Bis-(4-propylbenzyl)-amino]-2-methylbenzoylamino]-phenylthio]-butansäure,
4-[2-[4-[N,N-Bis-(4-propylbenzyl)-amino]-2-methylbenzoylamino]-phenylsulfinyl]-butansäure,
4-[2-[4-[N,N-Bis-(2,4-difluorbenzyl)-amino]-2-methylbenzoylamino]-phenoxy]-butansäure,
4-[2-[4-[N-Propylbenzyl)-N-(4-propylbenzoyl)-amino]-2-methylbenzoylamino)-phenoxy]-butansäure,
4-[2-[4-[N,N-Bis-(2-trifluormethylbenzyl)-amino]-2-methylbenzoylamino]-phenoxy]-butansäure oder
4-[2-[4-[N,N-Bis-(4-trifluormethylbenzyl)-amino]-2-methylbenzoylamino]-phenoxy)-butansäure oder eines nicht-toxischen Salzes derselben.

19. Verfahren nach einem der vorhergehenden Ansprüche, gefolgt von einer Stufe der Vereinigung eines Benzoylaminophenoxybutansäurederivats nach Anspruch 1 oder eines nicht-toxischen Salzes desselben mit einem pharmazeutisch akzeptablen Träger oder Überzug.

20. Verwendung eines Benzoylaminophenoxybutansäurederivats nach Anspruch 1 oder eines nicht-toxischen Salzes desselben bei der Herstellung eines Medikaments zur Behandlung von Alopezie, Akne oder Prostatahypertropie.

21. Verwendung eines Benzoylaminophenoxybutansäurederivats nach Anspruch 1 oder eines nicht-toxischen Salzes desselben als kosmetisches Produkt.

22. Verfahren zur Herstellung einer Verbindung der allgemeinen Formel (11) gemäß Anspruch 1 durch
(A) Umsetzung der Carboxygruppe (COOH) einer Verbindung der allgemeinen Formel: 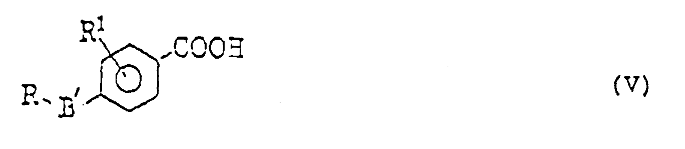 worin B' für ein Sauerstoffatom oder ein Schwefelatom steht, R¹ die in Anspruch 1 angegebene Bedeutung besitzt und R eine Gruppe der allgemeinen Formeln: 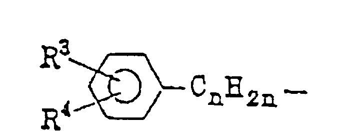 oder 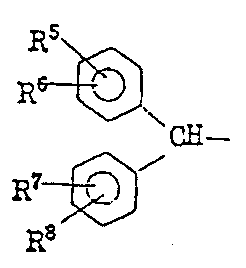 worin R³, R⁴, R⁵, R⁶, R⁷ und R⁸ die in Anspruch 1 angegebene Bedeutung besitzen, darstellt, mit der Aminogruppe (NH₂) einer Verbindung der allgemeinen Formel: 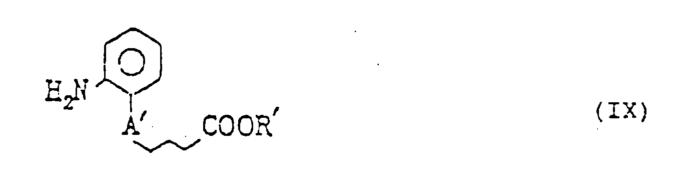 worin A' für ein Sauerstoffatom oder Schwefelatom steht und R' eine Alkylgruppe mit 1 bis 4 Kohlenstoffatom(en) darstellt, zur Bildung einer Amid (-CONH-)-Bindung;
(B) Umsetzung einer Verbindung der allgemeinen Formel 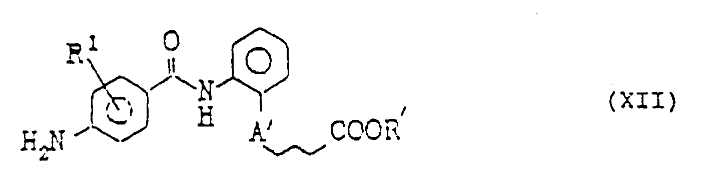 worin R¹ die in Anspruch 1 angegebene Bedeutung besitzt und A' und R' der zuvor angegebenen Definition genügen, mit einer Verbindung der allgemeinen Formel R-X¹, R⁹⁻X⁴ und/oder R¹⁰-X⁴ oder R¹¹-X⁵ mit R in der zuvor angegebenen Bedeutung, R⁹, R¹⁰ und R¹¹ in der in Anspruch 1 angegebenen Bedeutung und X¹, X⁴ und X⁵ jeweils gleich einem Halogenatom, einer Tosylgruppe oder einer Mesylgruppe oder
(C) Umsetzung einer Verbindung der allgemeinen Formel 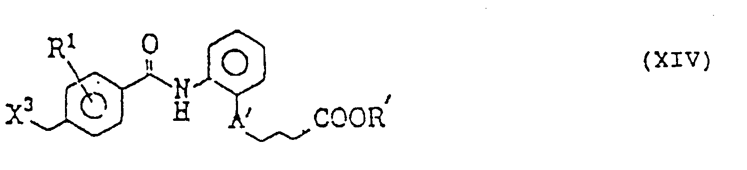 worin R¹ die in Anspruch 1 angegebene Bedeutung besitzt, A' und R' der zuvor angegebenen Definition genügen und X³ für ein Halogenatom, eine Tosylgruppe oder eine Mesylgruppe steht, mit einer Verbindung der allgemeinen Formel 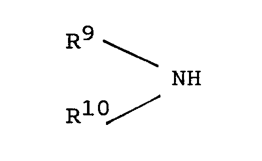 worin R⁹ und R¹⁰ die in Anspruch 1 angegebene Bedeutung besitzen.

## Patentansprüche (Patentansprüche für folgende(n) Vertragsstaat(en) : GR)

1. Verfahren zur Herstellung eines Benzoylaminophenoxybutansäurederivats der allgemeinen Formel: 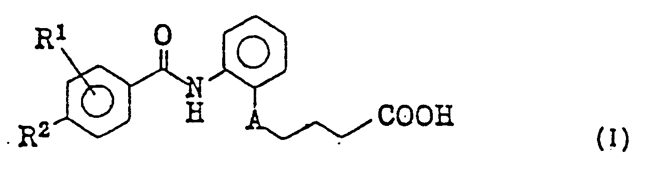 worin bedeuten:
A ein Sauerstoffatom, ein Schwefelatom oder eine Sulfinyl (SO)-Gruppe,
R¹ ein Wasserstoffatom oder eine Methylgruppe;
R² eine Gruppe der allgemeinen Formeln: 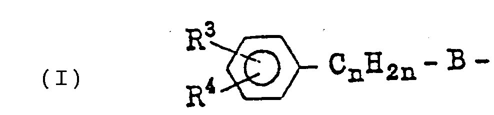 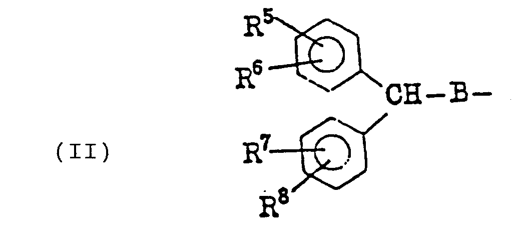 oder 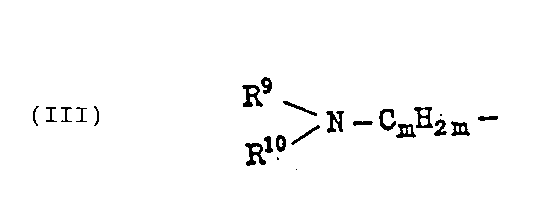
in welchen bedeuten:
B ein Sauerstoffatom, ein Schwefelatom oder eine Gruppe der allgemeinen Formel
NR¹¹
mit R11 gleich einem Wasserstoffatom oder einer Alkylgruppe mit 1 bis 4 Kohlenstoffatom(en);
R³, R⁴, R⁵, R⁶, R⁷ und R⁸ unabhängig voneinander jeweils ein Wasserstoffatom, eine Alkylgruppe mit 1 bis 4 Kohlenstoffatom(en), ein Halogenatom oder eine Trifluormethylgruppe;
m 0 oder 1;
n eine ganze Zahl von 1 bis 4 und
R⁹ und R¹⁰, die gleich oder verschieden sein können, jeweils ein Wasserstoffatom, eine Alkylgruppe mit 1 bis 5 Kohlenstoffatom(en) oder eine Gruppe der allgemeinen Formel: 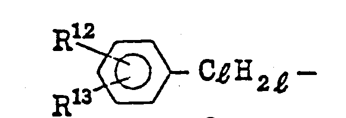 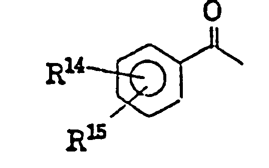
oder 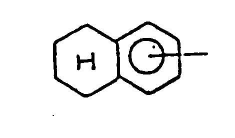 mit R¹², R¹³, R¹⁴ und R¹⁵ unabhängig voneinander jeweils gleich einem Wasserstoffatom, einer Alkylgruppe mit 1 bis 4 Kohlenstoffatom(en), einem Halogenatom oder einer Trifluormethylgruppe und I gleich einer ganzen Zahl von 1 bis 4,
wobei gilt, daß R⁹ und R¹⁰ nicht gleichzeitig für Wasserstoffatome stehen dürfen,
oder eines nicht-toxischen Salzes desselben, durch
(A) - wenn A für ein Schwefelatom oder ein Sauerstoffatom steht - Verseifung einer Verbindung der allgemeinen Formel: 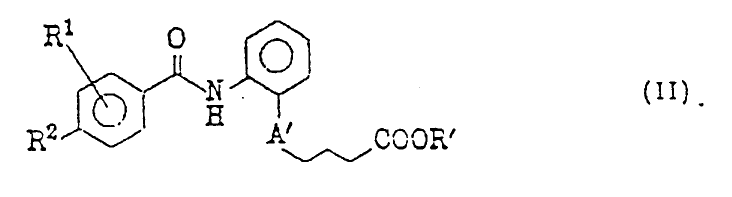 (worin R' für eine Alkylgruppe mit 1 bis 4 Kohlenstoffatom(en) steht, A' ein Sauerstoff- oder Schwefelatom darstellt und die sonstigen Symbole die zuvor angegebene Bedeutung besitzen), oder
(B) - wenn A in der allgemeinen Formel (I) für eine Sulfinylgruppe steht und die sonstigen Symbole die zuvor angegebene Bedeutung besitzen - Oxidation einer Verbindung der allgemeinen Formel: 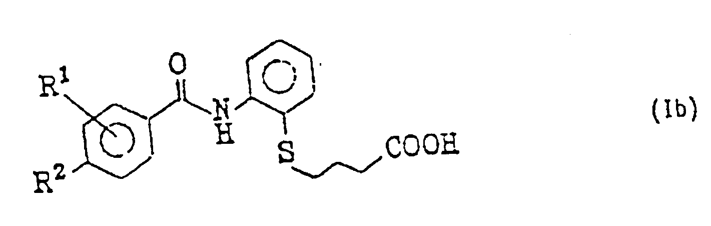 (worin R¹ und R² die zuvor angegebene Bedeutung besitzen) zur Umwandlung der Gruppe -S- in eine Gruppe -S(=O)-, und anschließend gewünschtenfalls Umwandlung einer Verbindung der allgemeinen Formel (I) in ein nicht-toxisches Salz derselben.

2. Verfahren nach Anspruch 1 zur Herstellung einer Verbindung der allgemeinen Formel: 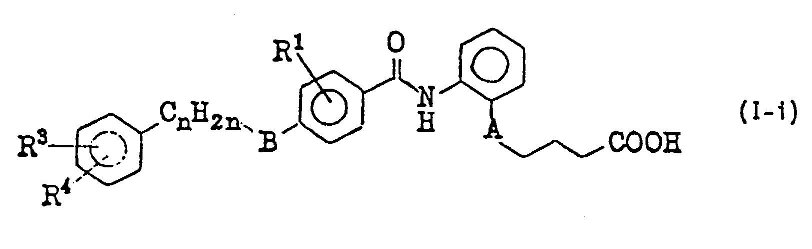 (worin die einzelnen Symbole die in Anspruch 1 angegebene Bedeutung besitzen).

3. Verfahren nach Anspruch 1 oder 2 zur Herstellung einer Verbindung der allgemeinen Formel (I), worin B für ein Sauerstoffatom steht.

4. Verfahren nach Anspruch 1, 2 oder 3 zur Herstellung einer Verbindung der allgemeinen Formel (I), nämlich:
4-[2-[4-(4-Isobutylphenylmethoxy)-benzoylamino]-phenoxy]-butansäure,
4-[2-(4-Benzyloxybenzoylamino)-phenoxy]-butansäure,
4-[2-[4-(4-Methylphenylmethoxy)-benzoylamino]-phenoxy]-butansäure,
4-[2-[4-(4-Ethylphenylmethoxy)-benzoylamino]-phenoxy]-butansäure,
4-[2-[4-(4-Propylphenylmethoxy)-benzoylamino]-phenoxy]-butansäure,
4-[2-[4-(4-Isopropylphenylmethoxy)-benzoylamino]-phenoxy]-butansäure,
4-[2-[4-(3-Phenylpropoxy)-benzoylamino]-phenoxy]-butansäure,
4-[2-[4-(4-Phenylbutoxy)-benzoylamino]-phenoxy]-butansäure,
4-[2-[4-(4-Isobutylphenylmethoxy)-2-methylbenzoylamino]-phenoxy]-butansäure oder
4-[2-[4-(4-Isobutylphenylmethoxy)-3-methylbenzoylamino]-phenoxy]-butansäure, oder eines nicht-toxischen Salzes derselben.

5. Verfahren nach Anspruch 1 oder 2 zur Herstellung einer Verbindung der allgemeinen formel (I), worin B für eine Gruppe der allgemeinen Formel NR¹¹ mit R¹¹ in der in Anspruch 1 angegebenen Bedeutung steht.

6. Verfahren nach Anspruch 1, 2 oder 5 zur Herstellung einer Verbindung der allgemeinen Formel (I), nämlich:
4-[2-[4-(4-Isobutylbenzylamino)-benzoylamino]-phenoxy]-butansäure,
4-[2-[4-(4-Isobutylbenzylamino)-2-methylbenzoylamino]-phenoxy]-butansäure,
4-[2-[4-(4-Propylbenzylamino)-2-methylbenzoylamino]-phenoxy]-butansäure,
4-[2-[4-[1-(4-Isobutylphenyl)-ethylamino]-2-methylbenzoylamino]-phenoxy]-butansäure,
4-[2-[4-(4-Isopropylbenzylamino)-2-methylbenzoylamino]-phenoxy]-butansäure,
4-[2-[4-(4-Propylbenzylamino)-2-methylbenzoylamino]-phenylthio]-butansäure oder
4-[2-[4-(2-Trifluormethylbenzylamino)-2-methylbenzoylamino]-phenoxy]-butansäure oder eines nicht-toxischen Salzes derselben.

7. Verfahren nach Anspruch 1 zur Herstellung einer Verbindung der allgemeinen Formel (I) entsprechend der allgemeinen Formel: 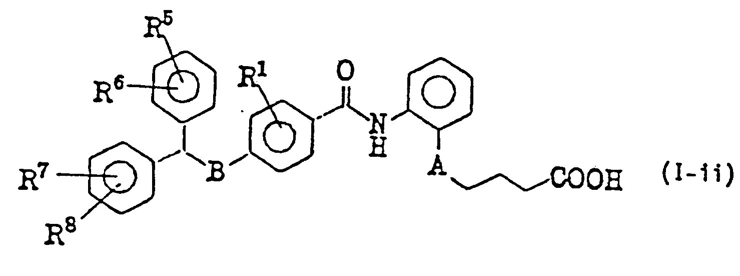 (worin sämtliche Symbole die in Anspruch 1 angegebene Bedeutung besitzen).

8. Verfahren nach Anspruch 1 oder 7 zur Herstellung einer Verbindung der allgemeinen Formel (I) mit B gleich einem Sauerstoffatom.

9. Verfahren nach Anspruch 1, 7 oder 8 zur Herstellung einer Verbindung der allgemeinen Formel (I), nämlich:
4-[2-[4-[Bis-(4-propylphenyl)-methoxy]-benzoylamino]-phenoxy]-butansäure,
4-[2-[4-[Bis-(4-propylphenyl)-methoxy]-2-methylbenzoylamino]-phenoxy]-butansäure,
4-[2-[4-[Bis-(4-ethylphenyl)-methoxy]-benzoylamino]-phenoxy]-butansäure,
4-[2-[4-[Bis-(4-pentylphenyl)-methoxy]-benzoylamino]-phenoxy]-butansäure oder
4-[2-[4-[Bis-(4-butylphenyl)-methoxy]-benzoylamino]-phenoxy]-butansäure oder eines nicht-toxischen Salzes derselben.

10. Verfahren nach Anspruch 1 oder 7 zur Herstellung einer Verbindung der allgemeinen Formel (I), worin B für eine Gruppe der allgemeinen Formel NR¹¹ mit R11 in der in Anspruch 1 angegebenen Bedeutung steht.

11. Verfahren nach Anspruch 1, 7 oder 10 zur Herstellung einer Verbindung der allgemeinen Formel (I), nämlich:
4-[2-[4-[Bis-(4-propylphenyl)-methylamino]-2-methylbenzoylamino]-phenoxy]-butansäure,
4-[2-[4-[Bis-(4-propylphenyl)-methylamino]-benzoylamino]-phenoxy]-butansäure oder
4-[2-[4-[N-Bis-(4-propylphenylmethyl)-N-methylamino]-benzoylamino]-phenoxy]-butansäure oder eines nicht-toxischen Salzes derselben.

12. Verfahren nach Anspruch 1 oder 7 zur Herstellung einer Verbindung der allgemeinen Formel (I), worin B für ein Schwefelatom steht.

13. Verfahren nach Anspruch 1, 7 oder 12 zur Herstellung einer Verbindung der allgemeinen Formel (I), nämlich:
4-[2-[4-[Bis-(4-propylphenyl)-methylthio]-benzoylamino]-phenoxy]-butansäure oder eines nicht-toxischen Salzes derselben.

14. Verfahren nach Anspruch 1 zur Herstellung einer Verbindung der allgemeinen Formel (I) entsprechend der allgemeinen Formel: 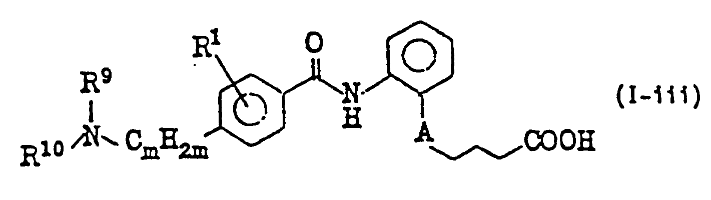 (worin die einzelnen Symbole die in Anspruch 1 angegebene Bedeutung besitzen).

15. Verfahren nach Anspruch 1 oder 14 zur Herstellung einer Verbindung der allgemeinen Formel (I), worin R¹ für ein Wasserstoffatom steht.

16. Verfahren nach Anspruch 1, 14 oder 15 zur Herstellung einer Verbindung der allgemeinen Formel (I), nämlich:
4-[2-[4-[N,N-Bis-(4-propylbenzyl)-amino]-benzoylamino]-phenoxy]-butansäure,
4-[2-[4-(4-Propylphenylaminomethyl)-benzoylamino]-phenoxy]-butansäure,
4-[2-[4-(5,6,7,8-Tetrahydronaphth-1 -yl)-aminomethylbenzoylamino]-phenoxy]-butansäure,
4-[2-[4-[N-Methyl-N-(5,6,7,8-tetrahydronaphth-1 -yl)-aminomethyl]-benzoylamino]-phenoxy]-butansäure oder eines nicht-toxischen Salzes derselben.

17. Verfahren nach Anspruch 1 oder 14 zur Herstellung einer Verbindung der allgemeinen Formel (I), worin R¹ für eine Methylgruppe steht.

18. Verfahren nach Anspruch 1, 14 oder 17 zur Herstellung einer Verbindung der allgemeinen Formel (I), nämlich:
4-[2-[2-Methyl-4-[N,N-bis-(4-propylbenzyl)-amino]-benzoylamino]-phenoxy)-butansäure,
4-[2-[4-[N,N-Bis-(4-isobutylbenzyl)-amino]-2-methylbenzoylamino]-phenoxy]-butansäure,
4-[2-[4-(N,N-Dipentylamino)-2-methylbenzoylamino]-phenoxy]-butansäure,
4-[2-[4-[N-Pentyl-N-(4-propylbenzyl)-amino]-2-methylbenzoylamino]-phenoxy]-butansäure,
4-[2-[4-[N-Methyl-N-(4-propylbenzyl)-amino]-2-methylbenzoylamino]-phenoxy]-butansäure,
4-[2-[4-[N,N-Bis-(4-phenylbutyl)-amino]-2-methylbenzoylamino)-phenoxy]-butansäure,
4-[2-[4-[N,N-Bis-(4-isopropylbenzyl)-amino]-2-methylbenzoylamino]-phenoxy]-butansäure,
4-[2-[4-[N,N-Bis-(4-propylbenzyl)-amino]-2-methylbenzoylamino]-phenylthio]-butansäure,
4-[2-[4-[N,N-Bis-(4-propylbenzyl)-amino]-2-methylbenzoylamino]-phenylsulfinyl]-butansäure,
4-[2-[4-[N,N-Bis-(2,4-difluorbenzyl)-amino]-2-methylbenzoylamino]-phenoxy]-butansäure,
4-[2-[4-[N-Propylbenzyl)-N-(4-propylbenzoyl)-amino]-2-methylbenzoylamino]-phenoxy]-butansäure,
4-[2-[4-[N,N-Bis-(2-trifluormethylbenzyl)-amino]-2-methylbenzoylamino]-phenoxy]-butansäure oder
4-[2-[4-[N,N-Bis-(4-trifluormethylbenzyl)-amino]-2-methylbenzoylamino]-phenoxy]-butansäure oder eines nicht- toxischen Salzes derselben.

19. Verfahren nach einem der vorhergehenden Ansprüche, gefolgt von einer Stufe der Vereinigung eines Benzoylaminophenoxybutansäurederivats nach Anspruch 1 oder eines nicht-toxischen Salzes desrselben mit einem pharmazeutisch akzeptablen Träger oder Überzug.

20. Verwendung eines Benzoylaminophenoxybutansäurederivats nach Anspruch 1 oder eines nicht-toxischen Salzes desselben bei der Herstellung eines Medikaments zur Behandlung von Alopezie, Akne oder Prostatahypertropie.

21. Verwendung eines Benzoylaminophenoxybutansäurederivats nach Anspruch 1 oder eines nicht-toxischen Salzes desselben als kosmetisches Produkt.

22. Verbindung der in Anspruch 1 angegebenen allgemeinen Formel (11), worin die verschiedenen Symbole die in Anspruch 1 angegebene Bedeutung besitzen.

23. Verfahren zur Herstellung einer Verbindung der allgemeinen Formel (11) gemäß Anspruch 1 durch
(A) Umsetzung der Carboxygruppe (COOH) einer Verbindung der allgemeinen Formel: 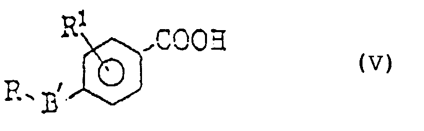 worin B' für ein Sauerstoffatom oder ein Schwefelatom steht, R¹ die in Anspruch 1 angegebene Bedeutung besitzt und R eine Gruppe der allgemeinen Formeln: 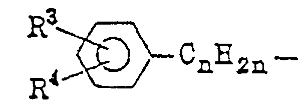 oder 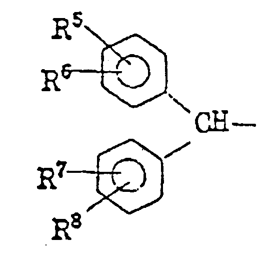 worin R³, R⁴, R⁵, R⁶, R⁷ und R⁸ die in Anspruch 1 angegebene Bedeutung besitzen, darstellt, mit der Aminogruppe (NH₂) einer Verbindung der allgemeinen Formel: 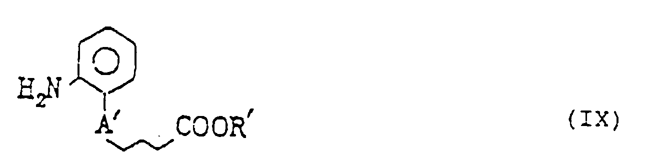 worin A' für ein Sauerstoffatom oder Schwefelatom steht und R' eine Alkylgruppe mit 1 bis 4 Kohlenstoffatom(en) darstellt, zur Bildung einer Amid (-CONH-)-Bindung;
(B) Umsetzung einer Verbindung der allgemeinen Formel 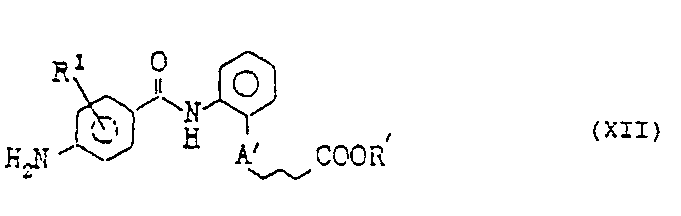 worin R¹ die in Anspruch 1 angegebene Bedeutung besitzt und A' und R' der zuvor angegebenen Definition genügen, mit einer Verbindung der allgemeinen Formel R-X¹, R⁹⁻X⁴ und/oder R¹⁰-X⁴ oder R¹¹-X⁵ mit R in der zuvor angegebenen Bedeutung, R⁹, R¹⁰ und R¹¹ in der in Anspruch 1 angegebenen Bedeutung und X¹, X⁴ und X⁵ jeweils gleich einem Halogenatom, einer Tosylgruppe oder einer Mesylgruppe oder
(C) Umsetzung einer Verbindung der allgemeinen Formel 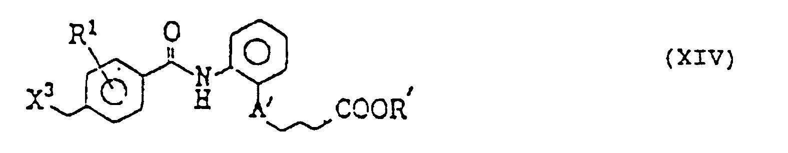 worin R¹ die in Anspruch 1 angegebene Bedeutung besitzt, A' und R' der zuvor angegebenen Definition genügen und X³ für ein Halogenatom, eine Tosylgruppe oder eine Mesylgruppe steht, mit einer Verbindung der allgemeinen Formel 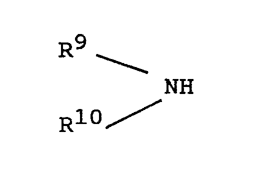 worin R⁹ und R¹⁰ die in Anspruch 1 angegebene Bedeutung besitzen.

## Revendications (Revendications pour l'(les) Etat(s) contractant(s) suivant(s) : AT, BE, CH, DE, FR, GB, IT, LI, LU, NL, SE)

1. Dérivé d'acide benzoylaminophénoxybutanoïque de formule générale : 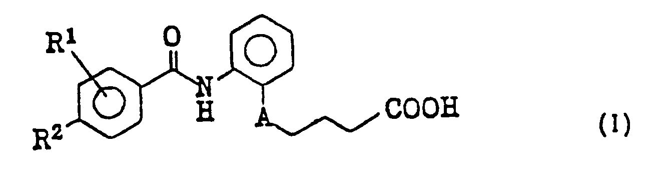 [dans laquelle
A représente un atome d'oxygène, un atome de soufre ou un groupement sulfinyle (SO),
R¹ représente un atome d'hydrogène ou un groupement méthyle,
R² représente un groupement de formule générale : 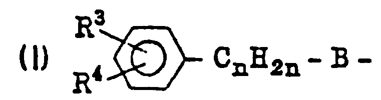 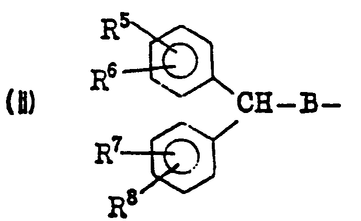 ou 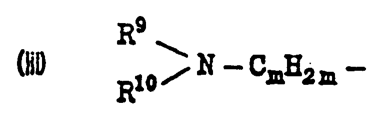 [dans lesquelles
B représente un atome d'oxygène, un atome de soufre ou un groupement de formule générale :
NR¹¹ (dans laquelle R¹¹ représente un atome d'hydrogène ou un groupement alkyle en C₁-C₄), R³, R⁴,
R^{s}, R⁶, R⁷ et R⁸ représentent indépendamment un atome d'hydrogène, un groupement alkyle en C₁-
C₄, un atome d'halogène ou un groupement trifluorométhyle,
m représente 0 ou 1,
n représente un nombre entier compris entre 1 et 4, et
R⁹ et R¹⁰, qui peuvent être identiques ou différents, représentent chacun un atome d'hydrogène, un groupement alkyle en C₁-C₅ ou un groupement de formule générale : 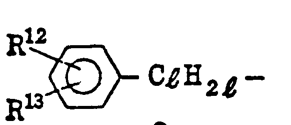 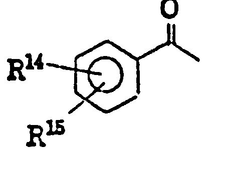 ou 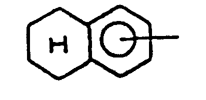 (dans lesquelles
R¹², R¹³, R¹⁴ et R¹⁵ représentent indépendamment un atome d'hydrogène, un groupement alkyle en C₁-C₄, un atome d'halogène ou un groupement trifluorométhyle et 1 représente un nombre entier compris entre 1 et 4) à condition que R⁹ et R¹⁰ ne représentent pas simultanément des atomes d'hydrogène] ou un sel non toxique de celui-ci.

2. Composé selon la revendication 1 qui répond à la formule générale : 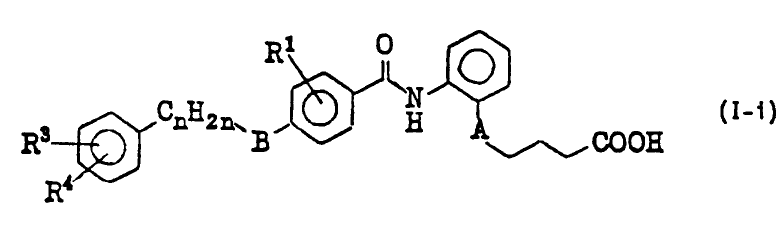 (dans laquelle
les symboles sont tels que définis dans la revendication 1).

3. Composé selon la revendication 1 ou 2, dans lequel B est un atome d'oxygène.

4. Composé selon la revendication 1, 2 ou 3, qui est :
l'acide 4-[2-[4-(4-isobutylphénylméthoxy)benzoylamino]phénoxy]butano'ique,
l'acide 4-[2-(4-benzyloxybenzoylamino)phénoxy]butanoïque,
l'acide 4-[2-[4-(4-méthylphénylméthoxy)benzoylamino]phénoxy]butanoïque,
l'acide 4-[2-[4-(4-éthylphénylméthoxy)benzoylamino]phénoxy]butanoïque,
l'acide 4-[2-[4-(4-propylphénylméthoxy)benzoylamino]phénoxy]butanoïque,
l'acide 4-[2-[4-(4-isopropylphénylméthoxy)benzoylamino]phénoxy]butanoïque,
l'acide 4-[2-[4-(3-phénylpropoxy)benzoylamino]phénoxy]butanoïque,
l'acide 4-[2-[4-(4-phénylbutoxy)benzoylamino]phénoxy]butanoïque,
l'acide 4-[2-[4-(4-isobutylphénylméthoxy)-2-méthylbenzoylamino]phénoxy]butanoïque ou
l'acide 4-[2-[4-(4-isobutylphénylméthoxy)-3-méthylbenzoylamino]phénoxy]butanoïque, ou un sel non toxique de ceux-ci.

5. Composé selon la revendication 1 ou 2, dans lequel B est un groupement de formule générale NR" (où R¹¹ est tel que défini dans la revendication 1).

6. Composé selon la revendication 1, 2 ou 5, qui est : l'acide 4-[2-[4-(4-isobutylbenzylamino)benzoylamino]phénoxy]butanoïque,
l'acide 4-[2-[4-(4-isobutylbenzylamino)-2-méthylbenzoylamino]phénoxy]butanoïque,
l'acide 4-[2-[4-(4-propylbenzylamino)-2-méthylbenzoylamino]phénoxy]butanoïque,
l'acide 4-[2-[4-[1-(4-isobutylphényl)éthylamino]-2-méthylbenzoylamino]phénoxy]butanoïque,
l'acide 4-[2-[4-(4-isopropylbenzylamino)-2-méthylbenzoylamino]phénoxy]butanoïque,
l'acide 4-[2-[4-(4-propylbenzylamino)-2-méthylbenzoylamino]phénylthio]butanoïque, ou
l'acide 4-[2-[4-(2-trifluorométhylbenzylamino)-2-méthylbenzoylamino]phénoxy]butanoïque, ou un sel non toxique de ceux-ci.

7. Composé selon la revendication 1 qui répond à la formule générale : 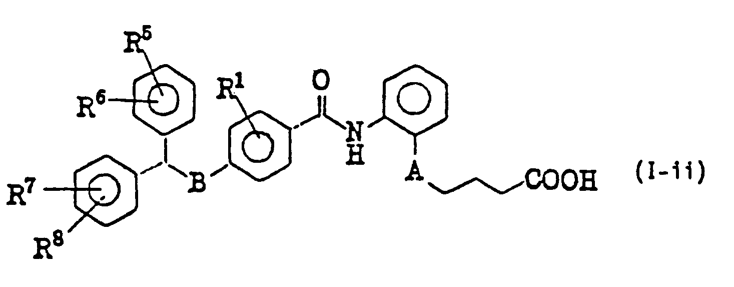
(dans laquelle
les symboles sont tels que définis dans la revendication 1).

8. Composé selon la revendication 1 ou 7, dans lequel B est un atome d'oxygène.

9. Composé selon la revendication 1, 7 ou 8, qui est : l'acide 4-[2-[4-[bis(4-propylphényl)méthoxy]benzoylamino]phénoxy]butanoïque,
l'acide 4-[2-[4-[bis(4-propylphényl)méthoxy]-2-méthylbenzoylamino]phénoxy]butano'ique,
l'acide 4-[2-[4-[bis(4-éthylphényl)méthoxy]benzoylamino]phénoxy]butanoïque,
l'acide 4-[2-[4-[bis(4-pentylphényl)méthoxy]benzoylamino]phénoxy]butanoïque, ou
l'acide 4-[2-[4-[bis(4-butylphényl)méthoxy]benzoylamino]phénoxy]butanoïque.

10. Composé selon la revendication 1 ou 7, dans lequel B est un groupement de formule générale NR" (où R¹¹ est tel que défini dans la revendication 1).

11. Composé selon la revendication 1, 7 ou 10, qui est :
l'acide 4-[2-[4-[bis(4-propylphényl)méthylamino]-2-méthylbenzoylamino]phénoxy]butanoïque,
l'acide 4-[2-[4-[bis(4-propylphényl)méthylamino]benzoylamino]phénoxy]butanoïque, ou
l'acide 4-[2-[4-[N-bis(4-propylphénylméthyl)-N-méthylamino]benzoylamino]phénoxy]butanoïque ou un sel non toxique de ceux-ci.

12. Composé selon la revendication 1 ou 7, dans lequel B est un atome de soufre.

13. Composé selon la revendication 1, 7 ou 12, qui est : l'acide 4-[2-[4-[bis(4-propylphényl)méthylthio]benzoylamino]phénoxy]butanoïque, ou un sel non toxique de celui-ci.

14. Composé selon la revendication 1, qui répond à la formule générale : 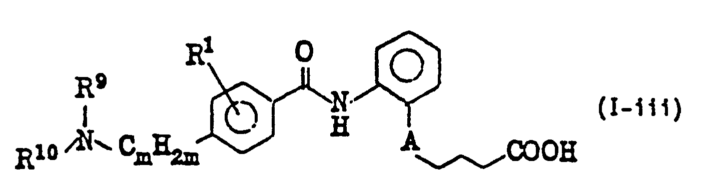
(dans laquelle
les symboles sont tels que définis dans la revendication 1).

15. Composé selon la revendication 1 ou 14, dans lequel R¹ est un atome d'hydrogène.

16. Composé selon la revendication 1, 14 ou 15, qui est :
l'acide 4-[2-[4-[N,N-bis(4-propylbenzyl)amino]benzoylamino]phénoxy]butanoïque,
l'acide 4-[2-[4-(4-propylphénylaminométhyl)benzoylamino]phénoxy]butanoïque,
l'acide 4-[2-[4-(5,6,7,8-tétrahydronapht-1-yl)aminométhylbenzoylamino]phénoxy]butanoïque, ou
l'acide 4-[2-[4-[N-méthyl-N-(5,6,7,8-tétrahydronapht-1 -yl)aminométhyl]benzoylamino]phénoxy]-butanoïque, ou un sel non toxique de ceux-ci.

17. Composé selon la revendication 1 ou 14, dans lequel R¹ est un groupement méthyle.

18. Composé selon la revendication 1, 14 ou 17, qui est :
l'acide 4-[2-[2-méthyl-4-[N,N-bis(4-propylbenzyl)amino]benzoylamino]phénoxy]butanoïque,
l'acide 4-[2-[4-[N,N-bis(4-isobutylbenzyl)amino]-2-méthylbenzoylamino]phénoxy]butanoïque,
l'acide 4-[2-[4-(N,N-dipentylamino)-2-méthylbenzoylamino]phénoxy]butanoïque,
l'acide 4-[2-[4-[N-pentyl-N-(4-propylbenzyl)amino]-2-méthylbenzoylamino]phénoxy]butanoïque,
l'acide 4-[2-[4-[N-méthyl-N-(4-propylbenzyl)amino]-2-méthylbenzoylamino]phénoxy]butanoïque,
l'acide 4-[2-[4-[N,N-bis(4-phénylbutyl)amino]-2-méthylbenzoylamino]phénoxy]butanoïque,
l'acide 4-[2-[4-[N,N-bis(4-isopropylbenzyl)amino]-2-méthylbenzoylamino]phénoxy]butanoïque,
l'acide 4-[2-[4-[N,N-bis(4-propylbenzyl)amino]-2-méthylbenzoylamino]phénylthio]butanoïque,
l'acide 4-[2-[4-[N,N-bis(4-propylbenzyl)amino]-2-méthylbenzoylamino]phénylsulfinyl]butanoïque,
l'acide 4-[2-[4-[N,N-bis(2,4-difluorobenzyl)amino]-2-méthylbenzoylamino]phénoxy]butanoïque,
l'acide 4-[2-[4-[N-(4-propylbenzyl)-N-(4-propylbenzoyl)amino]-2-méthylbenzoylamino]phénoxy]-butanoïque,
l'acide 4-[2-[4-[N,N-bis(2-trifluorométhylbenzyl)amino]-2-méthylbenzoylamino]phénoxy]butanoïque, ou
l'acide 4-[2-[4-[N,N-bis(4-trifluorométhylbenzyl)amino]-2-méthylbenzoylamino]phénoxy]butanoïque, ou un sel non toxique de ceux-ci.

19. Procédé de préparation d'un dérivé d'acide benzoylaminophénoxybutanoïque de formule générale : 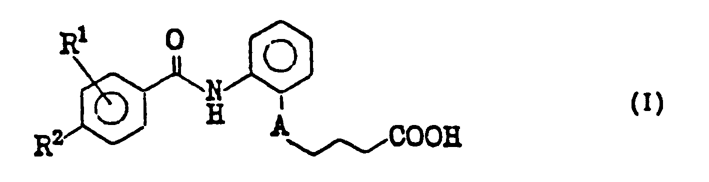 [dans laquelle
les différents symboles sont tels que définis dans la revendication 1 qui comprend :
(A) quand A représente un atome de soufre ou un atome d'oxygène, la saponification d'un composé de formule générale : 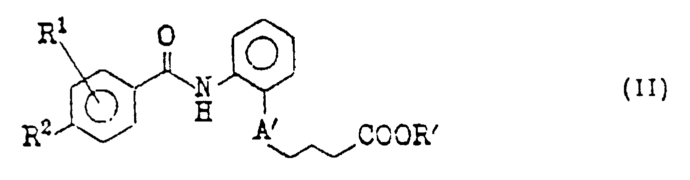 (dans laquelle
R' représente un groupement alkyle en C₁-C₄, A' représente un atome d'oxygène ou un atome de soufre et les autres symboles sont tels que définis dans la revendication 1), ou
(B) quand A, dans la formule générale (I) représente un groupement sulfinyle et que les autres symboles sont tels que définis dans la revendication 1, l'oxydation d'un composé de formule générale : 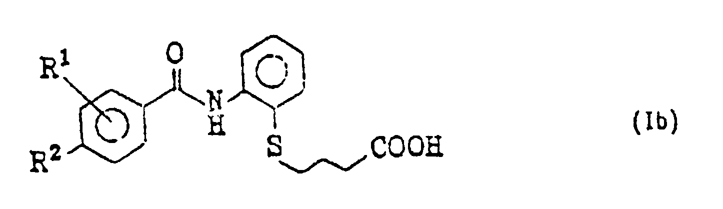 (dans laquelle
R¹ et R² sont tels que définis dans la revendication 1), afin de transformer le groupement -S- en un groupement -S( = 0)-,
et si on le souhaite, de transformer un composé de formule générale (I) en un sel non toxique de celui-ci.

20. Composition pharmaceutique qui comprend un dérivé d'acide benzoylaminophénoxybutanoïque selon la revendication 1 ou un sel non toxique de celui-ci en association avec un véhicule ou enrobage pharmaceutiquement acceptable.

21. Dérivé d'acide benzoylaminophénoxybutanoïque selon la revendication 1 ou sel non toxique de celui-ci, destiné à être utilisé dans la fabrication d'un médicament.

22. Utilisation d'un dérivé d'acide benzoylaminophénoxybutanoïque selon la revendication 1 ou d'un sel non toxique de celui-ci pour la fabrication d'un médicament destiné au traitement de l'alopécie, de l'acné ou de l'hypertrophie prostatique.

23. Composition cosmétique qui comprend un dérivé d'acide benzoyl-aminophénoxybutanoïque selon la revendication 1 ou un sel non toxique de celui-ci.

24. Utilisation d'un produit cosmétique constitué d'un dérivé d'acide benzoylaminophénoxybutanoïque selon la revendication 1 ou d'un sel non toxique de celui-ci.

25. Composé de formule générale (II) décrit dans la revendication 19 dans lequel R' et A' sont tels que définis dans la revendication 19 et les autres symboles sont tels que définis dans la revendication 1.

26. Procédé de préparation d'un composé de formule générale (II) tel que défini dans la revendication 19, qui comprend :
(A) la réaction du groupement carboxy (COOH) d'un composé de formule générale : 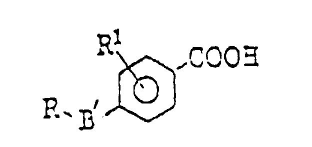 dans laquelle
B' représente un atome d'oxygène ou un atome de soufre, R¹ est tel que défini dans la revendication 1, et R représente un groupement de formule générale : 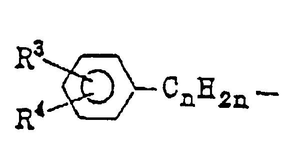 ou 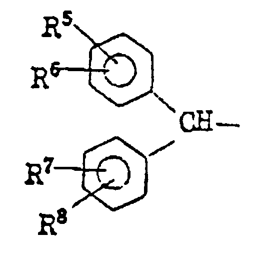 dans lesquelles
R³, R⁴, R⁵, R⁶, R⁷ et R⁸ sont tels que définis dans la revendication 1, avec le groupement amino (NH₂) d'un composé de formule générale : 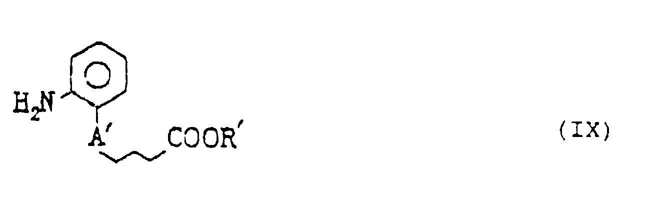 dans laquelle
A' représente un atome d'oxygène ou un atome de soufre et R' représente un groupement alkyle en C₁-C₄, afin de former une liaison amide (-CONH-);
(B) la réaction d'un composé de formule générale : 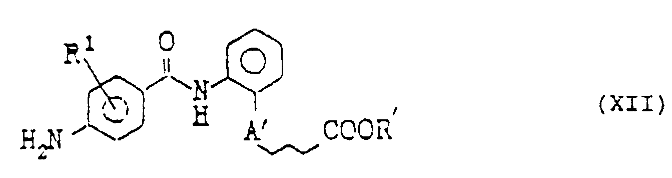 dans laquelle
R¹ est tel que défini dans la revendication 1, et A' et R' sont tels que définis ci-dessus, avec un composé de formule générale R-X¹, R⁹⁻X⁴ et/ou R¹⁰-X⁴ ou R¹¹-X⁵ où R est tel que défini ci-dessus, R⁹, R¹⁰ et R¹¹ sont tels que définis dans la revendication 1 et X¹, X⁴ et X⁵ représentent chacun un atome d'halogène, un groupement tosyle ou un groupement mésyle ; ou
(C) la réaction d'un composé de formule générale : 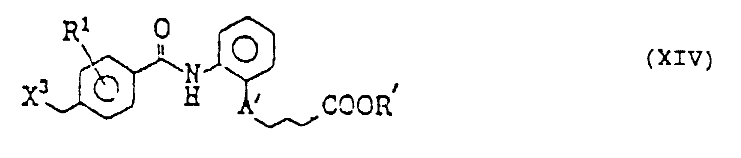 dans laquelle
R¹ est tel que défini dans la revendication 1, A' et R' sont tels que définis ci-avant et X³ représente un atome d'halogène, un groupement tosyle ou un groupement mésyle, avec un composé de formule générale : 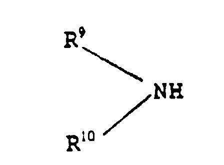 dans laquelle
R⁹ et R¹⁰ sont tels que définis dans la revendication 1.

## Revendications (Revendications pour l'(les) Etat(s) contractant(s) suivant(s) : ES)

1. Procédé de préparation d'un dérivé d'acide benzoylaminophénoxybutanoïque de formule générale : 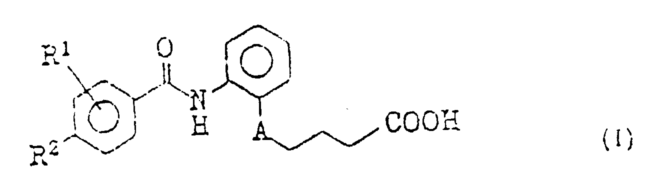 [dans laquelle
A représente un atome d'oxygène, un atome de soufre ou un groupement sulfinyle (SO),
R¹ représente un atome d'hydrogène ou un groupement méthyle,
R² représente un groupement de formule générale : 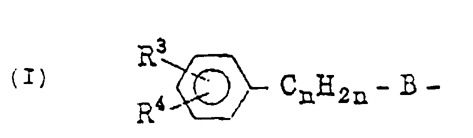 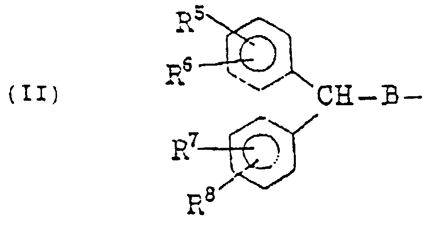 ou 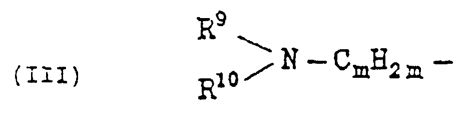 [dans lesquelles
B représente un atome d'oxygène, un atome de soufre ou un groupement de formule générale :
NR¹¹ (dans laquelle R¹¹ représente un atome d'hydrogène ou un groupement alkyle en C₁-C₄), R³, R⁴, R^{s}, R⁶, R⁷ et R⁸ représentent indépendamment un atome d'hydrogène, un groupement alkyle en C₁-C₄, un atome d'halogène ou un groupement trifluorométhyle,
m représente 0 ou 1,
n représente un nombre entier compris entre 1 et 4, et
R⁹ et R¹⁰, qui peuvent être identiques ou différents, représentent chacun un atome d'hydrogène, un groupement alkyle en C₁-C₅ ou un groupement de formule générale : 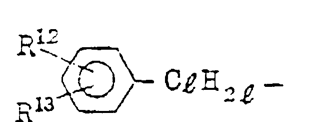 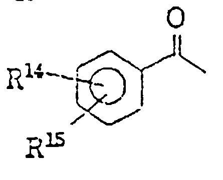 ou 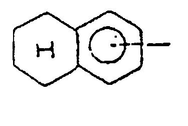 (dans lesquelles
R¹², R¹³, R¹⁴ et R¹⁵ représentent indépendamment un atome d'hydrogène, un groupement alkyle en C₁-C₄, un atome d'halogène ou un groupement trifluorométhyle et 1 représente un nombre entier compris entre 1 et 4), à condition que R⁹ et R¹⁰ ne représentent pas simultanément des atomes d'hydrogène] ou un sel non toxique de celui-ci, lequel procédé comprend :
(A) quand A représente un atome de soufre ou un atome d'oxygène, la saponification d'un composé de formule générale : 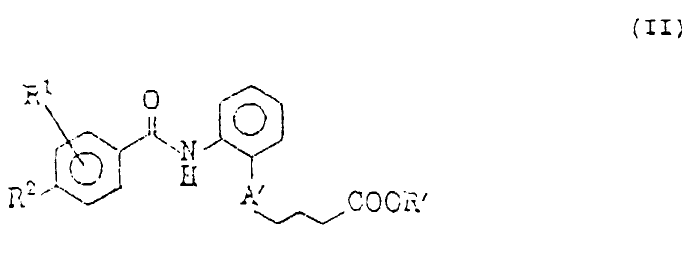 (dans laquelle R' représente un groupement alkyle en C₁-C₄, A' représente un atome d'oxygène ou un atome de soufre et les autres symboles sont tels que définis ci-avant), ou
(B) quand A, dans la formule générale (I) représente un groupement sulfinyle et que les autres symboles sont tels que définis ci-avant, l'oxydation d'un composé de formule générale : 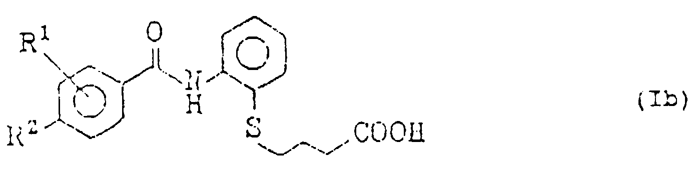 (dans laquelle R¹ et R² sont tels que définis ci-avant) afin de transformer le groupement -S- en un groupement -S-( = O)-, et si on le souhaite, de transformer un composé de formule générale (I) en un sel non toxique de celui-ci.

2. Procédé selon la revendication 1, pour la préparation d'un composé de formule générale : 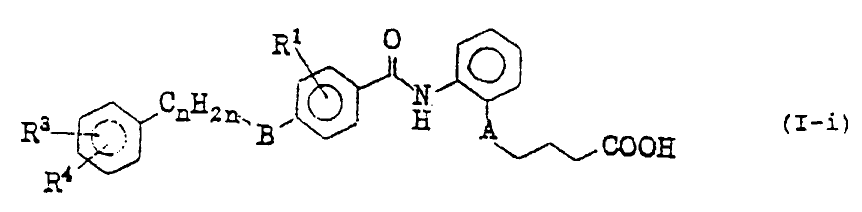 (dans laquelle
les symboles sont tels que définis dans la revendication 1).

3. Procédé selon la revendication 1 ou 2, pour la préparation d'un composé de formule générale (1), dans laquelle B représente un atome d'oxygène.

4. Procédé selon la revendication 1, 2 ou 3, pour la préparation d'un composé de formule générale (I) qui est :
l'acide 4-[2-[4-(4-isobutylphénylméthoxy)benzoylamino]phénoxy]butanoïque,
l'acide 4-[2-(4-benzyloxybenzoylamino)phénoxy]butanoïque,
l'acide 4-[2-[4-(4-méthylphénylméthoxy)benzoylamino]phénoxy]butanoïque,
l'acide 4-[2-[4-(4-éthylphénylméthoxy)benzoylamino]phénoxy]butanoïque,
l'acide 4-[2-[4-(4-propylphénylméthoxy)benzoylamino]phénoxy]butanoïque,
l'acide 4-[2-[4-(4-isopropylphénylméthoxy)benzoylamino]phénoxy]butanoïque,
l'acide 4-[2-[4-(3-phénylpropoxy)benzoylamino]phénoxy]butanoïque,
l'acide 4-[2-[4-(4-phénylbutoxy)benzoylamino]phénoxy]butanoïque,
l'acide 4-[2-[4-(4-isobutylphénylméthoxy)-2-méthylbenzoylamino]phénoxy]butanoïque ou
l'acide 4-[2-[4-(4-isobutylphénylméthoxy)-3-méthylbenzoylamino]phénoxy]butanoïque, ou un sel non toxique de ceux-ci.

5. Procédé selon la revendication 1 ou 2, pour la préparation d'un composé de formule générale (I) dans lequel B est un groupement de formule générale NR" (dans laquelle R¹¹ est tel que défini dans la revendication 1).

6. Procédé selon la revendication 1, 2 ou 5, pour la préparation d'un composé de formule générale (I) qui est :
l'acide 4-[2-[4-(4-isobutylbenzylamino)benzoylamino]phénoxy]butanoïque,
l'acide 4-[2-[4-(4-isobutylbenzylamino)-2-méthylbenzoylamino]phénoxy]butanoïque,
l'acide 4-[2-[4-(4-propylbenzylamino)-2-méthylbenzoylamino]phénoxy]butanoïque,
l'acide 4-[2-[4-[1-(4-isobutylphényl)éthylamino]-2-méthylbenzoylamino]phénoxy]butanoïque,
l'acide 4-[2-[4-(4-isopropylbenzylamino)-2-méthylbenzoylamino]phénoxy]butanoïque,
l'acide 4-[2-[4-(4-propylbenzylamino)-2-méthylbenzoylamino]phénylthio]butanoïque, ou
l'acide 4-[2-[4-(2-trifluorométhylbenzylamino)-2-méthylbenzoylamino]phénoxy]butanoïque, ou un sel non toxique de ceux-ci.

7. Procédé selon la revendication 1 pour la préparation d'un composé de formule générale (I) qui répond à la formule générale : 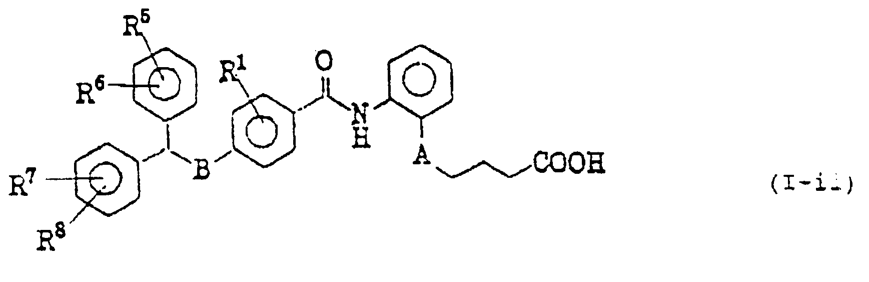 (dans laquelle
les symboles sont tels que définis dans la revendication 1).

8. Procédé selon la revendication 1 ou 7, pour la préparation d'un composé de formule générale (I) dans laquelle B représente un atome d'oxygène.

9. Procédé selon la revendication 1, 7 ou 8, pour la préparation d'un composé de formule générale (I) qui est :
l'acide 4-[2-[4-[bis(4-propylphényl)méthoxy]benzoylamino]phénoxy]butanoïque,
l'acide 4-[2-[4-[bis(4-propylphényl)méthoxy]-2-méthylbenzoylamino]phénoxy]butanoïque,
l'acide 4-[2-[4-[bis(4-éthylphényl)méthoxy]benzoylamino]phénoxy]butanoïque,
l'acide 4-[2-[4-[bis(4-pentylphényl)méthoxy]benzoylamino]phénoxy]butanoïque, ou
l'acide 4-[2-[4-[bis(4-butylphényl)méthoxy]benzoylamino]phénoxy]butanoïque ou un sel non toxique de celui-ci.

10. Procédé selon la revendication 1 ou 7, pour la préparation d'un composé de formule générale (I) dans laquelle B représente un groupement de formule générale NR¹¹ (dans laquelle R¹¹ est tel que défini dans la revendication 1).

11. Procédé selon la revendication 1, 7 ou 10, pour la préparation d'un composé de formule générale (I) qui est :
l'acide 4-[2-[4-[bis(4-propylphényl)méthylamino]-2-méthylbenzoylamino]phénoxy]butanoïque,
l'acide 4-[2-[4-[bis(4-propylphényl)méthylamino]benzoylamino]phénoxy]butanoïque, ou
l'acide 4-[2-[4-[N-bis(4-propylphénylméthyl)-N-méthylamino]benzoylamino]phénoxy]butanoïque ou un sel non toxique de ceux-ci.

12. Procédé selon la revendication 1 ou 7, pour la préparation d'un composé de formule générale (1), dans laquelle B représente un atome de soufre.

13. Procédé selon la revendication 1, 7 ou 12, pour la préparation d'un composé de formule générale (1), qui est :
l'acide 4-[2-[4-[bis(4-propylphényl)méthylthio]benzoylamino]phénoxy]butanoïque, ou un sel non toxique de celui-ci.

14. Procédé selon la revendication 1, pour la préparation d'un composé de formule générale (1), qui répond à la formule générale : 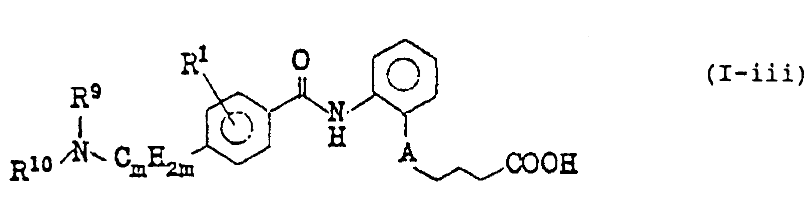 (dans laquelle
les symboles sont tels que définis dans la revendication 1).

15. Procédé selon la revendication 1 ou 14, pour la préparation d'un composé de formule générale (1), dans laquelle R¹ représente un atome d'hydrogène.

16. Procédé selon la revendication 1, 14 ou 15, pour la préparation d'un composé de formule générale (1), qui est :
l'acide 4-[2-[4-[N,N-bis(4-propylbenzyl)amino]benzoylamino]phénoxy]butano'ique,
l'acide 4-[2-[4-(4-propylphénylaminométhyl)benzoylamino]phénoxy]butanoïque,
l'acide 4-[2-[4-(5,6,7,8-tétrahydronapht-1-yl)aminométhylbenzoylamino]phénoxy]butanoïque, ou
l'acide 4-[2-[4-[N-méthyl-N-(5,6,7,8-tétrahydronapht-1 -yl)aminométhyl]benzoylamino]phénoxy]-butanoïque, ou un sel non toxique de ceux-ci.

17. Procédé selon la revendication 1 ou 14, pour la préparation d'un composé de formule générale (1), dans laquelle R¹ représente un groupement méthyle.

18. Procédé selon la revendication 1, 14 ou 17, pour la préparation d'un composé de formule générale (1), qui est :
l'acide 4-[2-[2-méthyl-4-[N,N-bis(4-propylbenzyl)amino]benzoylamino]phénoxy]butanoïque,
l'acide 4-[2-[4-[N,N-bis(4-isobutylbenzyl)amino]-2-méthylbenzoylamino]phénoxy]butanoïque,
l'acide 4-[2-[4-(N,N-dipentylamino)-2-méthylbenzoylamino]phénoxy]butanoïque,
l'acide 4-[2-[4-[N-pentyl-N-(4-propylbenzyl)amino]-2-méthylbenzoylamino]phénoxy]butano'ique,
l'acide 4-[2-[4-[N-méthyl-N-(4-propylbenzyl)amino]-2-méthylbenzoylamino]phénoxy]butanoïque,
l'acide 4-[2-[4-[N,N-bis(4-phénylbutyl)amino]-2-méthylbenzoylamino]phénoxy]butanoïque,
l'acide 4-[2-[4-[N,N-bis(4-isopropylbenzyl)amino]-2-méthylbenzoylamino]phénoxy]butanoïque,
l'acide 4-[2-[4-[N,N-bis(4-propylbenzyl)amino]-2-méthylbenzoylamino]phénylthio]butanoïque,
l'acide 4-[2-[4-[N,N-bis(4-propylbenzyl)amino]-2-méthylbenzoylamino]phénylsulfinyl]butanoïque,
l'acide 4-[2-[4-[N,N-bis(2,4-difluorobenzyl)amino]-2-méthylbenzoylamino]phénoxy]butanoïque,
l'acide 4-[2-[4-[N-(4-propylbenzyl)-N-(4-propylbenzoyl)amino]-2-méthylbenzoylamino]phénoxy]-butanoïque,
l'acide 4-[2-[4-[N,N-bis(2-trifluorométhylbenzyl)amino]-2-méthylbenzoylamino]phénoxy]butanoïque, ou
l'acide 4-[2-[4-[N,N-bis(4-trifluorométhylbenzyl)amino]-2-méthylbenzoylamino]phénoxy]butanoïque, ou un sel non toxique de ceux-ci.

19. Procédé selon l'une quelconque des revendications précédentes suivi par l'étape consistant à formuler un dérivé d'acide benzoylaminophénoxybutanoïque selon la revendication 1 ou un sel non toxique de celui-ci avec un véhicule ou enrobage pharmaceutiquement acceptable.

20. Utilisation d'un dérivé d'acide benzoylaminophénoxybutanoïque selon la revendication 1 ou d'un sel non toxique de celui-ci pour la fabrication d'un médicament pour le traitement de l'alopécie, de l'acné ou de l'hypertrophie prostatique.

21. Utilisation d'un produit cosmétique constitué d'un dérivé d'acide benzoylaminophénoxybutanoïque selon la revendication 1 ou d'un sel non toxique de celui-ci.

22. Procédé de préparation d'un composé de formule générale (II) tel que défini dans la revendication 1, qui comprend :
(A) la réaction du groupement carboxy (COOH) d'un composé de formule générale : 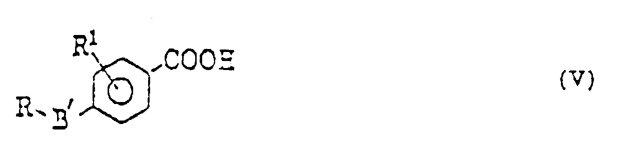 dans laquelle
B' représente un atome d'oxygène ou un atome de soufre, R¹ est tel que défini dans la revendication 1, et R représente un groupement de formule générale : 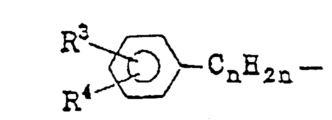 ou 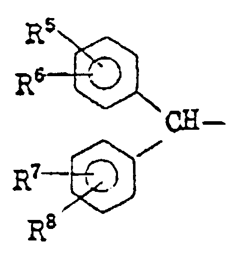 dans lesquelles
R³, R⁴, R⁵, R⁶, R⁷ et R⁸ sont tels que définis dans la revendication 1, avec le groupement amino (NH₂) d'un composé de formule générale : 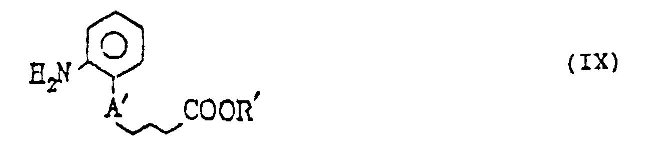 dans laquelle
A' représente un atome d'oxygène ou un atome de soufre et R' représente un groupement alkyle en C₁-C₄, afin de former une liaison amide (-CONH-);
(B) la réaction d'un composé de formule générale : 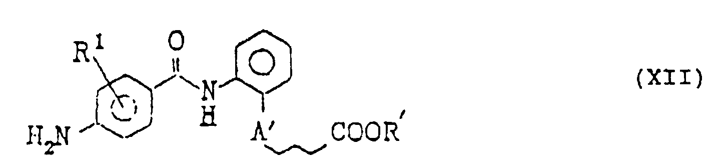 dans laquelle
R¹ est tel que défini dans la revendication 1, et A' et R' sont tels que définis ci-dessus, avec un composé de formule générale R-X¹, R⁹⁻X⁴ et/ou R¹⁰-X⁴ ou R¹¹-X⁵ où R est tel que défini ci-dessus, R⁹, R¹⁰ et R¹¹ sont tels que définis dans la revendication 1 et X¹, X⁴ et X⁵ représentent chacun un atome d'halogène, un groupement tosyle ou un groupement mésyle ; ou

(C) la réaction d'un composé de formule générale : 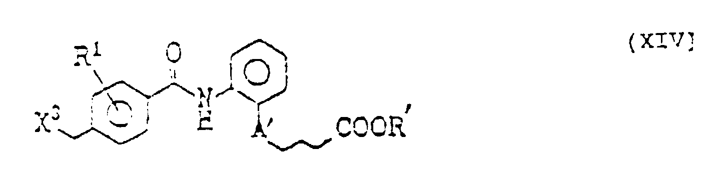 (dans laquelle
R¹ est tel que défini dans la revendication 1, A' et R' sont tels que définis ci-avant et X³ représente un atome d'halogène, un groupement tosyle ou un groupement mésyle avec un composé de formule générale : 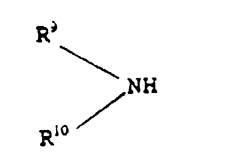 dans laquelle
R⁹ et R¹⁰ sont tels que définis dans la revendication 1.

## Revendications (Revendications pour l'(les) Etat(s) contractant(s) suivant(s) : GR)

1. Procédé de préparation d'un dérivé d'acide benzoylaminophénoxybutanoïque de formule générale : 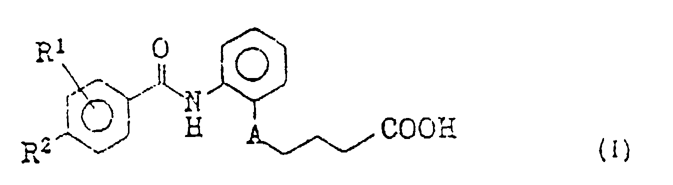 [dans laquelle
A représente un atome d'oxygène, un atome de soufre ou un groupement sulfinyle (SO),
R¹ représente un atome d'hydrogène ou un groupement méthyle,
R² représente un groupement de formule générale : 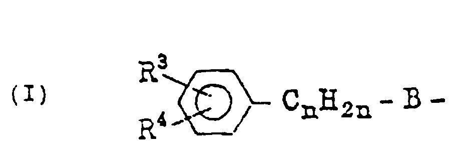 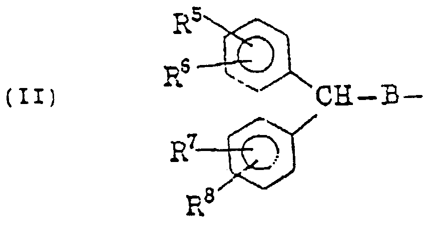 ou 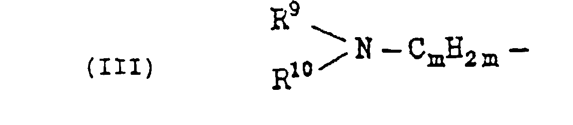 [dans lesquelles
B représente un atome d'oxygène, un atome de soufre ou un groupement de formule générale :
NR¹¹ (dans laquelle R¹¹ représente un atome d'hydrogène ou un groupement alkyle en C₁-C₄), R³, R⁴, R^{s}, R⁶, R⁷ et R⁸ représentent indépendamment un atome d'hydrogène, un groupement alkyle en C₁-C₄, un atome d'halogène ou un groupement trifluorométhyle,
m représente 0 ou 1,
n représente un nombre entier compris entre 1 et 4, et
R⁹ et R¹⁰, qui peuvent être identiques ou différents, représentent chacun un atome d'hydrogène, un groupement alkyle en C₁-C₅ ou un groupement de formule générale : 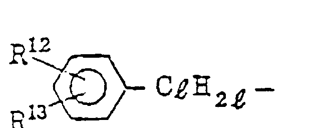 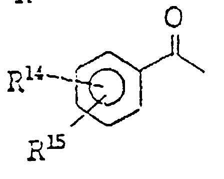 ou 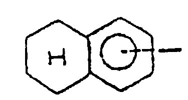 (dans lesquelles
R¹², R¹³, R¹⁴ et R¹⁵ représentent indépendamment un atome d'hydrogène, un groupement alkyle en C₁-C₄, un atome d'halogène ou un groupement trifluorométhyle et 1 représente un nombre entier compris entre 1 et 4), à condition que R⁹ et R¹⁰ ne représentent pas simultanément des atomes d'hydrogène] ou un sel non toxique de celui-ci, lequel procédé comprend :
(A) quand A représente un atome de soufre ou un atome d'oxygène, la saponification d'un composé de formule générale : 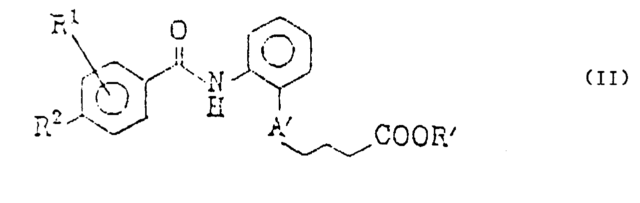 (dans laquelle
R' représente un groupement alkyle en C₁-C₄, A' représente un atome d'oxygène ou un atome de soufre et les autres symboles sont tels que définis ci-avant), ou
(B) quand A, dans la formule générale (I) représente un groupement sulfinyle et que les autres symboles sont tels que définis ci-avant, l'oxydation d'un composé de formule générale : 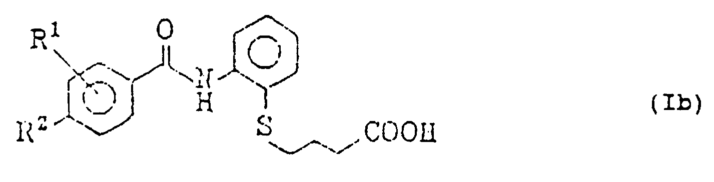 (dans laquelle
R¹ et R² sont tels que définis ci-avant) afin de transformer le groupement -S- en un groupement -S-( = O)-,
et si on le souhaite, de transformer un composé de formule générale (I) en un sel non toxique de celui-ci.

2. Procédé selon la revendication 1, pour la préparation d'un composé de formule générale : 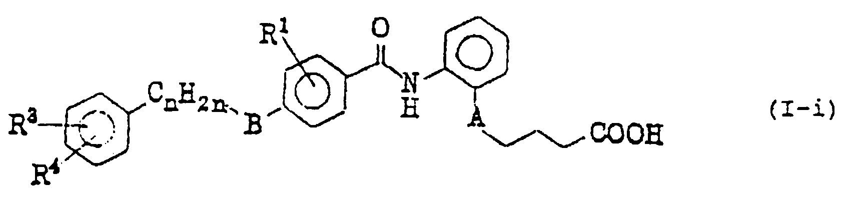 (dans laquelle les symboles sont tels que définis dans la revendication 1).

3. Procédé selon la revendication 1 ou 2, pour la préparation d'un composé de formule générale (1), dans laquelle B représente un atome d'oxygène.

4. Procédé selon la revendication 1, 2 ou 3, pour la préparation d'un composé de formule générale (I) qui est :
l'acide 4-[2-[4-(4-isobutylphénylméthoxy)benzoylamino]phénoxy]butanoïque,
l'acide 4-[2-(4-benzyloxybenzoylamino)phénoxy]butanoïque,
l'acide 4-[2-[4-(4-méthylphénylméthoxy)benzoylamino]phénoxy]butanoïque,
l'acide 4-[2-[4-(4-éthylphénylméthoxy)benzoylamino]phénoxy]butanoïque,
l'acide 4-[2-[4-(4-propylphénylméthoxy)benzoylamino]phénoxy]butanoïque,
l'acide 4-[2-[4-(4-isopropylphénylméthoxy)benzoylamino]phénoxy]butanoïque,
l'acide 4-[2-[4-(3-phénylpropoxy)benzoylamino]phénoxy]butanoïque,
l'acide 4-[2-[4-(4-phénylbutoxy)benzoylamino]phénoxy]butanoïque,
l'acide 4-[2-[4-(4-isobutylphénylméthoxy)-2-méthylbenzoylamino]phénoxy]butanoïque ou
l'acide 4-[2-[4-(4-isobutylphénylméthoxy)-3-méthylbenzoylamino]phénoxy]butanoïque, ou un sel non toxique de ceux-ci.

5. Procédé selon la revendication 1 ou 2, pour la préparation d'un composé de formule générale (I) dans lequel B est un groupement de formule générale NR" (dans laquelle R¹¹ est tel que défini dans la revendication 1).

6. Procédé selon la revendication 1, 2 ou 5, pour la préparation d'un composé de formule générale (I) qui est :
l'acide 4-[2-[4-(4-isobutylbenzylamino)benzoylamino]phénoxy]butanoïque,
l'acide 4-[2-[4-(4-isobutylbenzylamino)-2-méthylbenzoylamino]phénoxy]butanoïque,
l'acide 4-[2-[4-(4-propylbenzylamino)-2-méthylbenzoylamino]phénoxy]butanoïque,
l'acide 4-[2-[4-[1-(4-isobutylphényl)éthylamino]-2-méthylbenzoylamino]phénoxy]butanoïque,
l'acide 4-[2-[4-(4-isopropylbenzylamino)-2-méthylbenzoylamino]phénoxy]butanoïque,
l'acide 4-[2-[4-(4-propylbenzylamino)-2-méthylbenzoylamino]phénylthio]butanoïque, ou
l'acide 4-[2-[4-(2-trifluorométhylbenzylamino)-2-méthylbenzoylamino]phénoxy]butanoïque, ou un sel non toxique de ceux-ci.

7. Procédé selon la revendication 1 pour la préparation d'un composé de formule générale (I) qui répond à la formule générale : 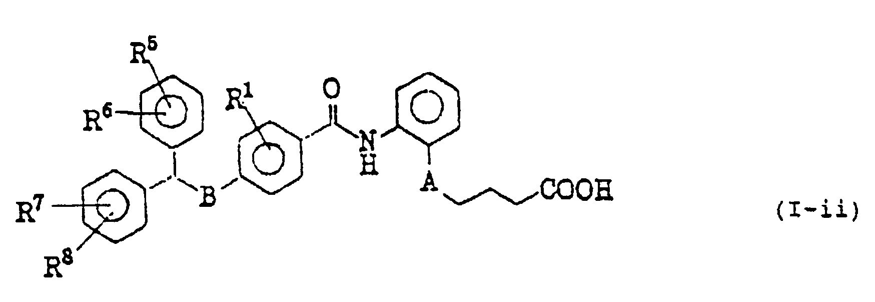 (dans laquelle
les symboles sont tels que définis dans la revendication 1).

8. Procédé selon la revendication 1 ou 7, pour la préparation d'un composé de formule générale (I) dans laquelle B représente un atome d'oxygène.

9. Procédé selon la revendication 1, 7 ou 8, pour la préparation d'un composé de formule générale (I) qui est :
l'acide 4-[2-[4-[bis(4-propylphényl)méthoxy]benzoylamino]phénoxy]butanoïque,
l'acide 4-[2-[4-[bis(4-propylphényl)méthoxy]-2-méthylbenzoylamino]-phénoxy]butanoïque,
l'acide 4-[2-[4-[bis(4-éthylphényl)méthoxy]benzoylamino]phénoxy]butanoïque,
l'acide 4-[2-[4-[bis(4-pentylphényl)méthoxy]benzoylamino]phénoxy]butanoïque, ou
l'acide 4-[2-[4-[bis(4-butylphényl)méthoxy]benzoylamino]phénoxy]butanoïque ou un sel non toxique de ceux-ci.

10. Procédé selon la revendication 1 ou 7, pour la préparation d'un composé de formule générale (I) dans laquelle B représente un groupement de formule générale NR¹¹ (dans laquelle R¹¹ est tel que défini dans la revendication 1).

11. Procédé selon la revendication 1, 7 ou 10, pour la préparation d'un composé de formule générale (I) qui est :
l'acide 4-[2-[4-[bis(4-propylphényl)méthylamino]-2-méthylbenzoylamino]phénoxy]butanoïque,
l'acide 4-[2-[4-[bis(4-propylphényl)méthylamino]benzoylamino]phénoxy]butanoïque, ou
l'acide 4-[2-[4-[N-bis(4-propylphénylméthyl)-N-méthylamino]benzoylamino]phénoxy]butanoïque ou un sel non toxique de ceux-ci.

12. Procédé selon la revendication 1 ou 7, pour la préparation d'un composé de formule générale (1), dans laquelle B représente un atome de soufre.

13. Procédé selon la revendication 1, 7 ou 12, pour la préparation d'un composé de formule générale (1), qui est :
l'acide 4-[2-[4-[bis(4-propylphényl)méthylthio]benzoylamino]phénoxy]butanoïque, ou un sel non toxique de celui-ci.

14. Procédé selon la revendication 1, pour la préparation d'un composé de formule générale (1), qui répond à la formule générale : 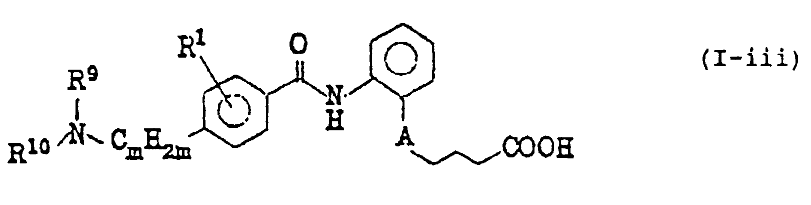 (dans laquelle
les symboles sont tels que définis dans la revendication 1).

15. Procédé selon la revendication 1 ou 14, pour la préparation d'un composé de formule générale (1), dans laquelle R¹ représente un atome d'hydrogène.

16. Procédé selon la revendication 1, 14 ou 15, pour la préparation d'un composé de formule générale (1), qui est :
l'acide 4-[2-[4-[N,N-bis(4-propylbenzyl)amino]benzoylamino]phénoxy]butanoïque,
l'acide 4-[2-[4-(4-propylphénylaminométhyl)benzoylamino]phénoxy]butanoïque,
l'acide 4-[2-[4-(5,6,7,8-tétrahydronapht-1-yl)aminométhylbenzoylamino]phénoxy]butanoïque, ou
l'acide 4-[2-[4-[N-méthyl-N-(5,6,7,8-tétrahydronapht-1-yl)aminométhyl]benzoylamino]phénoxy]-butanoïque, ou un sel non toxique de ceux-ci.

17. Procédé selon la revendication 1 ou 14, pour la préparation d'un composé de formule générale (1), dans laquelle R¹ représente un groupement méthyle.

18. Procédé selon la revendication 1, 14 ou 17, pour la préparation d'un composé de formule générale (I), qui est :
l'acide 4-[2-[2-méthyl-4-[N,N-bis(4-propylbenzyl)amino]benzoylamino]phénoxy]butanoïque,
l'acide 4-[2-[4-[N,N-bis(4-isobutylbenzyl)amino]-2-méthylbenzoylamino]phénoxy]butanoïque,
l'acide 4-[2-[4-(N,N-dipentylamino)-2-méthylbenzoylamino]phénoxy]butanoïque,
l'acide 4-[2-[4-[N-pentyl-N-(4-propylbenzyl)amino]-2-méthylbenzoylamino]phénoxy]butanoïque,
l'acide 4-[2-[4-[N-méthyl-N-(4-propylbenzyl)amino]-2-méthylbenzoylamino]phénoxy]butanoïque,
l'acide 4-[2-[4-[N,N-bis(4-phénylbutyl)amino]-2-méthylbenzoylamino]phénoxy]butanoïque,
l'acide 4-[2-[4-[N,N-bis(4-isopropylbenzyl)amino]-2-méthylbenzoylamino]phénoxy]butanoïque,
l'acide 4-[2-[4-[N,N-bis(4-propylbenzyl)amino]-2-méthylbenzoylamino]phénylthio]butanoïque,
l'acide 4-[2-[4-[N,N-bis(4-propylbenzyl)amino]-2-méthylbenzoylamino]phénylsulfinyl]butanoïque,
l'acide 4-[2-[4-[N,N-bis(2,4-difluorobenzyl)amino]-2-méthylbenzoylamino]phénoxy]butanoïque,
l'acide 4-[2-[4-[N-(4-propylbenzyl)-N-(4-propylbenzoyl)amino]-2-méthylbenzoylamino]phénoxy]-butanoïque,
l'acide 4-[2-[4-[N,N-bis(2-trifluorométhylbenzyl)amino]-2-méthylbenzoylamino]phénoxy]butanoïque, ou
l'acide 4-[2-[4-[N,N-bis(4-trifluorométhylbenzyl)amino]-2-méthylbenzoylamino]phénoxy]butanoïque, ou un sel non toxique de ceux-ci.

19. Procédé selon l'une quelconque des revendications précédentes suivi par l'étape consistant à formuler un dérivé d'acide benzoylaminophénoxybutanoïque selon la revendication 1 ou un sel non toxique de celui-ci avec un véhicule ou enrobage pharmaceutiquement acceptable.

20. Utilisation d'un dérivé d'acide benzoylaminophénoxybutanoïque selon la revendication 1 ou d'un sel non toxique de celui-ci pour la fabrication d'un médicament pour le traitement de l'alopécie, de l'acné ou de l'hypertrophie prostatique.

21. Utilisation d'un produit cosmétique constitué d'un dérivé d'acide benzoylaminophénoxybutanoïque selon la revendication 1 ou d'un sel non toxique de celui-ci.

22. Composé de formule générale (II) décrit dans la revendication 1 dans lequel les différents symboles sont tels que définis dans la revendication 1.

23. Procédé de préparation d'un composé de formule générale (II) tel que défini dans la revendication 1, qui comprend :
(A) la réaction du groupement carboxy (COOH) d'un composé de formule générale : 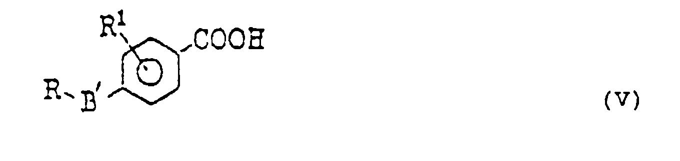 dans laquelle
B' représente un atome d'oxygène ou un atome de soufre, R¹ est tel que défini dans la revendication 1, et R représente un groupement de formule générale : 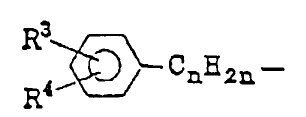 ou 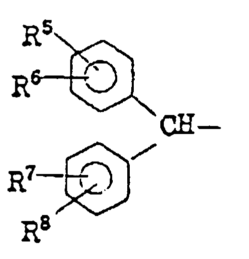 dans lesquelles
R³, R⁴, R⁵, R⁶, R⁷ et R⁸ sont tels que définis dans la revendication 1, avec le groupement amino (NH₂) d'un composé de formule générale : 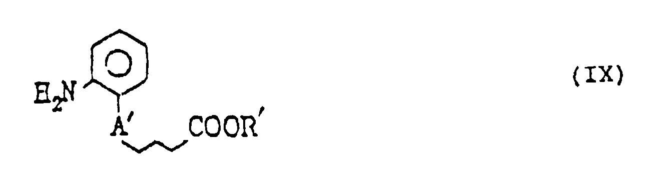 dans laquelle
A' représente un atome d'oxygène ou un atome de soufre et R' représente un groupement alkyle en C₁-C₄, afin de former une liaison amide (-CONH-);
(B) la réaction d'un composé de formule générale : 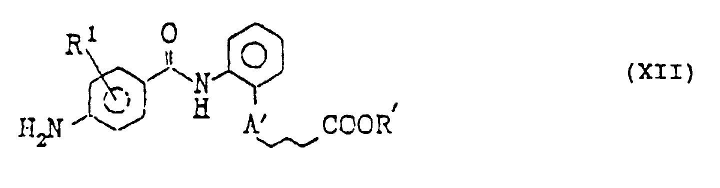 dans laquelle
R¹ est tel que défini dans la revendication 1, et A' et R' sont tels que définis ci-dessus, avec un composé de formule générale R-X¹, R⁹⁻X⁴ et/ou R¹⁰-X⁴ ou R¹¹-X⁵ où R est tel que défini ci-dessus, R⁹, R¹⁰ et R¹¹ sont tels que définis dans la revendication 1 et X¹, X⁴ et X⁵ représentent chacun un atome d'halogène, un groupement tosyle ou un groupement mésyle ; ou
(C) la réaction d'un composé de formule générale : 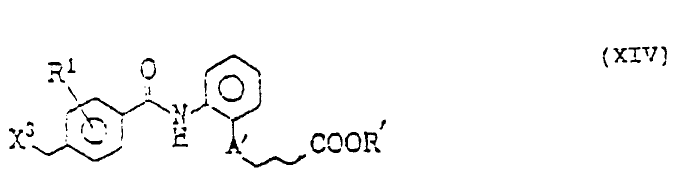 (dans laquelle
R¹ est tel que défini dans la revendication 1, A' et R' sont tels que définis ci-avant et X³ représente un atome d'halogène, un groupement tosyle ou un groupement mésyle avec un composé de formule générale : 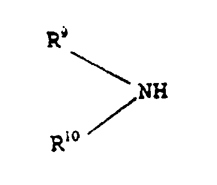 dans laquelle
R⁹ et R¹⁰ sont tels que définis dans la revendication 1.
